(19) Europäisches Patentamt
European Patent Office
Office européen des brevets

(11) EP 2 333 100 B1

(12) **EUROPÄISCHE PATENTSCHRIFT**

(45) Veröffentlichungstag und Bekanntmachung des Hinweises auf die Patenterteilung:
**09.11.2016 Patentblatt 2016/45**

(51) Int Cl.:
*C12P 33/02* (2006.01) *C12N 9/04* (2006.01)

(21) Anmeldenummer: **10194494.0**

(22) Anmeldetag: **10.12.2010**

(54) **NAD(P)+-Cofaktorregenerierungssystem und dessen Anwendungen**

NAD(P)+-cofactor regeneration system und its use

Système de régénération de cofacteur NAD(P)+ et ses applications

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **11.12.2009 EP 09178963**

(43) Veröffentlichungstag der Anmeldung:
**15.06.2011 Patentblatt 2011/24**

(73) Patentinhaber: **PharmaZell GmbH**
**83064 Raubling (DE)**

(72) Erfinder:
- **Liu, Luo**
  **102445 Beijing (CN)**
- **Momoi, Kyoko**
  **70825 Korntal (DE)**
- **Schmid, Rolf D.,**
  **DE-70569 Stuttgart (DE)**
- **Aigner, Arno**
  **DE-83104 Tuntenhausen (DE)**

(74) Vertreter: **Reitstötter Kinzebach**
**Patentanwälte**
**Sternwartstrasse 4**
**81679 München (DE)**

(56) Entgegenhaltungen:
**EP-A1- 1 731 618** **WO-A1-03/071283**
**WO-A2-2009/118176**

- **LEE L G ET AL: "ENZYME-CATALYZED ORGANIC SYNTHESIS: A COMPARISON OF STRATEGIES FOR IN SITU REGENERATION OF NAD FROM NADH", JOURNAL OF THE AMERICAN CHEMICAL SOCIETY, AMERICAN CHEMICAL SOCIETY, NEW YORK, US LNKD- DOI:10.1021/JA00310A043, Bd. 107, Nr. 24, 1. Januar 1985 (1985-01-01), Seiten 6999-7008, XP001056787, ISSN: 0002-7863**
- **DRUECKHAMMER D G ET AL: "FMN reductase catalyzed regeneration of NADP for use in enzymatic synthesis", JOURNAL OF ORGANIC CHEMISTRY, AMERICAN CHEMICAL SOCIETY, EASTON.; US LNKD- DOI:10.1021/JO00225A080, Bd. 50, Nr. 25, 1. Januar 1985 (1985-01-01), Seiten 5387-5389, XP002238520, ISSN: 0022-3263**
- **KIM Y ET AL: "Photogeneration of NADPH by oligothiophenes coupled with ferredoxin-NADP reductase", JOURNAL OF BIOTECHNOLOGY, ELSEVIER SCIENCE PUBLISHERS, AMSTERDAM, NL LNKD- DOI:10.1016/S0168-1656(97)00155-7, Bd. 59, Nr. 3, 3. Januar 1998 (1998-01-03) , Seiten 213-220, XP004113752, ISSN: 0168-1656**
- **JONES J B ET AL: "NICOTINAMIDE COENZYME REGENERATION. FLAVIN MONUNUCLEOTIDE (RIBOFLAVIN PHOSPHATE) AS AN EFFICIENT, ECONOMICAL, AND ENZYME-COMPATIBLE RECYCLING AGENT", CANADIAN JOURNAL OF CHEMISTRY, NRC RESEARCH PRESS, CA LNKD- DOI:10.1139/V76-420, Bd. 54, Nr. 19, 1. Oktober 1976 (1976-10-01), Seiten 2969-2973, XP008016220, ISSN: 0008-4042**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 333 100 B1

**Beschreibung**

Die vorliegende Erfindung betrifft ein Verfahren zur enzymatischen Oxidation von 12α-Hydroxysteroiden, wie insbesondere die enzymatische Oxidation von CS, oder ein Verfahren zur Herstellung von Ursodesoxycholsäure (UDCS) unter Anwendung eines erfindungsgemäßen Cofaktorregenerierungssystems, welches insbesondere in der Lage ist, genügend Cofaktor, wie z.B. $NADP^+$, in-situ bereitzustellen, um die enzymatische Oxidation von Steroiden, wie z.B. Cholsäure (CS), durch geeignete Dehydrogenasen, wie z.B. Hydroxysteroid-Dehydrogenasen (HSDH) in technischem Maßstab zu ermöglichen.

## Hintergrund der Erfindung

Die regio- und stereoselektive Oxidation von organischen Substanzen, katalysiert durch Dehydrogenasen, ist von großem Interesse bei der Synthese von Feinchemikalien in der pharmazeutischen Industrie. Prinzipiell wird bei der Reaktion, die durch eine Dehydrogenase katalysiert wird, eine äquimolare Menge eines biologischen Elektronenakzeptors (Cofaktor, in den meisten Fällen $NADP^+$ oder $NAD^+$) reduziert, um die gleiche Menge an einer oxidierten Substanz zu produzieren. Bei der großtechnischen Herstellung ist eine effiziente Reoxidation des Cofaktors erforderlich, insbesondere aufgrund der hohen Kosten des Cofaktors. Verschiedene Ansätze (enzymatisch, chemisch, photochemisch und elektrochemisch) wurden in der Literatur hierzu bereits beschrieben (vgl. R. Wichmann, 2005, Adv. Biochem. Engin / Biotechnol (2005) 92: 225-260). Bisher wurde jedoch noch kein breiter anwendbares, insbesondere generell anwendbares Regenerierungssystem mit guter Effizienz entwickelt.

12alpha-Ketochenodioxycholsäure (12alpha-KetoCDCS) stellt eine wichtige Zwischenstufe für die Synthese von Ursodesoxycholsäure (UDCS) dar, welche für die nichtoperative Behandlung von Cholesterin-bedingten Gallensteinen verwendet wird. 12alpha-KetoCDCS wird dabei enzymatisch aus Cholsäure (CS) über die selektive Oxidation an der 12-Position produziert. Die Reaktion wird durch 12alpha-Hydroxysteroiddehydrogenase (12alpha-HSDH) katalysiert und erfordert ein wirkungsvolles, effizientes $NADP^+$-Regenerierungssystem unter den dort herrschenden speziellen Verfahrensbedingungen.

Aus der älteren Internationalen Patentanmeldung PCT/EP2009/002190 (veröffentlicht unter Nr. WO2009/118176) sind neuartige 12α-Hydroxysteroiddehydrogenasen, dafür kodierende Nukleinsäuresequenzen, Expressionskassetten und Vektoren; rekombinante Mikroorganismen, enthaltend entsprechende kodierende Nukleinsäuresequenzen; Verfahren zur Herstellung solcher 12 α-Hydroxysteroiddehydrogenasen; Verfahren zur enzymatischen Oxidation von 12α-Hydroxysteroiden unter Verwendung derartiger Enzyme, Verfahren zur enzymatischen Reduktion von 12-Ketosteroiden unter Verwendung solcher Enzyme; Verfahren zur qualitativen oder quantitativen Bestimmung von 12-Ketosteroiden bzw. 12α-Hydroxysteroiden unter Verwendung dieser 12α-Hydroxysteroiddehydrogenasen; sowie ein Verfahren zur Herstellung von Ursodesoxycholsäure, umfassend die enzymkatalysierte Cholsäureoxidation unter Verwendung dieser 12α-Hydroxysteroiddehydrogenasen bekannt.

Lee, L. G. und Whitesides, G.M. beschreiben in J. Am. Chem.Soc. 1985, 107, 6999-7008 verschiedene Methoden zur in-situ Regeneration von $NAD^+$ aus NADH unter anderem mit Hilfe des Enzyms Diaphorase aus *Clostridium kluyveri* und methylenblau als intermediärem Elektronenträger. Insbesondere wurde die NAD-verbrauchende Oxidation von cis-Cyclohexandimethanol, katalysiert durch die Pferdeleber-Alkoholdehydrogenase (HLADH) untersucht. Die Anwendung des Systems auf andere Redoxreaktionen, wie z.B. Steroidverbindungen, wird darin nicht vorgeschlagen.

Die WO 03/071283 beschreibt einen Assay zur Bestimmung der Konzentration von NADH oder NADPH in einer Probe bei dem man eine Reduktase und eine redoxaktive Substanz mit der Probe in Kontakt bringt. Bei der Reduktase kann es sich beispielsweise um die Flavin (FAD/FMN)-Domäne des $P450_{BM-3}$-Enzyms aus *Bacillus megaterium* handeln, bei der redoxaktiven Substanz beispielswiese um $Fe(CN)_6^{3+}$.

Drueckhammer, D. G. et al. beschreiben in J. Org. Chem. 1985, 50, 5387-5389 die durch FMN Reduktase katalysierte Regeneration von NAD(P) zur Verwendung in der enzymatischen Synthese.

## Kurzfassung der Erfindung

Es ist deshalb Aufgabe der vorliegenden Erfindung, ein verbessertes Cofaktorregenerierungssystems bereitzustellen, welches bei enzymatischen Oxidationen von 12α-Hydroxysteroiden, die durch das Enzym 12α-HSDH katalysiert werden, eine effiziente Cofaktorregenerierung über den Labormaßstab hinaus ermöglicht.

Überraschenderweise konnte obige Aufgabe durch Bereitstellung von Verfahren gemäß beiliegenden Patentansprüchen gelöst werden.

Erfindungsgemäß wird die enzymatische Eigenschaft von Dehydrogenasen genutzt, um Elektronen von dem reduzierten Cofaktor NAD(P)H auf verschiedene Redox-Mediatoren zu übertragen. In Abhängigkeit von deren Redoxpotential können dann verschiedene reduzierte Mediatoren in unterschiedlicher Geschwindigkeit nicht-enzymatisch durch molekularen Sauerstoff oxidiert werden. Der oxidierte Mediator wird erneut durch die Dehydrogenase reduziert

(Redox-Zyklus). Der Redoxzyklus setzt sich fort bis sämtliches NAD(P)H oxidiert ist oder einer der Faktoren inaktiviert wird (vgl. auch Figur 1).

Im Vergleich zur Verwendung anderer üblicher Cofaktor-Regenerierungssysteme, z.B. basierend auf Glutamat Dehydrogenase/alpha-Ketoglutarat oder Alkohol Dehydrogenase/Aceton, bietet das erfindungsgemäße Verfahren den überraschenden Vorteil, dass die Reaktion aufgrund des hohen Redox-Potentials von Sauerstoff praktisch irreversibel ist, sehr geringe Mediator-Konzentrationen erforderlich sind und die damit gekoppelte chemische Umsetzung, wie z.B. CS zu 12-Keto-CDCS, praktisch vollständig und in einem Schritt verläuft.

## Figurenbeschreibung

Figur 1 zeigt das Prinzip eines Redoxzyklus zur Cofaktorregenerierung mit Hilfe des Enzyms Flavodoxin Reduktase, einer erfindungsgemäß verwendeten Dehydrogenase. $E_0$ beschreibt die Redoxpotentiale für NAD(P)H, FAD (aktives Zentrum der Reduktase), Methylenblau (verwendeter Mediator) und molekularen Sauerstoff.

Figur 2 zeigt eine Dünnschichtchromatographie von Reaktionsansätzen für die enzymatische Oxidation von Cholsäure mit dem erfindungsgemäßen Cofaktorregenerationssystem. **Bahn 1:** ohne Redoxmediator, Bahnen 2 bis 4: mit Redoxmediator, und zwar **Bahn 2:** Dichlorindophenol; **Bahn 3:** Methylenblau; **Bahn 4:** Indigocarmin.

Figur 3 zeigt eine Abschätzung der Zusammensetzung des Reaktionsproduktes bei der Cholsäure-Oxidation mit Hilfe des erfindungsgemäßen Regenerierungssystems. **RM:** Reaktionsprodukt 89-90 % 12-Keto-CDCS); **98:** 98 % 12-Keto-CDCS und 2 % CS; **95:** 95 % 12-Keto-CDCS und 5 % CS; **90:** 90 % 12-Keto-CDCS und 10 % CS.

Figur 4 zeigt einen Vergleich der Umsetzung von CS mit 12$\alpha$-HSDH in Gegenwart verschiedener $NADP^+$-regenerierender Enzyme. 0,18 U des NADPH-oxidierenden Enzyms wurden in 1 ml einer Cholsäure-Oxidationsmischung, enthaltend 25 mmol Tris-HCl, 6 % CS, 0,05 % Methylenblau und 0,05 mg/ml Katalase sowie 5 U/ml 12$\alpha$-HSDH und 10 $\mu$mol NADPH über Nacht bei Zimmertemperatur oxidiert. Die Reaktionsansätze wurden dünnschichtchromatographisch aufgetrennt. Gezeigt sind die Banden für CS und 12-Keto-CDCS; **Bahn 1:** Flavodoxin Reduktase aus *E. coli*; **Bahn 2:** Reduktase Domäne der CYP102A1; **Bahn 3:** NADPH Dehydrogenase aus G. *stearothermophilus* Sulfitreduktase; **Bahn 4:** Diaphorase aus *Clostridium kluyveri*; **Bahn 5:** Standardmischung aus 12-Keto-CDCS und CS (molares Verhältnis 98:2); **Bahn 6:** Standardmischung aus 12-Keto-CDCS und CS (molares Verhältnis 90:10).

Figur 5 veranschaulicht die Umsetzung von Cholsäure zu 12-keto-Chenodesoxycholsäure (12-keto CDCS) unter Verwendung von Riboflavin als Redoxmediator. **Bahn 1:** NAD(P)H-Flavin Reduktase aus *E.coli*; **Bahn 2:** NADPH Dehydrogenase aus *G. Stearothermophilus* Sulfitreduktase; **Bahn 3:** Ansatz ohne Enzym; **Bahn 4:** Standardgemisch von 12-keto-Chenodesoxycholsäure und Cholsäure (molares Verhältnis 90:10); **Bahn 5:** Standardgemisch von 12-keto-Chenodesoxycholsäure und Cholsäure (molares Verhältnis 98:2).

Figur 6 zeigt die Nukleotid- und Aminosäuresequenzen verwendeter NAD(P)H Dehydrogenasen: **(A)** Reduktase Domäne der CYP102A1 aus *Bacillus megaterium;* Originalprotein ID: UniProtKB/Swiss-Prot P14779 (Bifunctional P-450/NADPH-P450 Reductase). Die angegebene Sequenz kodiert die NADPH-P-450 Reduktase Region des Originalproteins mit His-Tag am N-Terminus.; **(B)** Flavodoxin Reduktase aus *Escherichia coli* strain K12 (EC 1.18.1.2, GeneID: 948414, Product: P_418359.1); **(C)** NADPH Dehydrogenase aus *Geobacillus stearothermophilus* Sulfit Reduktase (EC 1.8.1.2) mit His-Tag am N-Terminus; **(D)** NAD(P)H-Flavin Reduktase (EC 1.5.1.29 /1.16.1.3, GeneID: 6061282, Product YP_001732642.1).

Figur 7 veranschaulicht am Beispiel der Flavodoxin Reduktase den Verlauf der Enzymaktivität in Abhängigkeit von steigenden Cholsäurekonzentrationen im Bereich von 0 bis 400 mM Cholsäure.

## Detaillierte Beschreibung der Erfindung

### 1. Bevorzugte Ausführungsformen

Hierin offenbart sind folgende Ausführungsformen:

1. Ein (nicht beanspruchtes) enzymatisches Cofaktorregenerierungssystem zur Regenerierung von verbrauchten Redox-Äquivalenten, umfassend

a) eine NAD(P)H Dehydrogenase (EC 1.-; wie z.B. EC 1.1, insbesondere EC 1.1.1) mit Cholsäuretoleranz; und
b) einen Redox-Mediator, welche die Fähigkeit besitzt, Elektronen, die von NAD(P)H stammen, auf molekularen Sauerstoff zu übertragen und umgekehrt, wobei der Mediator insbesondere einen $E_0$-Wert aufweist, der größer als der $E_0$-Wert der NAD(P)H Dehydrogenase und kleiner als der $E_0$-Wert von molekularen Sauerstoff (oder präziser $O_2/H_2O_2$) ist;

wobei die Regenerierung insbesondere in Gegenwart von Sauerstoff und/oder Wasserstoffperoxid, je nach dem ob der zu regenerierende Cofaktor zu oxidieren oder zu reduzieren ist, erfolgt. Ist beispielsweise der zu regenerierende Cofaktor $NAD^+$ oder $NADP^+$, so werden Elektronen von der reduzierten Cofaktorform (NADH bzw. NADPH) über den Mediator auf Sauerstoff unter Bildung von Wasserstoffperoxid übertragen. Die gegenläufige Reaktion, Regenerierung von NADPH bzw. NADH unter Verbrauch von Wasserstoffperoxid ist mit umfasst. Somit können $O_2$ und/oder $H_2O_2$ grundsätzlich auch als weiterer Bestandteil des Systems angesehen werden.

2. Ein Cofaktorregenerierungssystem nach Ausführungsform 1, wobei die NAD(P)H Dehydrogenase ausgewählt ist unter natürlichen oder rekombinanten, isolierten oder angereicherten Flavodoxin-Reduktasen (FDR; EC 1.18.1.2), NADPH Dehydrogenasen aus *G. stearothermophilus* Sulfit Reduktasen (SR; EC1.8.1.2), Reduktase Domänen von CYP102A1 (Cytochrom-P450-BM3-Reduktasen) (EC1.6.2.4), NAD(P) H-Flavin Reduktase (EC 1.5.1.29 / 1.16.1.3) und davon abgeleiteten funktionalen Äquivalenten, wie insbesondere Domänen davon.

3. Ein Cofaktorregenerierungssystem nach Ausführungsform 1 oder 2, wobei die Redoxäquivalente einen $E_0$-Wert kleiner als der $E_0$-Wert der Dehydrogenase besitzen und/oder wobei die Redoxäquivalente insbesondere ausgewählt sind unter NADH, $NAD^+$, NADPH und $NADP^+$.

4. Ein Cofaktorregenerierungssystem nach einer der vorhergehenden Ausführungsformen, wobei der Mediator ausgewählt ist unter Methylenblau, Dichlorphenolindophenol (DCIP), Ferricyanid, Riboflavin und Indigocarmin.

5. Ein Cofaktorregenerierungssystem, nach einer der vorhergehenden Ausführungsformen, wobei die NAD(P)H Dehydrogenase ein natürliches oder rekombinantes, isoliertes oder angereichertes Enzym pro- oder eukaryotischen Ursprungs, oder ein davon abgeleitetes funktionales Äquivalent ist.

6. Ein (nicht beanspruchtes) Verfahren zur Cofaktorregenerierung, wobei man eine Redoxreaktion durchführt, bei welcher unter Verbrauch wenigstens eines Redox-Äquivalents ein Substrat zum korrespondierenden oxidierten oder reduzierten Produkt umgesetzt wird, und man den verbrauchten Cofaktor unter Verwendung eines Cofaktorregenerierungssystems nach einer der vorhergehenden Ausführungsformen regeneriert.

7. Ein Verfahren nach Ausführungsform 6, wobei das umgesetzte Substrat eine Steroidverbindung (insbesondere ein Hydroxy- oder Ketosteroid) ist, wobei die Redoxreaktion z.B. in wenigstens einer der Ringpositionen 3, 7 und 12 erfolgen kann.

8. Ein Verfahren nach Ausführungsform 7, wobei das Substrat ausgewählt ist unter Cholsäuren (CS) und 12-Ketochenodesoxycholsäure (12-Keto CDCS) der folgenden Formeln (2) bzw. (3)

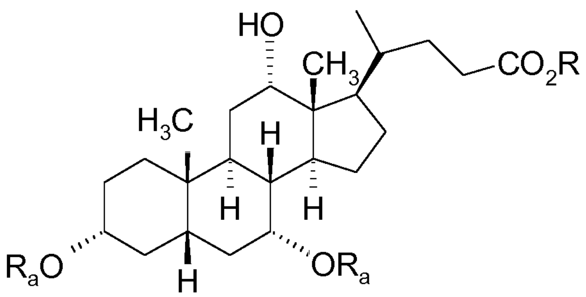

(2)

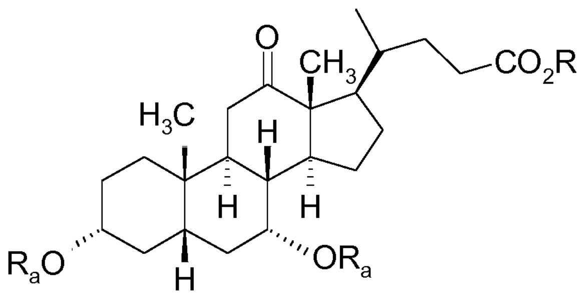

(3)

worin
R für Alkyl, H, ein Alkalimetallion oder $N(R^3)_4^+$steht, worin die Reste $R^3$ gleich oder verschieden sind und für H oder Alkyl stehen, und
die Reste $R_a$ gleich oder verschieden sind und für H oder Acyl stehen.

9. Ein Verfahren nach Ausführungsform 7, wobei die Steroidverbindung mittels einer Hydroxysteroiddehydrogenase (HSDH) (insbesondere einer 12α-HSDH) umgesetzt wird.

10. Ein Verfahren nach Ausführungsform 9, wobei die HSDH eine gereinigte 12α-Hydroxysteroiddehydrogenase (12α-HSDH) erhältlich aus *Clostridium* sp. mit einem Molekulargewicht im Bereich von etwa 27 bis 30 kD ist.

11. Ein Verfahren nach Ausführungsform 10 wobei 12α-Hydroxysteroiddehydrogenase erhältlich ist aus *Clostridium* sp. group P strain 48-50 (DSM4029).

12. Ein Verfahren nach Ausführungsform 11, wobei die 12α-HSDH mit einer spezifischen Aktivität im Bereich von mehr als etwa 10 U/mg verwendet wird.

13. Ein Verfahren nach einer der Ausführungsformen 9 bis 12, wobei eine 12α-Hydroxysteroiddehydrogenase, umfassend wenigstens eines der folgenden Sequenzmotive:

a) LINN (SEQ ID NO:5)
b) RMGIFD (SEQ ID NO: 11)
c) N-terminale Sequenz, ausgewählt unter

(1) MDFIDFKEMGRMGIFDGKVAIITGGGKAKSIGYGIAVAYAK (SEQ ID NO: 6)
(2) MDFIDFKEMGRMGI (SEQ ID NO:7)
(3) ITGGGKAKSIGYGIA (SEQ ID NO:8)
(4) IFDGK (SEQ ID NO: 9)
(5) GIFDGK (SEQ ID NO: 10)

d) FGDPELDI (SEQ ID NO:13)

verwendet wird.

14. Ein Verfahren nach einer der Ausführungsformen 9 bis 13, wobei eine 12α-Hydroxysteroiddehydrogenase,

a) umfassend eine der Aminosäuresequenzen gemäß SEQ ID NO: 2 oder 4, jeweils beginnend bei Position +1 oder +2; oder
b) umfassend eine von einer Sequenz gemäß a) abgeleiteten Aminosäuresequenz mit einer prozentualen Sequenz-Identität von wenigsten 60 %; oder
c) kodiert von einer ein Protein gemäß a) und b) kodierenden Nukleinsäuresequenz; oder
d) kodiert von einer kodierenden Nukleinsäuresequenz gemäß SEQ ID NO: 1 oder 3; oder

e) kodiert von einer der Nukleinsäuresequenzen gemäß SEQ ID NO: 1 oder 3 abgeleiteten kodierenden Sequenz mit einer prozentualen Sequenz-Identität von wenigstens 60 %, verwendet wird.

15. Ein Verfahren nach einer der Ausführungsformen 9 bis 14, wobei eine 12α-Hydroxysteroiddehydrogenase-Mutante mit modifizierter Cosubstrat-Nutzung verwendet wird, wobei die Mutante z.B. abgeleitet ist von einer 12α-Hydroxysteroiddehydrogenase gemäß obiger Definition, mit wenigstens einer die Cosubstrat-Nutzung modifizierenden Mutation im Sequenzmotiv VLTGRNE (SEQ ID NO: 12).

16. Ein Verfahren nach Ausführungsform 13, wobei eine 12α-Hydroxysteroiddehydrogenase, erhältlich durch heterologe Expression einer 12α-Hydroxysteroiddehydrogenase-kodierenden Nukleinsäuresequenz gemäß Ausführungsform 14 c), d) oder e) verwendet wird.

17. Ein Verfahren nach Ausführungsform 16, wobei eine 12α-Hydroxysteroiddehydrogenase, exprimiert in einem nicht-pathogenen Mikroorganismus, insbesondere einem Bakterium der Gattung *Escherichia,* insbesondere der Spezies *E. coli,* verwendet wird.

18. Ein Verfahren zur enzymatischen Oxidation von 12α-Hydroxysteroiden, wobei man das Hydroxysteroid in Gegenwart einer 12α-Hydroxysteroiddehydrogenase, insbesondere gemäß der Definition in einer der Ausführungsformen 10 bis 15 und in Gegenwart eines Cofaktorregenerierungssystems gemäß der Definition in einer der Ausführungsformen 1 bis 5 umsetzt, und wenigstens ein gebildetes Oxidationsprodukt aus dem Reaktionsansatz gegebenenfalls isoliert. Dieses Verfahren ist erfindungsgemäß, wenn das 12α-Hydroxysteroid der allgemeinen Formel

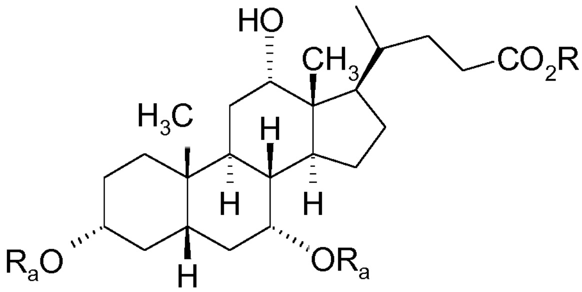

(2)

entspricht, worin
R für Alkyl, H, ein Alkalimetallion oder $N(R^3)_4^+$ steht, worin die Reste $R^3$ gleich oder verschieden sind und für H oder Alkyl stehen, und
die Reste $R_a$ gleich oder verschieden sind und für H oder Acyl stehen,
und das Cofaktorregenerierungssystem

a) eine NAD(P)H Dehydrogenase mit Cholsäuretoleranz, ausgewählt unter Flavodoxin Reduktasen (FDR; EC 1.18.1.2), NADPH Dehydrogenase aus G. *stearothermophilus* Sulfit Reduktasen (SR; EC 1.8.1.2), und Reduktase Domänen von CYP102A1 (EC 1.6.2.4) und davon abgeleiteten funktionalen Äquivalenten, die zur Cofaktorregenerierung befähigt sind und eine Sequenzidentität von wenigstens 85 % zur nativen Sequenz besitzen; und
b) einen Redox-Mediator, welcher Elektronen auf molekularen Sauerstoff überträgt, umfasst.

19. Ein Verfahren nach Ausführungsform 18, wobei das Hydroxysteroid Cholsäure (CS) oder eine Cholsäurederivat, wie insbesondere ein Salz, Amid oder Alkylester, ist.

20. Ein Verfahren nach Ausführungsform 19, wobei CS oder ein Derivat davon zu 12-Ketochenodesoxycholsäure (12-Keto-CDCS) oder zum entsprechenden Derivat umgesetzt wird.

21. Ein Verfahren nach einer der Ausführungsformen 18 bis 20, wobei die Reaktion in Gegenwart von $NAD(P)^+$ erfolgt.

22. Ein (nicht beanspruchtes) Verfahren zur enzymatischen Reduktion von 12-Ketosteroiden, wobei man das Ketosteroid in Gegenwart einer 12α-Hydroxysteroiddehydrogenase, insbesondere gemäß der Definition in einer der Ausführungsformen 10 bis 15, und in Gegenwart eines Cofaktorregenerierungssystems gemäß der Definition in einer der Ausführungsformen 1 bis 5 umsetzt, und ein gebildetes Reduktionsprodukt aus dem Reaktionsansatz gegebenenfalls isoliert.

23. Ein Verfahren nach Ausführungsform 22, wobei das Ketosteroid 12-Keto-CDCS oder ein Derivat davon, wie insbesondere ein Salz, Amid oder Alkylester, ist.

24. Ein Verfahren nach einer der Ausführungsformen 22 und 23, wobei das Ketosteroid oder dessen Derivat zum korrespondierenden 12α-Hydroxysteroid oder dessen Derivat reduziert wird.

25. Ein Verfahren nach einer der Ausführungsformen 22 bis 24, wobei die Reaktion in Gegenwart von NAD(P)H erfolgt.

26. Ein Verfahren nach einer der Ausführungsformen 18 bis 25, wobei die Reaktion mit einer NAD(P)H-Dehydroxygenase und/oder einer 12α-Hydroxysteroiddehydrogenase in immobilisierter Form erfolgt.

27. Ein beanspruchtes Verfahren zur Herstellung von Ursodesoxycholsäure (UDCS) der Formel (1)

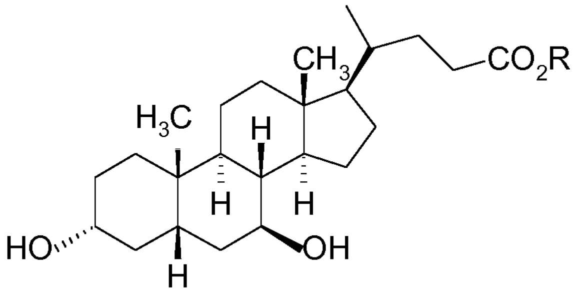

(1)

worin
R für Alkyl, H, ein Alkalimetallion oder $N(R^3)_4^+$steht, worin die Reste $R^3$ gleich oder verschieden sind und für H oder Alkyl stehen,
wobei man

a) eine Cholsäure (CS) der Formel (2)

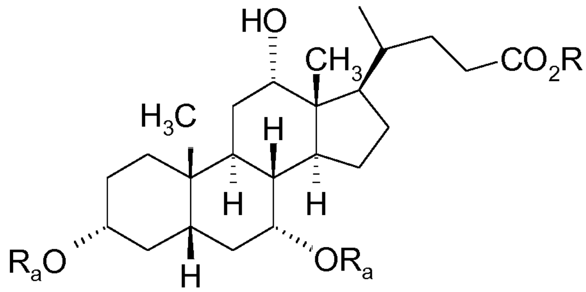

(2)

worin R die oben angegebenen Bedeutungen besitzen, und die Reste $R_a$ gleich oder verschieden sind und für H oder Acyl stehen, in Gegenwart einer 12α-Hydroxysteroiddehydrogenase, insbesondere gemäß der Definition in einer der Ausführungsformen 10 bis 15, und in Gegenwart eines Cofaktorregenerierungssystems gemäß der Definition in einer der Ausführungsformen 1 bis 5 zur korrespondierenden 12-Ketochenodesoxycholsäure (12-Keto CDCS) der Formel (3)

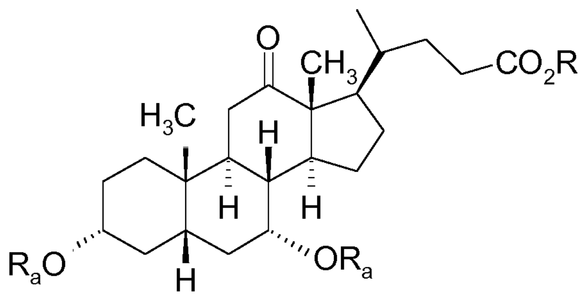

(3)

worin R und $R_a$ die oben angegebenen Bedeutungen besitzen, oxidiert und anschließend

b) 12-Keto-CDCS der Formel (3) durch Desoxygenierung zu Chenodesoxycholsäure (CDCS) der Formel (4)

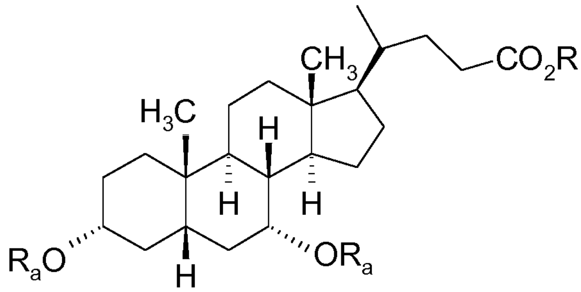

(4)

worin R und $R_a$ die oben angegebenen Bedeutungen besitzen, umsetzt und

c) CDCS der Formel (4) in Position 7 chemisch oxidiert zur 7-Keto-Lithocholsäure (KLCS) der Formel (5)

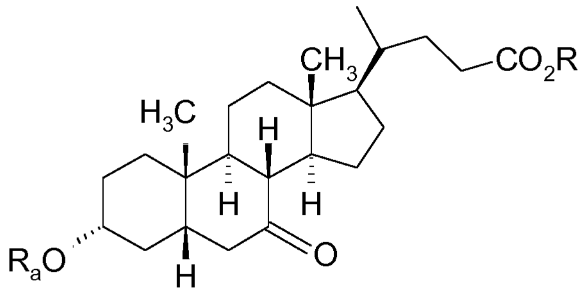

(5)

worin R und $R_a$ die oben angegebenen Bedeutungen besitzen; und

d) KLCS der Formel (5) reduziert und

e) das Reaktionsprodukt gegebenenfalls weiter aufreinigt.
Dieses Verfahren ist erfindungsgemäß, wenn das Cofaktorregenerierungssystem

a) eine NAD(P)H Dehydrogenase mit Cholsäuretoleranz, ausgewählt unter Flavodoxin Reduktasen (FDR; EC 1.18.1.2), NADPH Dehydrogenase aus G. *stearothermophilus* Sulfit Reduktasen (SR; EC 1.8.1.2), und Reduktase Domänen von CYP102A1 (EC 1.6.2.4) und davon abgeleiteten funktionalen Äquivalenten, die zur Cofaktorregenerierung befähigt sind und eine Sequenzidentität von wenigstens 85 % zur nativen Sequenz besitzen; und
b) einen Redox-Mediator, welcher Elektronen auf molekularen Sauerstoff überträgt, umfasst,

und, wenn $R_a$ für Acyl steht, diese Acylgruppe nach Durchführung der Reaktionsstufe b) oder d) gegebenenfalls abspaltet.

28. Ein Verfahren nach Ausführungsform 27, wobei wenn $R_a$ für Acyl steht, diese Acylgruppe nach Durchführung der Reaktionsstufe b) oder d) gegebenenfalls abspaltet.

29. Ein Verfahren nach einer der Ausführungsformen 27 und 28, wobei Stufe a) in Gegenwart von $NAD(P)^+$ erfolgt.

30. Ein Verfahren nach einer der Ausführungsformen 27 bis 29, wobei Stufe a) mit einer NAD(P)-Dehydrogenase und/oder einer 12$\alpha$-Hydroxysteroiddehydrogenase in immobilisierter Form erfolgt.

**2. Allgemeine Definitionen**

Der Begriff "Cholsäuretoleranz" beschreibt die Fähigkeit erfindungsgemäß eingesetzter NAD(P)H Dehydrogenasen in Gegenwart von Cholsäure, insbesondere unter Standardbedingungen, eine NAD(P)H abhängige Redoxreaktion zu katalysieren, wobei diese Redoxreaktion mit einem von Cholsäure oder dessen Oxidationsprodukten verschiedenen Substrat oder Elektronenakzeptor durchgeführt wird. Die zugegebene Cholsäuremenge bleibt während des Tests im Wesentlichen konstant. Dabei kann die Enzymaktivität, verglichen mit der unter ansonsten gleichen Bedingungen, jedoch

in Abwesenheit von Cholsäure bestimmten Aktivität prozentual größer oder geringer sein. So kann die Aktivität in Gegenwart von Cholsäure z.B. um 0,1 bis 1000%, wie z.B. 1 bis 500, 2 bis 400, 3 bis 300, 5 bis 200, 10 bis 100 oder 28 bis 80 % erhöht sein. Andererseits kann die Aktivität in Gegenwart von Cholsäure z.B. auf 10 bis 99,9, 20 bis 99, 30 bis 98, 40 bis 97, 50 bis 96, 60 bis 95, 70 bis 94 oder 80 bis 90 % erniedrigt sein. Beispielsweise kann die Enzymaktivität in Gegenwart von Cholsäure in einem Bereich liegen, der von einer um 10, 20, 50 oder 100 % erhöhten Aktivität bis zu einer auf etwa 10 %, 20 %, 30 % oder 40 % erniedrigten Aktivität reicht. Unter "Standardbedingungen" versteht man dabei insbesondere eine Aktivitätsmessung in Gegenwart von 0,1 M Cholsäure, bei einem pH Wert von etwa 7,5 bis 8,5, insbesondere etwa pH = 8,0. Die genannten Standardbedingungen sind weiterhin definierbar durch eine Temperatur im Bereich von etwa 15 bis 30, insbesondere etwa 20 °C sowie 50 bis 250, insbesondere etwa 1-100 $\mu$M, wie z.B. 10 $\mu$M NADH oder insbesondere NADPH. Die Konzentration des Substrats oder Elektronenakzeptors kann z.B. im Bereich von 5 $\mu$M bis 3 mM liegen, wie z.B. 0,3 bis 30mM, insbesondere 3 mM Methylenblau oder 5 bis 100 $\mu$M, insbesondere100 $\mu$M Riboflavin.

Der Begriff "HSDH" bezeichnet allgemein kurzkettige Alkohol Dehydrogenasen, welche hohe Aktivitäten bei hohen Substratkonzentrationen (wie z.B. 0,1 bis 0,5 M) bei pH-Werten von etwa 7,5 bis 9 zeigen können. Hierin wird insbesondere die 12$\alpha$-Oxidationen der Cholsäure durch das Enzym 12$\alpha$-HSDH untersucht. Die erfindungsgemäße Lehre, insbesondere die Befunde zur Cofaktorregenerierung können jedoch auch auf andere HSDHs übertragen werden, wie z.B. 7$\alpha$-HSDH und 3$\alpha$-HSDH.

Werden keine anderen Angaben gemacht, so bezeichnet der Begriff "12 $\alpha$-HSDH" ein Dehydrogenaseenzym, welches wenigstens die stereospezifische Oxidation von Cholsäure zu 12-Keto-Chenodesoxycholsäure unter stöchiometrischem Verbrauch von $NAD^+$ bzw. $NADP^+$ katalysiert. Das Enzym kann dabei ein natives bzw. rekombinant hergestelltes Enzym sein. Das Enzym kann grundsätzlich im Gemisch mit zellulären, wie z.B. Proteinverunreinigungen, vorzugsweise aber in Reinform vorliegen.

Der Begriff "NAD(P)H-Dehydrogenase" (Synonym: NAD(P)H-Diaphorase) ist die Sammelbezeichnung für Enzyme mit NAD(P)H: Akzeptor Oxidoreduktase-Aktivität.

Werden keine anderen Angaben gemacht, so bezeichnet der Begriff "NAD(P)H-Dehydrogenase" im Rahmen der Erfindung ein Dehydrogenaseenzym, welches wenigstens die Oxidation von NADH oder NADPH unter stöchiometrischer Übertragung von Elektronen auf einen Akzeptor unter Bildung von $NAD^+$ bzw. $NADP^+$ katalysiert. Das Enzym kann dabei ein natives bzw. rekombinant hergestelltes Enzym sein. Das Enzym kann grundsätzlich im Gemisch mit zellulären, wie z.B. Proteinverunreinigungen, vorzugsweise aber in Reinform vorliegen.

Der Begriff "NAD(P)H Dehydrogenase" ist insbesondere als Sammelbegriff für erfindungsgemäß geeignete Enzyme zu verstehen, welche die Fähigkeit besitzen, Elektronen von NAD(P)H auf einen Redoxmediator zu übertragen welche diese dann z.B. an molekularen Sauerstoff abgeben kann. Zudem sind derartige Dehydrogenasen insbesondere durch eine Cholsäuretoleranz, wie oben definiert, gekennzeichnet. Spezielle Beispiele umfassen die folgenden Enzyme:

Flavodoxin Reduktasen (FDR),
Sulfit Reduktasen (SR),
Reduktase Domäne der CYP102A1, und
NAD(P)H-Flavin Reduktase.

Der Begriff "Flavodoxin Reduktase" steht dabei für Enzyme der Klasse EC 1.18.1.2, welche die Fähigkeit besitzen, die Redoxreaktion:

$$\text{Flavodoxin(reduziert)} + NADP^+ \rightarrow \text{Flavodoxin(oxidiert)} + NADPH + H^+$$

oder die entsprechende Umkehrreaktion zu katalysieren.

Der Begriff "Sulfit Reduktase" steht dabei für Enzyme der Klasse EC 1.8.1.2 welche die Fähigkeit besitzen, die Redoxreaktion:

$$\text{Hydrogensulfid} + 3\ NADP^+ + 3\ H_2O \rightarrow \text{Sulfit} + 3\ NADPH + 3\ H^+$$

oder die entsprechende Umkehrreaktion zu katalysieren.

Der Begriff "Reduktase Domäne der CYP102A1 steht dabei für Enzyme der Klasse EC 1.6.2.4, welche die Fähigkeit besitzen die Redoxreaktion:

$$NADPH + 2\ \text{Ferricytochrom} \rightarrow NADP^+ + 2\ \text{Ferrocytochrom} + H^+$$

oder die entsprechende Umkehrreaktion zu katalysieren.

Der Begriff "NAD(P)H-Flavin Reduktase" steht dabei für Enzyme der Klasse EC 1.5.1.29 bzw. 1.16.1.3 welche

die Fähigkeit besitzen, die Redoxreaktionen:

$$2\ \text{Cob(II)alamin} + NAD^+ \rightarrow 2\ \text{Aquacob(III)alamin} + NADH + H^+\ \text{(für EC 1.16.1.3)}$$

$$FMNH_2 + NAD(P)^+ = FMN + NAD(P)H + H^+\ \text{( für 1.5.1.29)}$$

oder die entsprechende Umkehrreaktion zu katalysieren.

Unter einer "Reinform" oder einem "reinen" oder "im Wesentlichen reinen" Enzym versteht man erfindungsgemäß ein Enzym mit einem Reinheitsgrad von mehr als 80, vorzugsweise mehr als 90, insbesondere mehr als 95, und vor allem mehr als 99 Gew.-%, bezogen auf den Gesamtproteingehalt, bestimmt mit Hilfe üblicher Proteinnachweismethoden, wie z.B. der Biuret-Methode oder dem Proteinnachweis nach Lowry.et al. (vgl Beschreibung in R.K. Scopes, Protein Purification, Springer Verlag, New York, Heidelberg, Berlin (1982)). Die spezifische Aktivität eines erfindungsgemäß eingesetzten 12α-HSDH-Enzyms liegt dabei in dem oben angegebenen Bereich.

Unter einem "Redoxäquivalent" versteht man eine als Elektronendonor bzw. Elektronenakzeptor brauchbare, niedermolekulare organische Verbindung, wie beispielsweise Nikotinamidderivate wie $NAD^+$ und $NAPH^+$ bzw. deren reduzierte Formen NADH bzw. NADPH. "Redoxäquivalent" und "Cofaktor" werden im Kontext der vorliegenden Erfindung als Synonym verwendet. So kann ein "Cofaktor" im Sinne der Erfindung auch als "redoxfähiger Cofaktor", d.h. als Cofaktor, der in reduzierter und einer oxidierter Form vorliegen kann, umschrieben werden.

Unter einem "verbrauchten" Cofaktor versteht man diejenige reduzierte bzw. oxidierte Form des Cofaktors, die im Verlauf einer vorgegebene Reduktions- bzw. Oxidationsreaktion eines Substrats in die korrespondierende oxidierte bzw. reduzierte Form überführt wird. Durch Regenerierung wird die bei der Reaktion gebildete oxidierte bzw. reduzierte CofaktorForm wieder in die reduzierte bzw. oxidierte Ausgangsform zurück überführt, so dass diese für die Umsetzung des Substrats wieder zur Verfügung steht.

"In Gegenwart von (molekularem) Sauerstoff" bedeutet z.B. in Gegenwart von reinem molekularen Sauerstoff oder eines Sauerstoff-haltigen Gasgemisches, wie z.B. Luft. Dazu kann die Reaktion in einer Sauerstoff-haltigen Umgebung durchgeführt werden, so dass Sauerstoff z.B. in das Reaktionsmedium diffundiert. Sauerstoff oder das Sauerstoffhaltige Gasgemisch können aber auch in das Reaktionsmedium eingeleitet werden.

Unter einer Verbindung eines speziellen Typs, wie z.B. einer "Cholsäure-Verbindung" oder einer "Ursodesoxycholsäure-Verbindung" versteht man insbesondere auch Derivate der zugrunde liegenden Ausgangsverbindung (wie z.B. Cholsäure oder Ursodesoxycholsäure).

Derartige Derivate umfassen "Salze", wie z.B. Alkalimetallsalze wie Lithium-, Natrium- und Kaliumsalze der Verbindungen; sowie Ammoniumsalze, wobei man unter einem Ammoniumsalz das $NH_4^+$-Salz bzw. solche Ammoniumsalze umfasst, worin wenigstens ein Wasserstoffatom durch einen $C_1$-$C_6$-Alkylrest ersetzt sein kann. Typische Alkylreste sind insbesondere $C_1$-$C_4$-Alkylreste, wie Methyl, Ethyl, n- oder i-Propyl-, n-, sec- oder tert-Butyl, sowie n-Pentyl und n-Hexyl und die ein- oder mehrfach verzweigten Analoga davon

"Alkylester" erfindungsgemäß hergestellter Verbindungen sind insbesondere Niedrigalkylester, wie z.B. $C_1$-$C_6$-Alkylester. Als nicht limitierende Beispiele sind zu nennen Methyl-, Ethyl-, n- oder i-Propyl-, n-, sec- oder tert-Butylester, oder längerkettige Ester, wie z.B. n-Pentyl- und n-Hexylester sowie die ein- oder mehrfach verzweigten Analoga davon.

"Amide" sind insbesondere Umsetzungsprodukte erfindungsgemäß hergestellter Säuren mit Ammoniak oder primären oder sekundären Monoaminen. Derartige Amine sind beispielsweise Mono- oder Di-$C_1$-$C_6$-Alkyl-monoamine, wobei die Alkylreste unabhängig voneinander gegebenenfalls weiter substituiert sein können, wie z.B. durch Carboxy-, Hydroxy-, Halogen (wie F, Cl, Br, J)-, Nitro- und Sulfonatgruppen.

"Acylgruppen" sind insbesondere nichtaromatische Gruppen mit 2 bis 4 Kohlenstoffatomen, wie z.B. Acetyl, Propionyl und Butyryl, sowie aromatische Gruppen mit gegebenenfalls substituiertem einkernigen aromatischem Ring, wobei geeignete Substituenten z.B. ausgewählt sind unter Hydroxy-, Halogen (wie F, Cl, Br, J)-, Nitro- und $C_1$-$C_6$-Alkylgruppen, wie z.B. Benzoyl oder Toluoyl.

Die erfindungsgemäß eingesetzten bzw. hergestellten Hydroxysteroidverbindungen, wie Cholsäure, Glykocholsäure, Taurocholsäure Ursodesoxycholsäure, 12-Keto-Chenodesoxycholsäure, Chenodesoxycholsäure und 7-Keto-Lithocholsäure können in stereoisomerenreiner Reinform oder im Gemisch mit anderen Stereoisomeren im erfindungsgemäßen Verfahren eingesetzt oder daraus erhalten werden. Vorzugsweise werden jedoch die eingesetzten bzw. die hergestellten Verbindungen in im Wesentlichen stereoisomerenreiner Form eingesetzt bzw. isoliert.

Unter einer "Immobilisierung" versteht man erfindungsgemäß die kovalente oder nicht-kovalente Bindung eines erfindungsgemäß verwendeten Biokatalysators, wie z.B. einer 12α-HSDH an einem festen, d.h. in dem umgebenden flüssigen Medium im Wesentlichen unlöslichen, Trägermaterial.

"Poduktinhibition" der 12α-HSDH beschreibt die Verringerung der enzymatischen Aktivität in Gegenwart eines bei einer durch das Enzym katalysierten enzymatischen Umsetzung gebildeten Produkts. Bei Umsetzung zu CS ist so z.B. eine Inhibition durch 12-Keto-CDCS zu beobachten. Eine "Verringerung der Produktinhibition" beschreibt die verringerte prozentuale Abnahme der Enzymaktivität einer erfindungsgemäß eingesetzten Enzymmutante im Vergleich zu einem Referenzsystem, wie z.B. dem nativen HSDH-Enzym, jeweils bezogen auf die bei 0 % Umsatz (entsprechend 5mM CS) ermittelte Enzymaktivität als 100 %-Aktivitätswert. Diese Verringerung kann, wie im experimentellen Teil, bzw. in der Legende zu Figur 9 beschrieben, bestimmt werden. Verringerungen der Produktinhibition können auch über das Verhältnis der Restaktivitäten von Mutante zu Referenzenzym jeweils bestimmt bei gleichem prozentualen Umsatz, ausgedrückt werden. So kann die ererfindungsgemäß eingesetzte Mutante eine um den Faktor 1,1 bis 100, wie z.B. 1,5 bis 20 oder 2 bis 10 erhöhte Aktivität besitzen.

Der "$E_0$-Wert" ist definiert als Normalpotential eines Redox-Paares von chemischen Individuen, bestimmt mit einer Normal-Wasserstoffelektrode als Referenz.. Insbesondere ist $E_0$ durch das relative Potential einer Normal-Wasserstoffelektrode bei einer 1 M Konzentration der oxidierten und reduzierten Ionen, die an der Reaktion beteiligt sind bei 25°C definiert.

**3. Weitere Ausgestaltungen der Erfindung**

**3.1 Proteine**

Die vorliegende Erfindung ist nicht auf Verfahren mit den hierin konkret offenbarten Proteinen bzw. Enzymen mit 12α-HSDH-Aktivität oder NAD(P)H Dehydrogenasen beschränkt, sondern erstreckt sich vielmehr auch auf Verfahren mit funktionalen Äquivalenten davon.

"Funktionale Äquivalente" oder Analoga der konkret offenbarten Enzyme sind im Rahmen der vorliegenden Erfindung davon verschiedene Polypeptide, welche weiterhin die gewünschte biologische Aktivität, wie z.B. 12α-HSDH-Aktivität oder NAD(P)H-Dehydrogenase-Aktivität besitzen.

So versteht man beispielsweise unter "funktionalen Äquivalenten" Enzyme, die im verwendeten Test auf 12α-HSDH-Aktivität oder NAD(P)H-Dehydrogenase-Aktivität eine um mindestens 1 %, wie z.B. mindestens 10 % oder 20 %, wie z.B. mindestens 50 % oder 75 % oder 90 % höhere oder niedrigere Aktivität eines Enzyms, umfassend eine hierin definierte Aminosäuresequenz aufweisen. Funktionale Äquivalente sind außerdem vorzugsweise zwischen pH 4 bis 11 stabil und besitzen vorteilhaft ein pH-Optimum in einem Bereich von pH 6 bis 10, wie insbesondere 8,5 bis 9,5, sowie ein Temperaturoptimum im Bereich von 15 °C bis 80 °C oder 20 °C bis 70 °C, wie z.B. etwa 45 bis 60 °C oder etwa 50 bis 55 °C.

Die 12α-HSDH-Aktivität kann mit Hilfe verschiedener bekannter Tests nachgewiesen werden. Ohne darauf beschränkt zu sein, sei ein Test unter Verwendung eines Referenzsubstrates, wie z. B. Cholsäure, unter standardisierten Bedingungen wie im experimentellen Teil definiert, zu nennen.

Die NAD(P)H-Dehydrogenase-Aktivität kann bestimmt werden nach Literaturangaben, z.B. in Green DE, Needham DM, Dewan JG. Biochem J. 1937 vol. 31(12):2327-52. Dismutations and oxidoreductions;
Flavodoxin Reduktase (FDR)-Aktivität kann bestimmt werden nach Literaturangaben, z.B. in Fujii K, Huennekens FM., J Biol Chem. 1974 vol. 249(21):6745-53.

Activation of methionine synthetase by a reduced triphosphopyridine nucleotide-dependent flavoprotein system.

Sulfit Reduktase (SR)-Aktivität kann bestimmt werden nach Literaturangaben, z.B. in Coves J, Niviere V, Eschenbrenner M, Fontecave M., J Biol Chem. 1993 vol. 268(25):18604-9. NADPH-sulfite reductase from Escherichia coli. A flavin reductase participating in the generation of the free radical of ribonucleotide reductase.

Cytochrom P450 BM3 Reduktase-Aktivität kann bestimmt werden nach Literaturangaben, z.B. in Narhi LO, Fulco AJ.. J Biol Chem. 1986 vol. 261(16):7160-9.

Characterization of a catalytically self-sufficient 119,000-dalton cytochrome P-450 monooxygenase induced by barbiturates in Bacillus megaterium. und
NAD(P)H-Flavin Reduktase-Aktivität kann bestimmt werden nach Literaturangaben, z.B. in Duane W, Hastings JW. Mol Cell Biochem. 1975 vol. 6(1):53-64.

Flavin mononucleotide reductase of luminous bacteria.

Unter "funktionalen Äquivalenten" versteht man erfindungsgemäß insbesondere auch "Mutanten", welche in wenigstens einer Sequenzposition der oben genannten Aminosäuresequenzen eine andere als die konkret genannte Aminosäure aufweisen aber trotzdem eine der oben genannten biologischen Aktivitäten besitzen. "Funktionale Äquivalente" umfassen somit die durch eine oder mehrere Aminosäure-Additionen, -Substitutionen, - Deletionen und/oder -Inversionen erhältlichen Mutanten, wobei die genannten Veränderungen in jeglicher Sequenzposition auftreten können, solange sie zu einer Mutante mit dem erfindungsgemäßen Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d.h. beispielsweise gleiche Substrate mit unterschiedlicher Geschwindigkeit umgesetzt werden. Beispiele für geeignete Aminosäuresubstitutionen sind in folgender Tabelle zusammengefasst:

| Ursprünglicher Rest | Beispiele der Substitution |
|---|---|
| Ala | Ser |
| Arg | Lys |
| Asn | Gln; His |
| Asp | Glu |
| Cys | Ser |
| Gln | Asn |
| Glu | Asp |
| Gly | Pro |
| His | Asn ; Gln |
| Ile | Leu; Val |
| Leu | Ile; Val |
| Lys | Arg ; Gln ; Glu |
| Met | Leu ; Ile |
| Phe | Met; Leu ; Tyr |
| Ser | Thr |
| Thr | Ser |
| Trp | Tyr |
| Tyr | Trp ; Phe |
| Val | Ile; Leu |

"Funktionale Äquivalente" im obigen Sinne sind auch "Präkursoren" der beschriebenen Polypeptide sowie "funktionale Derivate" und "Salze" der Polypeptide.

"Präkursoren" sind dabei natürliche oder synthetische Vorstufen der Polypeptide mit oder ohne die gewünschte biologische Aktivität.

Unter dem Ausdruck "Salze" versteht man sowohl Salze von Carboxylgruppen als auch Säureadditionssalze von Aminogruppen der im erfindungsgemäßen Verfahren verwendeten Proteinmoleküle. Salze von Carboxylgruppen können in an sich bekannter Weise hergestellt werden und umfassen anorganische Salze, wie zum Beispiel Natrium-, Calcium-, Ammonium-, Eisen- und Zinksalze, sowie Salze mit organischen Basen, wie zum Beispiel Aminen, wie Triethanolamin, Arginin, Lysin, Piperidin und dergleichen. Säureadditionssalze, wie zum Beispiel Salze mit Mineralsäuren, wie Salzsäure oder Schwefelsäure und Salze mit organischen Säuren, wie Essigsäure und Oxalsäure sind ebenfalls mit umfasst.

"Funktionale Derivate" der im erfindungsgemäßen Verfahren verwendeten Polypeptide können an funktionellen Aminosäure-Seitengruppen oder an deren N- oder C-terminalen Ende mit Hilfe bekannter Techniken ebenfalls hergestellt werden. Derartige Derivate umfassen beispielsweise aliphatische Ester von Carbonsäuregruppen, Amide von Carbonsäuregruppen, erhältlich durch Umsetzung mit Ammoniak oder mit einem primären oder sekundären Amin; N-Acylderivate freier Aminogruppen, hergestellt durch Umsetzung mit Acylgruppen; oder O-Acylderivate freier Hydroxygruppen, hergestellt durch Umsetzung mit Acylgruppen.

"Funktionale Äquivalente" umfassen natürlich auch Polypeptide welche aus anderen Organismen zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Enzyme ermitteln.

"Funktionale Äquivalente" umfassen ebenfalls Fragmente, vorzugsweise einzelne Domänen oder Sequenzmotive, der im erfindungsgemäßen Verfahren verwendeten Polypeptide, welche z.B. die gewünschte biologische Funktion aufweisen.

"Funktionale Äquivalente" sind außerdem Fusionsproteine, welche eine der oben genannten Polypeptidsequenzen oder davon abgeleitete funktionale Äquivalente und wenigstens eine weitere, davon funktionell verschiedene, heterologe Sequenz in funktioneller N- oder C-terminaler Verknüpfung (d.h. ohne gegenseitigen wesentliche funktionelle Beeinträchtigung der Fusionsproteinteile) aufweisen. Nichtlimitierende Beispiele für derartige heterologe Sequenzen sind z.B. Signalpeptide, Histidin-Anker oder Enzyme.

Erfindungsgemäß mit umfasste "funktionale Äquivalente" sind Homologe zu den konkret offenbarten Proteinen. Diese besitzen wenigstens 60 %, vorzugsweise wenigstens 75 %, insbesondere wenigstens 85 %, wie z.B. 90, 91, 92, 93, 94, 95, 96, 97,98 oder 99 %, Homologie (bzw. Identität) zu einer der konkret offenbarten, z.B. nativen, Aminosäuresequenzen, berechnet nach dem Algorithmus von Pearson und Lipman, Proc. Natl. Acad, Sci. (USA) 85(8), 1988, 2444-2448. Eine prozentuale Homologie bzw. Identität eines erfindungsgemäß verwendeten homologen Polypeptids

bedeutet insbesondere prozentuale Identität der Aminosäurereste, bezogen auf die Gesamtlänge einer der hierin konkret beschriebenen Aminosäuresequenzen.

Die prozentualen Identitätswerte können auch anhand von BLAST Alignments, Algorithmus blastp (protein-protein BLAST), oder durch Anwendung der unten angegebenen Clustal-Einstellungen ermittelt werden.

Im Falle einer möglichen Proteinglykosylierung umfassen im erfindungsgemäßen Verfahren verwendete "funktionale Äquivalente" Proteine des oben bezeichneten Typs in deglykosylierter bzw. glykosylierter Form sowie durch Veränderung des Glykosylierungsmusters erhältliche abgewandelte Formen.

Homologe der im erfindungsgemäßen Verfahren verwendeten Proteine oder Polypeptide können durch Mutagenese erzeugt werden, z.B. durch Punktmutation, Verlängerung oder Verkürzung des Proteins.

Homologe der im erfindungsgemäßen Verfahren verwendeten Proteine können durch Screening kombinatorischer Banken von Mutanten, wie z.B. Verkürzungsmutanten, identifiziert werden. Beispielsweise kann eine variegierte Bank von Protein-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen kodieren. Verfahren zur Synthese degenerierter Oligonukleotide sind dem Fachmann bekannt (z.B. Narang, S.A. (1983) Tetrahedron 39: 3; Itakura et al. (1984) Annu. Rev. Biochem. 53: 323; Itakura et al., (1984) Science 198: 1056; Ike et al. (1983) Nucleic Acids Res. 11: 477).

Im Stand der Technik sind mehrere Techniken zum Screening von Genprodukten kombinatorischer Banken, die durch Punktmutationen oder Verkürzung hergestellt worden sind, und zum Screening von cDNA-Banken auf Genprodukte mit einer ausgewählten Eigenschaft bekannt. Diese Techniken lassen sich an das schnelle Screening der Genbanken anpassen, die durch kombinatorische Mutagenese Homologer erzeugt worden sind. Die am häufigsten verwendeten Techniken zum Screening großer Genbanken, die einer Analyse mit hohem Durchsatz unterliegen, umfassen das Klonieren der Genbank in replizierbare Expressionsvektoren, Transformieren der geeigneten Zellen mit der resultierenden Vektorenbank und Exprimieren der kombinatorischen Gene unter Bedingungen, unter denen der Nachweis der gewünschten Aktivität die Isolation des Vektors, der das Gen kodiert, dessen Produkt nachgewiesen wurde, erleichtert. Recursive-Ensemble-Mutagenese (REM), eine Technik, die die Häufigkeit funktioneller Mutanten in den Banken vergrößert, kann in Kombination mit den Screeningtests verwendet werden, um Homologe zu identifizieren (Arkin und Yourvan (1992) PNAS 89: 7811-7815; Delgrave et al. (1993) Protein Engineering 6(3): 327-331).

### 3.2 Nukleinsäuren und Konstrukte

#### 3.2.1 Nukleinsäuren

Offenbart sind auch Nukleinsäuresequenzen, die für ein Enzym mit 12$\alpha$-HSDH-Aktivität oder NAD(P)H-Dehydrogenase-Aktivität kodieren.

Die vorliegende Offenbarung betrifft auch Nukleinsäuren mit einem bestimmten Identitätsgrad zu den hierin beschriebenen konkreten Sequenzen.

Unter "Identität" zwischen zwei Nukleinsäuren wird die Identität der Nukleotide über die jeweils gesamte Nukleinsäurelänge verstanden, insbesondere die Identität, die durch Vergleich mit Hilfe der Vector NTI Suite 7.1 Software der Firma Informax (USA) unter Anwendung der Clustal-Methode (Higgins DG, Sharp PM. Fast and sensitive multiple sequence alignments on a microcomputer. Comput Appl. Biosci. 1989 Apr; 5(2):151-1) unter Einstellung folgender Parameter berechnet wird:

Multiple alignment parameters:

| | |
|---|---|
| Gap opening penalty | 10 |
| Gap extension penalty | 10 |
| Gap separation penalty range | 8 |
| Gap separation penalty | off |
| % identity for alignment delay | 40 |
| Residue specific gaps | off |
| Hydrophilic residue gap | off |

(fortgesetzt)

| | |
|---|---|
| Transition weighing | 0 |

Pairwise alignment parameter:

| | |
|---|---|
| FAST algorithm | on |
| K-tuplesize | 1 |
| Gap penalty | 3 |
| Window size | 5 |
| Number of best diagonals | 5 |

Alternativ dazu kann die Identität auch nach Chenna, Ramu, Sugawara, Hideaki, Koike,Tadashi, Lopez, Rodrigo, Gibson, Toby J, Higgins, Desmond G, Thompson, Julie D. Multiple sequence alignment with the Clustal series of programs. (2003) Nucleic Acids Res 31 (13):3497-500, gemäß Internetadresse: http://www.ebi.ac.uk/Tools/clustalw/index.html# und mit den folgenden Parametern bestimmt werden:

| | |
|---|---|
| DNA Gap Open Penalty | 15.0 |
| DNA Gap Extension Penalty | 6.66 |
| DNA Matrix | Identity |
| Protein Gap Open Penalty | 10.0 |
| Protein Gap Extension Penalty | 0.2 |
| Protein matrix | Gonnet |
| Protein/DNA ENDGAP | -1 |
| Protein/DNA GAPDIST | 4 |

Alle hierin erwähnten Nukleinsäuresequenzen (einzel- und doppelsträngige DNA- und RNA-Sequenzen, wie z.B. cDNA und mRNA) sind in an sich bekannter Weise durch chemische Synthese aus den Nukleotidbausteinen, wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seiten 896-897) erfolgen. Die Anlagerung synthetischer Oligonukleotide und Auffüllen von Lücken mit Hilfe des Klenow-Fragmentes der DNA-Polymerase und Ligationsreaktionen sowie allgemeine Klonierungsverfahren werden in Sambrook et al. (1989), Molecular Cloning: A laboratory manual, Cold Spring Harbor Laboratory Press, beschrieben.

Offenbart sind auch Nukleinsäuresequenzen (einzel- und doppelsträngige DNA- und RNA-Sequenzen, wie z.B. cDNA und mRNA), kodierend für eines der obigen Polypeptide und deren funktionalen Äquivalente, welche z.B. unter Verwendung künstlicher Nukleotidanaloga zugänglich sind.

Die Offenbarung betrifft sowohl isolierte Nukleinsäuremoleküle, welche für im erfindungsgemäßen Verfahren verwendete Polypeptide bzw. Proteine oder biologisch aktive Abschnitte davon kodieren, als auch Nukleinsäurefragmente, die z.B. zur Verwendung als Hybridisierungssonden oder Primer zur Identifizierung oder Amplifizierung kodierender Nukleinsäuren verwendet werden können.

Die Nukleinsäuremoleküle können zudem untranslatierte Sequenzen vom 3'- und/oder 5'-Ende des kodierenden Genbereichs enthalten

Die Offenbarung umfasst weiterhin die zu den konkret beschriebenen Nukleotidsequenzen komplementären Nukleinsäuremoleküle oder einen Abschnitt davon.

Die Nukleotidsequenzen ermöglichen die Erzeugung von Sonden und Primern, die zur Identifizierung und/oder Klonierung von homologen Sequenzen in anderen Zelltypen und Organismen verwendbar sind. Solche Sonden bzw. Primer umfassen gewöhnlich einen Nukleotidsequenzbereich, der unter "stringenten" Bedingungen (siehe unten) an mindestens etwa 12, vorzugsweise mindestens etwa 25, wie z.B. etwa 40, 50 oder 75 aufeinanderfolgende Nukleotide eines Sense-Stranges einer Nukleinsäuresequenz oder eines entsprechenden Antisense-Stranges hybridisiert.

Ein "isoliertes" Nukleinsäuremolekül wird von anderen Nukleinsäuremolekülen abgetrennt, die in der natürlichen Quelle der Nukleinsäure zugegen sind und kann überdies im Wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

Ein Nukleinsäuremolekül kann mittels molekularbiologischer Standard-Techniken und der hierin bereitgestellten Sequenzinformation isoliert werden. Beispielsweise kann cDNA aus einer geeigneten cDNA-Bank isoliert werden, indem eine der konkret offenbarten vollständigen Sequenzen oder ein Abschnitt davon als Hybridisierungssonde und Standard-Hybridisierungstechniken (wie z.B. beschrieben in Sambrook, J., Fritsch, E.F. und Maniatis, T. Molecular Cloning: A Laboratory Manual. 2. Aufl., Cold Spring Harbor Laboratory, Cold Spring Harbor Laboratory Press, Cold Spring Harbor, NY, 1989) verwendet werden. Überdies lässt sich ein Nukleinsäuremolekül, umfassend eine der offenbarten Sequenzen oder ein Abschnitt davon, durch Polymerasekettenreaktion isolieren, wobei die Oligonukleotidprimer, die auf der Basis dieser Sequenz erstellt wurden, verwendet werden. Die so amplifizierte Nukleinsäure kann in einen geeigneten Vektor kloniert und durch DNA-Sequenzanalyse charakterisiert werden. Die Oligonukleotide können ferner durch Standard-Syntheseverfahren, z.B. mit einem automatischen DNA-Synthesegerät, hergestellt werden.

Nukleinsäuresequenzen oder Derivate davon, Homologe oder Teile dieser Sequenzen, lassen sich beispielsweise mit üblichen Hybridisierungsverfahren oder der PCR-Technik aus anderen Bakterien, z.B. über genomische oder cDNA-Banken, isolieren. Diese DNA-Sequenzen hybridisieren unter Standardbedingungen mit den hierin offenbarten Sequenzen.

Unter "hybridisieren" versteht man die Fähigkeit eines Poly- oder Oligonukleotids, an eine nahezu komplementäre Sequenz unter Standardbedingungen zu binden, während unter diesen Bedingungen unspezifische Bindungen zwischen nicht-komplementären Partnern unterbleiben. Dazu können die Sequenzen zu 90-100 % komplementär sein. Die Eigenschaft komplementärer Sequenzen, spezifisch aneinander binden zu können, macht man sich beispielsweise in der Northern- oder Southern-Blot-Technik oder bei der Primerbindung in PCR oder RT-PCR zunutze.

Zur Hybridisierung werden vorteilhaft kurze Oligonukleotide der konservierten Bereiche verwendet. Es können aber auch längere Fragmente der Nukleinsäuren oder die vollständigen Sequenzen für die Hybridisierung verwendet werden. Je nach der verwendeten Nukleinsäure (Oligonukleotid, längeres Fragment oder vollständige Sequenz) oder je nachdem welche Nukleinsäureart DNA oder RNA für die Hybridisierung verwendet werden, variieren diese Standardbedingungen. So liegen beispielsweise die Schmelztemperaturen für DNA:DNA-Hybride ca. 10 °C niedriger als die von DNA:RNA-Hybriden gleicher Länge.

Unter Standardbedingungen sind beispielsweise je nach Nukleinsäure Temperaturen zwischen 42 und 58 °C in einer wässrigen Pufferlösung mit einer Konzentration zwischen 0,1 bis 5 x SSC (1 X SSC = 0,15 M NaCl, 15 mM Natriumcitrat, pH 7,2) oder zusätzlich in Gegenwart von 50 % Formamid wie beispielsweise 42 °C in 5 x SSC, 50 % Formamid zu verstehen. Vorteilhafterweise liegen die Hybridisierungsbedingungen für DNA:DNA-Hybride bei 0,1 x SSC und Temperaturen zwischen etwa 20 °C bis 45 °C, bevorzugt zwischen etwa 30 °C bis 45 °C. Für DNA:RNA-Hybride liegen die Hybridisierungsbedingungen vorteilhaft bei 0,1 x SSC und Temperaturen zwischen etwa 30 °C bis 55 °C, bevorzugt zwischen etwa 45 °C bis 55 °C. Diese angegebenen Temperaturen für die Hybridisierung sind beispielhaft kalkulierte Schmelztemperaturwerte für eine Nukleinsäure mit einer Länge von ca. 100 Nukleotiden und einem G + C-Gehalt von 50 % in Abwesenheit von Formamid. Die experimentellen Bedingungen für die DNA-Hybridisierung sind in einschlägigen Lehrbüchern der Genetik, wie beispielsweise Sambrook et al., "Molecular Cloning", Cold Spring Harbor Laboratory, 1989, beschrieben und lassen sich nach dem Fachmann bekannten Formeln beispielsweise abhängig von der Länge der Nukleinsäuren, der Art der Hybride oder dem G + C-Gehalt berechnen. Weitere Informationen zur Hybridisierung kann der Fachmann folgenden Lehrbüchern entnehmen: Ausubel et al. (Hrsg.), 1985, Current Protocols in Molecular Biology, John Wiley & Sons, New York; Hames and Higgins (Hrsg.), 1985, Nucleic Acids Hybridization: A Practical Approach, IRL Press at Oxford University Press, Oxford; Brown (Hrsg.), 1991, Essential Molecular Biology: A Practical Approach, IRL Press at Oxford University Press, Oxford.

Die "Hybridisierung" kann insbesondere unter stringenten Bedingungen erfolgen. Solche Hybridisierungsbedingungen sind beispielsweise bei Sambrook, J., Fritsch, E.F., Maniatis, T., in: Molecular Cloning (A Laboratory Manual), 2. Auflage, Cold Spring Harbor Laboratory Press, 1989, Seiten 9.31-9.57 oder in Current Protocols in Molecular Biology, John Wiley & Sons, N.Y. (1989), 6.3.1-6.3.6 beschrieben.

Unter "stringenten" Hybridisierungs-Bedingungen werden insbesondere verstanden: Die Inkubation bei 42 °C über Nacht in einer Lösung bestehend aus 50 % Formamid, 5 x SSC (750 mM NaCl, 75 mM Tri-Natrium-citrat), 50 mM Natriumphosphat (pH 7,6), 5x Denhardt Lösung, 10 % Dextransulfat und 20 g/ml denaturierte, gescherte Lachsspermien-DNA, gefolgt von einem Waschschritt der Filter mit 0,1x SSC bei 65°C.

Offenbart sind auch Derivate der konkret offenbarten oder ableitbaren Nukleinsäuresequenzen.

So können weitere Nukleinsäuresequenzen z.B. von SEQ ID NOs:1, 3, 17, 23, 25, 27 oder 29 abgeleitet sein und sich davon durch Addition, Substitution, Insertion oder Deletion einzelner oder mehrerer Nukleotide unterscheiden, aber weiterhin für Polypeptide mit dem gewünschten Eigenschaftsprofil kodieren.

Offenbart sind auch solche Nukleinsäuresequenzen, die sogenannte stumme Mutationen umfassen oder entsprechend der Codon-Nutzung eines speziellen Ursprungs- oder Wirtsorganismus, im Vergleich zu einer konkret genannten Sequenz verändert sind, ebenso wie natürlich vorkommende Varianten, wie z.B. Spleißvarianten oder Allelvarianten, davon.

Gegenstand sind ebenso durch konservative Nukleotidsubstitutionen (d.h. die betreffende Aminosäure wird

durch eine Aminosäure gleicher Ladung, Größe, Polarität und/oder Löslichkeit ersetzt) erhältliche Sequenzen.

Offenbart sind auch die durch Sequenzpolymorphismen von den konkret offenbarten Nukleinsäuren abgeleiteten Moleküle. Diese genetischen Polymorphismen können zwischen Individuen innerhalb einer Population aufgrund der natürlichen Variation existieren. Diese natürlichen Variationen bewirken üblicherweise eine Varianz von 1 bis 5 % in der Nukleotidsequenz eines Gens.

Unter Derivaten der Nukleinsäuresequenz mit der Sequenz SEQ ID NOs:1, 3, 17, 23, 25, 27 oder 29 sind beispielsweise Allelvarianten zu verstehen, die mindestens 60 % Homologie auf der abgeleiteten Aminosäureebene, bevorzugt mindestens 80 % Homologie, ganz besonders bevorzugt mindestens 90 % Homologie über den gesamten Sequenzbereich aufweisen (bezüglich Homologie auf Aminosäureebene sei auf obige Ausführungen zu den Polypeptiden verwiesen auf). Über Teilbereiche der Sequenzen können die Homologien vorteilhaft höher liegen.

Weiterhin sind unter Derivaten auch Homologe der Nukleinsäuresequenzen, insbesondere der NOs:1, 3, 17, 23, 25, 27 oder 29, beispielsweise pilzliche oder bakterielle Homologe, verkürzte Sequenzen, Einzelstrang-DNA oder RNA der kodierenden und nichtkodierenden DNA-Sequenz, zu verstehen. So besitzen z.B. Homologe zur der SEQ ID NO:1 auf DNA-Ebene eine Homologie von mindestens 40 %, bevorzugt von mindestens 60 %, besonders bevorzugt von mindestens 70 %, ganz besonders bevorzugt von mindestens 80 % über den gesamten in SEQ ID NO:7 angegebenen DNA-Bereich.

Außerdem sind unter Derivaten beispielsweise Fusionen mit Promotoren zu verstehen. Die Promotoren, die den angegebenen Nukleotidsequenzen vorgeschaltet sind, können durch wenigstens einen Nukleotidaustausch, wenigstens eine Insertion, Inversion und/oder Deletion verändert sein, ohne dass aber die Funktionalität bzw. Wirksamkeit der Promotoren beeinträchtigt sind. Des Weiteren können die Promotoren durch Veränderung ihrer Sequenz in ihrer Wirksamkeit erhöht oder komplett durch wirksamere Promotoren auch artfremder Organismen ausgetauscht werden.

Dem Fachmann sind darüber hinaus Verfahren zur Erzeugung funktionaler Mutanten bekannt.

Je nach verwendeter Technik kann der Fachmann völlig zufällige oder auch gezieltere Mutationen in Gene oder auch nicht codierende Nukleinsäurebereiche (die beispielsweise für die Regulation der Expression wichtig sind) einbringen und anschließend Genbanken erstellen. Die dazu erforderlichen molekularbiologischen Methoden sind dem Fachmann bekannt und beispielsweise beschrieben in Sambrook und Russell, Molecular Cloning. 3. Auflage, Cold Spring Harbor Laboratory Press 2001.

Methoden zur Veränderung von Genen und somit zur Veränderung der durch diese codierten Proteine sind dem Fachmann seit langem geläufig, wie beispielsweise

- die ortsspezifische Mutagenese, bei der gezielt einzelne oder mehrere Nukleotide eines Gens ausgetauscht werden (Trower MK (Hrsg.) 1996; In vitro mutagenesis protocols, Humana Press, New Jersey),
- die Sättigungsmutagenese, bei der an jeder beliebigen Stelle eines Gens ein Codon für eine beliebige Aminosäure ausgetauscht oder hinzugefügt werden kann (Kegler-Ebo DM, Docktor CM, DiMaio D (1994) Nucleic Acids Res 22:1593; Barettino D, Feigenbutz M, Valcárel R, Stunnenberg HG (1994) Nucleic Acids Res 22: 541; Barik S (1995) Mol Biotechnol 3:1),
- die fehleranfällige Polymerase-Kettenreaktion (error-prone PCR), bei der Nukleotidsequenzen durch fehlerhaft arbeitende DNA-Polymerasen mutiert werden (Eckert KA, Kunkel TA (1990) Nucleic Acids Res 18: 3739);
- das Passagieren von Genen in Mutator-Stämmen, in denen beispielsweise aufgrund defekter DNA-Reparaturmechanismen eine erhöhte Mutationsrate von Nukleotidsequenzen auftritt (Greener A, Callahan M, Jerpseth B (1996) An efficient random mutagenesis technique u-sing an E.coli mutator strain. In: Trower MK (Hrsg.) In vitro mutagenesis protocols. Humana Press, New Jersey), oder
- das DNA-Shuffling, bei dem ein Pool aus nahe verwandten Genen gebildet und verdaut wird und die Bruchstücke als Templates für eine Polymerase-Kettenreaktion verwendet werden, bei der durch wiederholte Strangtrennung und Wiederannäherung letztendlich Mosaikgene voller Länge erzeugt werden (Stemmer WPC (1994) Nature 370: 389; Stemmer WPC (1994) Proc Natl Acad Sci USA 91: 10747).

Unter Anwendung der sogenannten gerichteten Evolution ("directed evolution"; beschrieben unter anderem in Reetz MT und Jaeger K-E (1999), Topics Curr Chem 200: 31; Zhao H, Moore JC, Volkov AA, Arnold FH (1999), Methods for optimizing industrial enzymes by directed evolution, In: Demain AL, Davies JE (Hrsg.) Manual of industrial microbiology and biotechnology. American Society for Microbiology) kann der Fachmann auch in gezielter Weise und auch in großem Maßstab funktionale Mutanten erzeugen. Dabei werden in einem ersten Schritt zunächst Genbanken der jeweiligen Proteine erzeugt, wobei beispielsweise die oben angegebenen Methoden zur Anwendung kommen können. Die Genbanken werden auf geeignete Weise exprimiert, beispielsweise durch Bakterien oder durch Phagen-Display-Systeme.

Die betreffenden Gene von Wirtsorganismen, die funktionale Mutanten mit Eigenschaften exprimieren, welche den gewünschten Eigenschaften weitgehend entsprechen, können einer weiteren Mutationsrunde unterworfen werden. Die Schritte der Mutation und der Selektion oder des Screening können iterativ solange wiederholt werden, bis die vorliegenden funktionalen Mutanten die gewünschten Eigenschaften in ausreichendem Maße aufweisen. Durch diese

iterative Arbeitsweise können stufenweise eine begrenzte Anzahl von Mutationen, wie z.B. 1 bis 5 Mutationen, vorgenommen und auf deren Einfluss auf die betreffende Enzymeigenschaft bewertet und selektiert werden. Die selektierte Mutante kann dann in gleicher Weise einem weiteren Mutationsschritt unterworfen werden. Dadurch lässt sich die Anzahl der zu untersuchenden Einzelmutanten signifikant verringern.

Die Ergebnisse liefern wichtige Informationen in Bezug auf Struktur und Sequenz der betreffenden Enzyme, die erforderlich sind, um gezielt weitere Enzyme mit gewünschten modifizierten Eigenschaften zu generieren. Insbesondere können sogenannte "hot spots" definiert werden, d.h. Sequenzabschnitte, die sich potentiell eignen, um über die Einführung gezielter Mutationen eine Enzymeigenschaft zu modifizieren.

Nicht limitierende Beispiele solcher Hot-spot-Regionen der erfindungsgemäß verwendeten HSDH sind im Folgenden zusammengefasst:

35-40, insbesondere 37-38, (jeweils bezogen auf die Aminosäuresequenz von HSDH_kurz (SEQ ID NO:4).

90-105, 93-100 oder 96-100, insbesondere 97 und/oder 98, (jeweils bezogen auf die Aminosäuresequenz von HSDH_kurz (SEQ ID NO:4)

### 3.2.2 Konstrukte

Offenbart sind außerdem Expressionskonstrukte, enthaltend unter der genetischen Kontrolle regulativer Nukleinsäuresequenzen eine für ein im erfindungsgemäßen Verfahren verwendetes Polypeptid kodierende Nukleinsäuresequenz; sowie Vektoren, umfassend wenigstens eines dieser Expressionskonstrukte.

Unter einer "Expressionseinheit" wird hierin eine Nukleinsäure mit Expressionsaktivität verstanden, die einen Promotor, wie hierin definiert umfasst, und nach funktioneller Verknüpfung mit einer zu exprimierenden Nukleinsäure oder einem Gen, die Expression, also die Transkription und die Translation dieser Nukleinsäure oder dieses Gens reguliert. Man spricht deshalb auch in diesem Zusammenhang von einer "regulativen Nukleinsäuresequenz". Zusätzlich zum Promotor können weitere, regulative Elemente, wie z.B. *Enhancer*, enthalten sein.

Unter einer "Expressionskassette" oder "Expressionskonstrukt" wird hierin eine Expressionseinheit verstanden, die mit der zu exprimierenden Nukleinsäure oder dem zu exprimierenden Gen funktionell verknüpft ist. Im Gegensatz zu einer Expressionseinheit umfasst eine Expressionskassette somit nicht nur Nukleinsäuresequenzen, welche Transkription und Translation regulieren, sondern auch die Nukleinsäuresequenzen, welche als Folge der Transkription und Translation als Protein exprimiert werden sollen.

Die Begriffe "Expression" oder "Überexpression" beschreiben im Kontext der Erfindung die Produktion bzw. Erhöhung der intrazellulären Aktivität eines oder mehrerer Enzyme in einem Mikroorganismus, die durch die entsprechende DNA kodiert werden. Dazu kann man beispielsweise ein Gen in einen Organismus einbringen, ein vorhandenes Gen durch ein anderes Gen ersetzen, die Kopienzahl des Gens bzw. der Gene erhöhen, einen starken Promotor verwenden oder ein Gen verwenden, das für ein entsprechendes Enzym mit einer hohen Aktivität kodiert und man kann gegebenenfalls diese Maßnahmen kombinieren.

Vorzugsweise umfassen solche Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz.

Unter einem "Promotor", einer "Nukleinsäure mit Promotoraktivität" oder einer "Promotorsequenz" wird hierin eine Nukleinsäure verstanden, die in funktioneller Verknüpfung mit einer zu transkribierenden Nukleinsäure die Transkription dieser Nukleinsäure reguliert.

Unter einer "funktionellen" oder "operativen" Verknüpfung versteht man in diesem Zusammenhang beispielsweise die sequentielle Anordnung einer der Nukleinsäuren mit Promotoraktivität und einer zu transkribierenden Nukleinsäuresequenz und gegebenenfalls weiterer regulativer Elemente, wie zum Beispiel Nukleinsäuresequenzen, die die Transkription von Nukleinsäuren gewährleisten, sowie zum Beispiel einen Terminator, derart, dass jedes der regulativen Elemente seine Funktion bei der Transkription der Nukleinsäuresequenz erfüllen kann. Dazu ist nicht unbedingt eine direkte Verknüpfung im chemischen Sinne erforderlich. Genetische Kontrollsequenzen, wie zum Beispiel *Enhancer*-Sequenzen, können ihre Funktion auch von weiter entfernten Positionen oder gar von anderen DNA-Molekülen aus auf die Zielsequenz ausüben. Bevorzugt sind Anordnungen, in denen die zu transkribierende Nukleinsäuresequenz hinter (d.h. am 3'-Ende) der Promotorsequenz positioniert wird, so dass beide Sequenzen kovalent miteinander verbunden sind. Dabei kann der Abstand zwischen der Promotorsequenz und der transgen zu exprimierenden Nukleinsäuresequenz geringer als 200 Basenpaare, oder kleiner als 100 Basenpaare oder kleiner als 50 Basenpaare sein.

Neben Promotoren und Terminator sind als Beispiele weiterer regulativer Elemente zu nennen Targeting-Sequenzen, Enhancer, Polyadenylierungssignale, selektierbare Marker, Amplifikationssignale, Replikationsursprünge und dergleichen. Geeignete regulatorische Sequenzen sind z.B. beschrieben in Goeddel, Gene Expression Technology: Methods in Enzymology 185, Academic Press, San Diego, CA (1990).

Die hierin offenbarten Nukleinsäurekonstrukte umfassen insbesondere Sequenz SEQ ID NO: 1 oder 3 oder Derivate und Homologe davon, sowie die davon ableitbaren Nukleinsäuresequenzen, die mit einem oder mehreren Regulationssignalen vorteilhafterweise zur Steuerung, z.B. Erhöhung, der Genexpression operativ oder funktionell verknüpft wurden.

Zusätzlich zu diesen Regulationssequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde. Das Nukleinsäurekonstrukt kann aber auch einfacher aufgebaut sein, das heißt, es wurden keine zusätzlichen Regulationssignale vor die kodierende Sequenz insertiert und der natürliche Promotor mit seiner Regulation wurde nicht entfernt. Stattdessen wird die natürliche Regulationssequenz so mutiert, dass keine Regulation mehr erfolgt und die Genexpression gesteigert wird.

Ein bevorzugtes Nukleinsäurekonstrukt enthält vorteilhafterweise auch eine oder mehrere der schon erwähnten "Enhancer"-Sequenzen, funktionell verknüpft mit dem Promotor, die eine erhöhte Expression der Nukleinsäuresequenz ermöglichen. Auch am 3'-Ende der DNA-Sequenzen können zusätzliche vorteilhafte Sequenzen inseriert werden, wie weitere regulatorische Elemente oder Terminatoren. Die hierin offenbarten Nukleinsäuren können in einer oder mehreren Kopien im Konstrukt enthalten sein. Im Konstrukt können noch weitere Marker, wie Antibiotikaresistenzen oder Auxotrophien komplementierende Gene, gegebenenfalls zur Selektion auf das Konstrukt enthalten sein.

Beispiele geeigneter Regulationssequenzen sind in Promotoren wie cos-, tac-, trp-, tet-, trp-tet-, lpp-, lac-, lpp-lac-, $lacI^q$ T7-, T5-, T3-, gal-, trc-, ara-, rhaP ($rhaP_{BAD}$)SP6-, lambda-$P_R$- oder im lambda-$P_L$-Promotor enthalten, die vorteilhafterweise in gram-negativen Bakterien Anwendung finden. Weitere vorteilhafte Regulationssequenzen sind beispielsweise in den gram-positiven Promotoren amy und SPO2, in den Hefe- oder Pilzpromotoren ADC1, MFalpha, AC, P-60, CYC1, GAPDH, TEF, rp28, ADH enthalten. Es können auch künstliche Promotoren für die Regulation verwendet werden.

Das Nukleinsäurekonstrukt wird zur Expression in einem Wirtsorganismus vorteilhafterweise in einen Vektor, wie beispielsweise einem Plasmid oder einem Phagen inseriert, der eine optimale Expression der Gene im Wirt ermöglicht. Unter Vektoren sind außer Plasmiden und Phagen auch alle anderen dem Fachmann bekannten Vektoren, also z.B. Viren, wie SV40, CMV, Baculovirus und Adenovirus, Transposons, IS-Elemente, Phasmide, Cosmide, und lineare oder zirkuläre DNA zu verstehen. Diese Vektoren können autonom im Wirtsorganismus repliziert oder chromosomal repliziert werden. Diese Vektoren stellen eine weitere Ausgestaltung der vorliegenden Offenbarung dar.

Geeignete Plasmide sind beispielsweise in E. coli pLG338, pACYC184, pBR322, pUC18, pUC19, pKC30, pRep4, pHS1, pKK223-3, pDHE19.2, pHS2, pPLc236, pMBL24, pLG200, pUR290, $pIN\text{-}III^{113}$-B1, $\lambda$gt11 oder pBdCl, in Streptomyces pIJ101, pIJ364, pIJ702 oder pIJ361, in Bacillus pUB110, pC194 oder pBD214, in Corynebacterium pSA77 oder pAJ667, in Pilzen pALS1, pIL2 oder pBB116, in Hefen 2alphaM, pAG-1, YEp6, YEp13 oder pEMBLYe23 oder in Pflanzen pLGV23, $pGHlac^+$, pBIN19, pAK2004 oder pDH51. Die genannten Plasmide stellen eine kleine Auswahl der möglichen Plasmide dar. Weitere Plasmide sind dem Fachmann wohl bekannt und können beispielsweise dem Buch Cloning Vectors (Hrsg. Pouwels P. H. et al. Elsevier, Amsterdam-New York-Oxford, 1985, ISBN 0 444 904018) entnommen werden.

In einer weiteren Ausgestaltungsform des Vektors kann der das Nukleinsäurekonstrukt oder die Nukleinsäure enthaltende Vektor auch vorteilhafterweise in Form einer linearen DNA in die Mikroorganismen eingeführt werden und über heterologe oder homologe Rekombination in das Genom des Wirtsorganismus integriert werden. Diese lineare DNA kann aus einem linearisierten Vektor wie einem Plasmid oder nur aus dem Nukleinsäurekonstrukt oder der Nukleinsäure bestehen.

Für eine optimale Expression heterologer Gene in Organismen ist es vorteilhaft, die Nukleinsäuresequenzen entsprechend der im Organismus verwendeten spezifischen "Codonnutzung" zu verändern. Die "Codonnutzung" lässt sich anhand von Computerauswertungen anderer, bekannter Gene des betreffenden Organismus leicht ermitteln.

Die Herstellung einer Expressionskassette erfolgt durch Fusion eines geeigneten Promotors mit einer geeigneten kodierenden Nukleotidsequenz sowie einem Terminator- oder Polyadenylierungssignal. Dazu verwendet man gängige Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley Interscience (1987) beschrieben sind.

Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im Wirt ermöglicht. Vektoren sind dem Fachmann wohlbekannt und können beispielsweise aus "Cloning Vectors" (Pouwels P. H. et al., Hrsg, Elsevier, Amsterdam-New York-Oxford, 1985) entnommen werden.

### 3.3 Mikroorganismen

Je nach Zusammenhang kann unter dem Begriff "Mikroorganismus" der Ausgangsmikroorganismus (Wildtyp) oder ein genetisch veränderter, rekombinanter Mikroorganismus oder beides verstanden werden.

Mit Hilfe der Vektoren sind rekombinante Mikroorganismen herstellbar, welche beispielsweise mit wenigstens einem Vektor transformiert sind und zur Produktion der im erfindungsgemäßen Verfahren verwendeten Polypeptide eingesetzt werden können. Vorteilhafterweise werden die oben beschriebenen rekombinanten Konstrukte in ein geeignetes Wirtssystem eingebracht und exprimiert. Dabei werden vorzugsweise dem Fachmann bekannte geläufige Klonierungs- und Transfektionsmethoden, wie beispielsweise Co-Präzipitation, Protoplastenfusion, Elektroporation, retrovirale Transfektion und dergleichen, verwendet, um die genannten Nukleinsäuren im jeweiligen Expressionssystem zur Expression zu bringen. Geeignete Systeme werden beispielsweise in Current Protocols in Molecular Biology, F. Ausubel et al., Hrsg., Wiley Interscience, New York 1997, oder Sambrook et al. Molecular Cloning: A Laboratory Manual. 2. Aufl., Cold Spring Harbor Laboratory, Cold Spring Harbor Laboratory Press, Cold Spring Harbor, NY, 1989 beschrieben.

Als rekombinante Wirtsorganismen für die Nukleinsäure oder dem Nukleinsäurekonstrukt kommen prinzipiell alle prokaryontischen oder eukaryontischen Organismen in Frage. Vorteilhafterweise werden als Wirtsorganismen Mikroorganismen wie Bakterien, Pilze oder Hefen verwendet. Vorteilhaft werden gram-positive oder gram-negative Bakterien, bevorzugt Bakterien der Familien Enterobacteriaceae, Pseudomonadaceae, Rhizobiaceae, Streptomycetaceae oder Nocardiaceae, besonders bevorzugt Bakterien der Gattungen Escherichia, Pseudomonas, Streptomyces, Nocardia, Burkholderia, Salmonella, Agrobacterium, Clostridium oder Rhodococcus verwendet. Ganz besonders bevorzugt ist die Gattung und Art Escherichia coli. Weitere vorteilhafte Bakterien sind darüber hinaus in der Gruppe der alpha-Proteobacterien, beta-Proteobacterien oder gamma-Proteobacterien zu finden

Der Wirtsorganismus oder die Wirtsorganismen enthalten dabei vorzugsweise mindestens eine der hierin beschriebenen Nukleinsäuresequenzen, Nukleinsäurekonstrukte oder Vektoren, die für ein Enzym mit 12$\alpha$-HSDH-Aktivität gemäß obiger Definition kodieren.

Die im erfindungsgemäßen Verfahren verwendeten Organismen werden je nach Wirtsorganismus in dem Fachmann bekannter Weise angezogen bzw. gezüchtet. Mikroorganismen werden in der Regel in einem flüssigen Medium, das eine Kohlenstoffquelle meist in Form von Zuckern, eine Stickstoffquelle meist in Form von organischen Stickstoffquellen wie Hefeextrakt oder Salzen wie Ammoniumsulfat, Spurenelemente wie Eisen-, Mangan-, Magnesiumsalze und gegebenenfalls Vitamine enthält, bei Temperaturen zwischen 0 °C und 100 °C, bevorzugt zwischen 10 °C bis 60 °C unter Sauerstoffbegasung angezogen. Dabei kann der pH der Nährflüssigkeit auf einem festen Wert gehalten werden, das heißt während der Anzucht reguliert werden oder nicht. Die Anzucht kann "batch"-weise, "semi batch"-weise oder kontinuierlich erfolgen. Nährstoffe können zu Beginn der Fermentation vorgelegt oder semikontinuierlich oder kontinuierlich nachgefüttert werden.

### 3.4 Herstellung von UDCS

#### 3.4.1 Einleitung

Zur medikamentösen Behandlung von Gallensteinleiden werden seit vielen Jahren u. a. die Wirkstoffe Ursodesoxycholsäure (UDCS) und das zugehörige Diastereomer Chenodesoxycholsäure (CDCS) eingesetzt. Beide Verbindungen unterscheiden sich lediglich durch die Konfiguration der Hydroxygruppe am C-Atom 7 (UDCS: $\beta$ -Konfiguration, CDCS: $\alpha$-Konfiguration). Zur Herstellung von kommerziellen Mengen UDCS wird bisher bevorzugt ein Verfahren verwendet, bei dem CDCS als Rohstoff eingesetzt wird. CDCS wiederum wird vorzugsweise aus Cholsäure (CS) hergestellt.

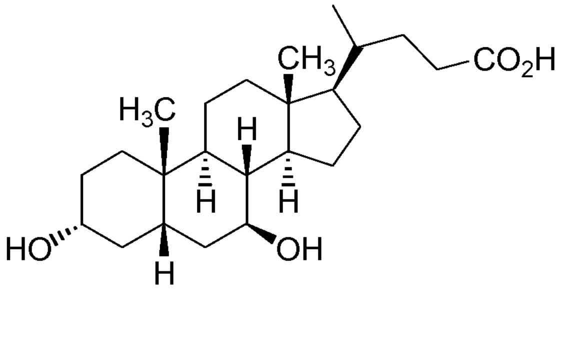

UDCS

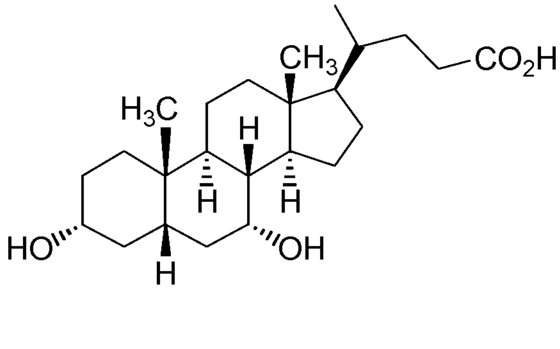

CDCS

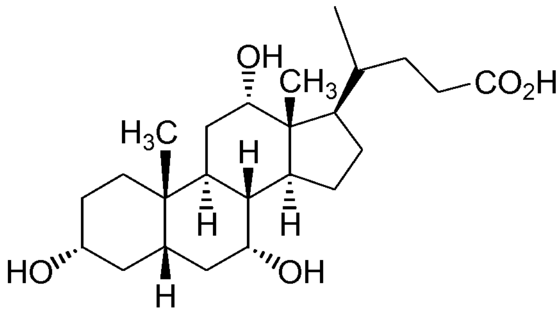

CS

### 3.4.2 Herstellung von CDCS

Als Rohstoff für die Herstellung von CDCS (CAS 474-25-9) wird CS (CAS 81-25-4) verwendet. Die klassische nicht beanspruchte chemische Route 1 bedient sich ausschließlich chemischer Verfahrensschritte. In diesem Fall sind vier Schritte erforderlich, um CS zu CDCS umzuwandeln. Die alternative Route 2 umfasst die Enzym-katalysierte Umsetzung. Dieser Weg führt in nur zwei Schritten von CS zu CDCS.

#### 3.4.2.1 Route 1 (chemischer Weg)

Im ersten Schritt dieser Synthese wird die Carbonsäuregruppe der CS zum Methylester (CDCS I, CAS 1448-36-8) verestert. Es schließt sich die regioselektiv verlaufende Acetylierung der Hydroxygruppen in den Positionen 3 und 7 an. Das Acetylierungsprodukt, 3,7-Di-O-acetylcholsäuremethylester (CDCS II, CAS 3749-87-9) fällt kristallin an und wird isoliert. In der Folgestufe (Schritt 3) wird die freie Hydroxygruppe in Position 12 oxidiert. Der 3,7-Di-O-acetyl-12-ketocholansäuremethylester (CDCS III, CAS 4651-67-6) wird im vierten und letzten Schritt in einer Wolff-Kishner-Reduktion zu CDCS desoxygeniert.

*1. Schritt: Veresterung*

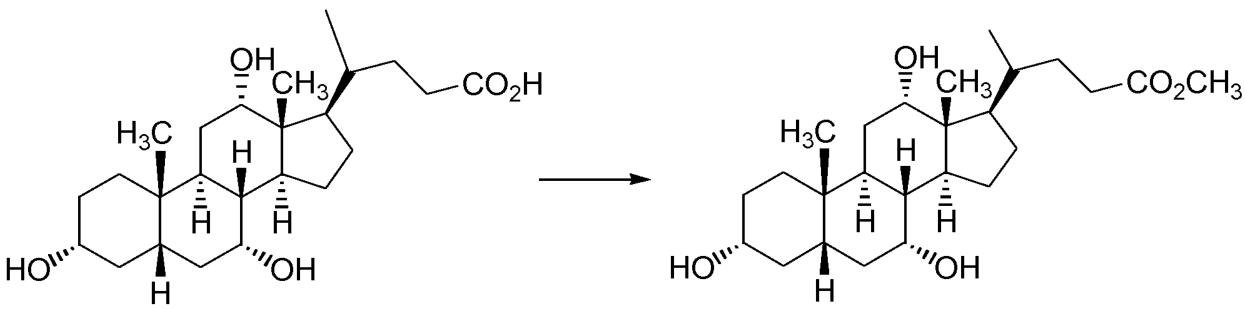

CS CDCS I

*2. Schritt: Acetylierung*

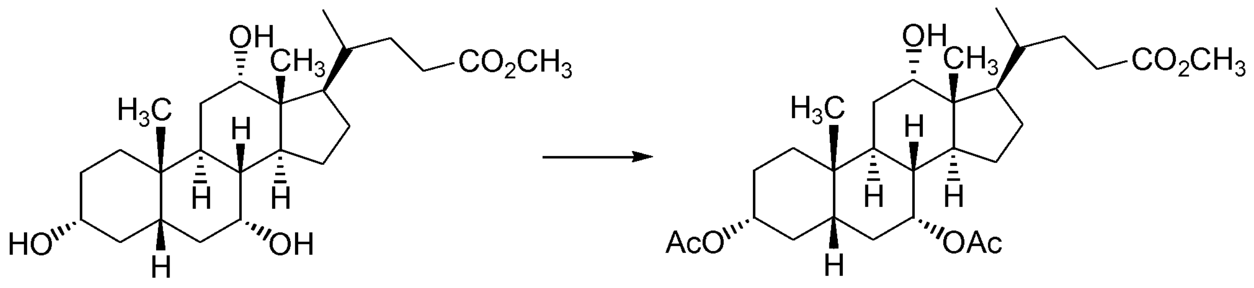

CDCS I CDCS II

*3. Schritt: Oxidation*

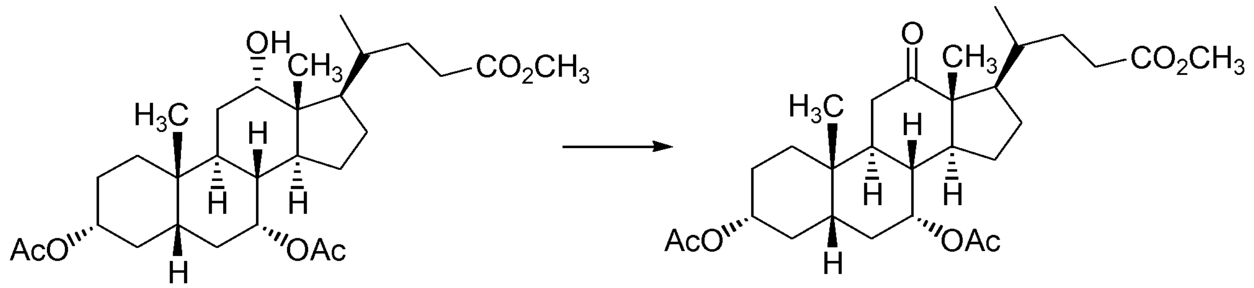

CDCS II CDCS III

*4. Schritt: Desoxygenierung*

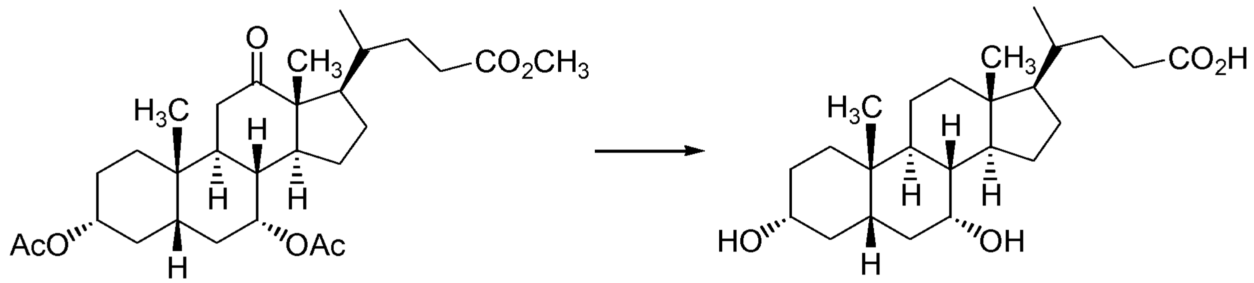

CDCS III CDCS

Im Einzelnen wird der Prozess wie folgt durchgeführt:

In Stufe 1 wird CS mit Methanol säurekatalysiert zum Cholsäuremethylester (CDCS I) verestert. Es folgt die regioselektive Acetylierung der Hydroxylgruppen in den Positionen 3 und 7 mit Acetanhydrid. Zur Reaktion wird eine organische Stickstoffbase und optional ein Acylierungskatalysator verwendet. Durch Optimierung der Reaktionszeit wird hier ein Maximum der Diacetylverbindung (CDCS II) erreicht. Das Produkt wird nach Kristallisation isoliert und getrocknet. Acetylierungsbedingungen, insbesondere die Kombination Acetanhydrid, Triethylamin und DMAP werden in der EP 0 424 232 beschrieben. Die Selektivität der Acetylierung entscheidet über die spätere Qualität des (Zwischen-) Produkts CDCS. Das Nebenprodukt 3-O-Monoacetylcholsäuremethylester führt im weiteren Syntheseverlauf zu Lithocholsäure. Diese ist toxisch und in den Monographien des Endprodukts UDCS auf einen niedrigen Wert limitiert (Ph. Eur. 0,1 %, USP 0,05 %). Bei einer Überacetylierung zum 3,7,12-Tri-O-acetylcholsäuremethylester

enthält die später erhaltene CDCS anteilig mehr CS als Verunreinigung.

Die Oxidation der CDCS II zu CDCS III erfolgt mit wässriger Natriumhypochloritlösung. Das Produkt fällt aus der Reaktionslösung aus, wird abfiltriert und getrocknet. Auch dieses Procedere wird in der EP 0 424 232 beschrieben. Generell finden sich in der Literatur noch andere Oxidationsmittel als Alternativen, wie Chromsäure.

Zur Desoxygenierung der CDCS III zu CDCS sind verschiedene Varianten der Wolff-Kishner-Reduktion bekannt. Bei einer Methode wird CDCS III mit Hydrazin und Natriumhydroxid in Triethylenglycol bei 200 °C umgesetzt. Das Produkt wird durch Ansäuern mit Salzsäure aus der Reaktionslösung gefällt, anschließend abfiltriert und getrocknet. Eine andere Methode ist in EP 0 424 232 beschrieben und arbeitet bei niedrigerer Temperatur. CDCS III wird hier mit Hydrazin und Kaliumhydroxid in 2-Butanol umgesetzt. Das Produkt wird aus Wasser wie bei Variante 1 durch Zugabe von Salzsäure gefällt.

Die nach diesem Verfahren erhaltene CDCS besitzt eine definierte und spezifizierte Qualität, die geeignet ist, um nach dem später beschriebenen Verfahren UDCS in Arzneibuchqualität herzustellen.

**3.4.2.2 Route 2 (enzymatischer Weg)**

Als Alternative zum ausschließlich chemischen Verfahren wird erfindungsgemäß eine enzymkatalysierte Oxidation von CS zu 12-Ketochenodesoxycholsäure (12-Keto-CDCS, CAS 2458-08-4), die dann weiter zu CDCS umgesetzt wird, bereitgestellt. Dieser Syntheseweg beinhaltet nur zwei Schritte und ist damit im Vergleich zu der rein chemischen Route deutlich einfacher durchzuführen.

*1. Schritt: enzymatische Oxidation*

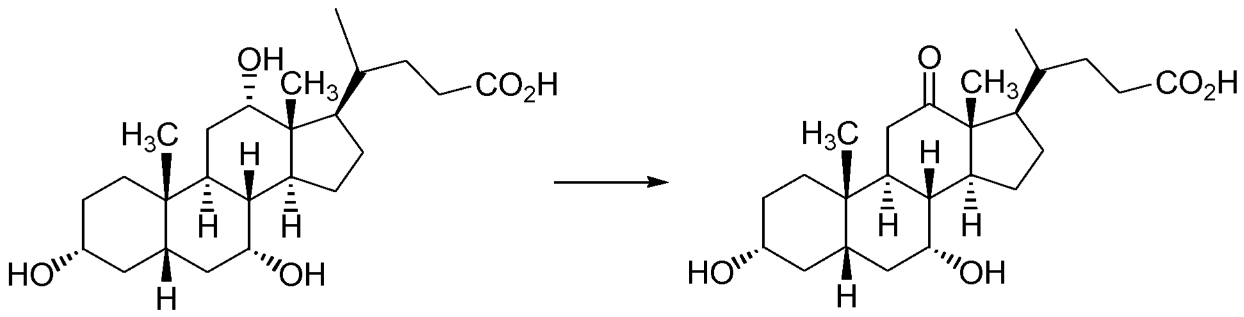

CS

12-Keto-CDCS

*2. Schritt: Desoxygenierung*

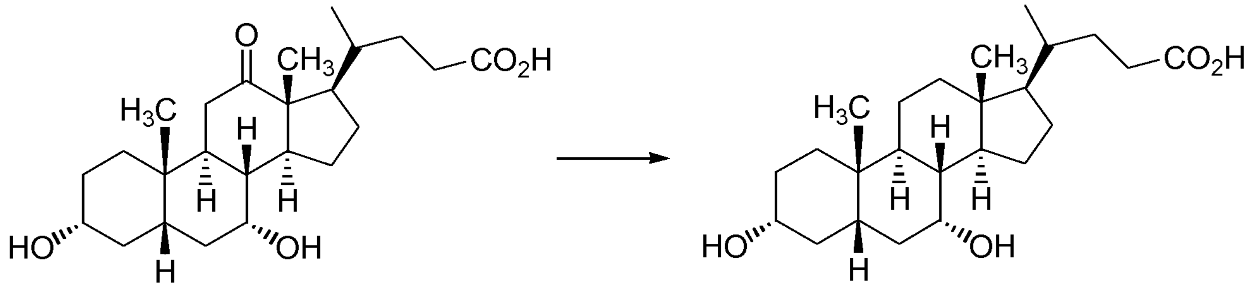

12-Keto-CDCS

CDCS

Gemäß Schritt 1 wird Cholsäure mittels 12α-HSDH zu 12-Ketochenodesoxycholsäure (12-Keto-CDCS) $NADP^+$-abhängig oxidiert. Diese Reaktion ist reversibel. 12α-HSDHs gehören der Enzymklasse 1.1.1.176 an und werden hauptsächlich in Bakterien der Gattung *Clostridium* gefunden. Es existieren sowohl $NADP^+$-abhängige (Harris and Hylemon (1978) Biochim Biophys Acta 528(1): 148-57), als auch $NAD^+$-abhängige Vertreter (Macdonald et al. 1976) Biochim Biophys Acta 450(2): 142-53.

Der einzige bekannte Mikroorganismus, der eine hohe 12α-HSDH-Aktivität in Abwesenheit anderer HSDH exprimiert, stellt *Clostridium sp.* group P strain 48-50 DSM 4029 dar (Macdonald *et al.* 1979. a.a. O.). Deshalb wurde dieser Organismus bisher als Produzent von 12α-HSDH eingesetzt, wobei eine anspruchsvolle anaerobe Fermentation mit kostenintensivem Medium nötig ist (Macdonald 1981) Experientia 37(5): 451-2. Allerdings konnte Letzteres durch Hefeautolysat ersetzt werden (Braun, M. *et al.* 1991, a.a.O).

Die enzymatische Oxidation erfolgt erfindungsgemäß bevorzugt mittels einer hierin beschriebenen 12α-HSDH (Lang- oder Kurzversion) und Cofaktorregenerierung mittels eines hierin beschriebenen Systems basierend auf NAD(P)H-Dehydrogenasen.

Die Desoxygenierung gemäß Schritt 2 ist eine klassische chemische Wolff-Kishner-Reduktion und wird analog zur oben beschriebenen Desoxygenierung der CDCS III durchgeführt. Ein wesentlicher Vorteil dieser Route ist, dass durch die Selektivität des Enzyms die Verunreinigung Lithocholsäure nicht entsteht.

**3.4.3.3 Herstellung von UDCS**

Als Rohstoff für UDCS (CAS 128-13-2) wird CDCS verwendet. Im ersten Syntheseschritt wird die Hydroxylgruppe in Position 7 der CDCS zum entsprechenden Keton oxidiert. Es resultiert die 7-Ketolithocholsäure (3α-Hydroxy-7-ketochoiansäure, kurz: KLCS, CAS 4651-67-6). Im zweiten Schritt folgt die stereoselektive Reduktion der Ketogruppe in Position 7. Ziel ist es, mit möglichst hoher Diastereoselektivität UDCS zu erhalten. In der Regel enthält die UDCS direkt nach der Reduktion noch einige Prozent des Diastereomers CDCS. Um zum Wirkstoff UDCS zu gelangen, muss UDCS roh in einem dritten Schritt gereinigt werden.

*1. Schritt: Oxidation*

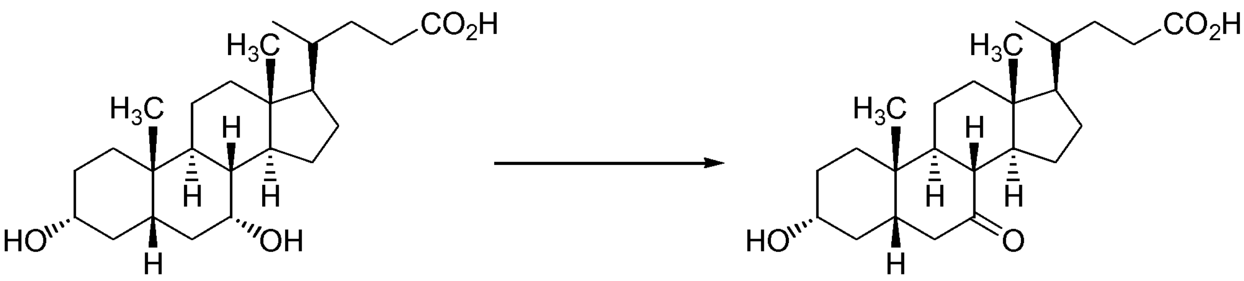

CDCS KLCS

*2. Schritt: Reduktion*

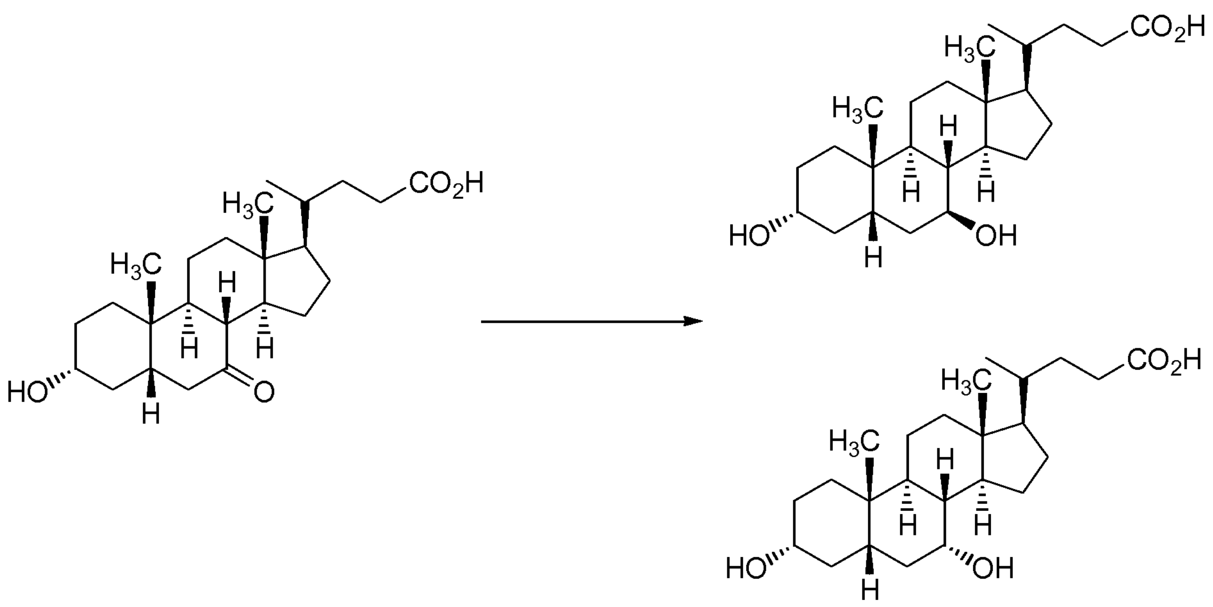

KLCS

UDCS roh (bestehend aus UDCS und CDCS)

*3. Schritt: Reinigung*

UDCS roh -> UDCS rein

Die Oxidation der CDCS erfolgt üblicherweise mit wässriger Natriumhypochloritlösung. In der Literatur findet sich noch die Chromsäureoxidation als Alternative. KLCS fällt als Feststoff an, der dann im zweiten Schritt weiter verarbeitet wird. Die Reduktion kann mit Natriummetall in Alkoholen durchgeführt werden. Es resultiert ein Rohprodukt mit einer Zusammensetzung von UDCS: CDCS von ca. 85 : 15. In alternativen Verfahren wird KLCS mit Wasserstoff an einem Nickelkatalysator (Raney-Nickel) in Alkoholen (wie z.B. aliphatischen Alkoholen) als Lösungsmittel zusammen mit einer Base, wie Kalium-t-butylat oder Kaliumhydroxid, reduziert (EP-A-0 230 085). Zusätzlich ist noch die Reduktion mit Kalium und Lithium (höhere Selektivität als Natrium, C. Giordano et al. Angew. Chem. 1985, 97, 510) sowie Zink (ES 489661) und elektrochemisch (US 4,547,271) möglich.

Bei der Aufreinigung von UDCS roh zu UDCS rein handelt es sich um eine Trennung diastereomerer Salze. Sie erfolgt durch Herstellung, Isolierung und nachfolgende Spaltung eines geeigneten Salzes von UDCS. In der Literatur werden folgende alternativen Reinigungsmethoden genannt: Herstellung, Umkristallisation und Spaltung eines korrespondierenden UDCS-Esters (EP-A-0386 538), Extraktionen (JP 60006699) und chromatographische Verfahren (IT 2000MI1177).

**3.5 Rekombinante Herstellung von erfindungsgemäß verwendeten Enzymen**

Erfindungsgemäß verwendete Polypeptide oder funktionelle, biologisch aktive Fragmente davon sind insbesondere auch rekombinant herstellbar, wobei man einen Polypeptidproduzierenden Mikroorganismus kultiviert, gegebenenfalls die Expression der Polypeptide induziert und diese aus der Kultur isoliert. Die Polypeptide können so auch in großtechnischem Maßstab produziert werden, falls dies erwünscht ist.

Die hergestellten Mikroorganismen können kontinuierlich oder diskontinuierlich im batch-Verfahren (Satzkultivierung) oder im fed batch (Zulaufverfahren) oder repeated fed batch Verfahren (repetitives Zulaufverfahren) kultiviert werden. Eine Zusammenfassung über bekannte Kultivierungsmethoden ist im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) zu finden.

Das zu verwendende Kulturmedium hat in geeigneter Weise den Ansprüchen der jeweiligen Stämme zu genügen. Beschreibungen von Kulturmedien verschiedener Mikroorganismen sind im Handbuch "Manual of Methods for

General Bacteriology" der American Society for Bacteriology (Washington D. C., USA, 1981) enthalten.

Diese erfindungsgemäß einsetzbaren Medien umfassen gewöhnlich eine oder mehrere Kohlenstoffquellen, Stickstoffquellen, anorganische Salze, Vitamine und/oder Spurenelemente.

Bevorzugte Kohlenstoffquellen sind Zucker, wie Mono-, Di- oder Polysaccharide. Sehr gute Kohlenstoffquellen sind beispielsweise Glucose, Fructose, Mannose, Galactose, Ribose, Sorbose, Ribulose, Lactose, Maltose, Saccharose, Raffinose, Stärke oder Cellulose. Man kann Zucker auch über komplexe Verbindungen, wie Melassen, oder andere Nebenprodukte der Zucker-Raffinierung zu den Medien geben. Es kann auch vorteilhaft sein, Gemische verschiedener Kohlenstoffquellen zuzugeben. Andere mögliche Kohlenstoffquellen sind Öle und Fette wie z. B. Sojaöl. Sonnenblumenöl. Erdnussöl und Kokosfett, Fettsäuren wie z. B. Palmitinsäure, Stearinsäure oder Linolsäure, Alkohole wie z. B. Glycerin, Methanol oder Ethanol und organische Säuren wie z. B. Essigsäure oder Milchsäure.

Stickstoffquellen sind gewöhnlich organische oder anorganische Stickstoffverbindungen oder Materialien, die diese Verbindungen enthalten. Beispielhafte Stickstoffquellen umfassen Ammoniak-Gas oder Ammoniumsalze, wie Ammoniumsulfat, Ammoniumchlorid, Ammoniumphosphat, Ammoniumcarbonat oder Ammoniumnitrat, Nitrate, Harnstoff, Aminosäuren oder komplexe Stickstoffquellen, wie Maisquellwasser, Sojamehl, Sojaprotein, Hefeextrakt, Fleischextrakt und andere. Die Stickstoffquellen können einzeln oder als Mischung verwendet werden.

Anorganische Salzverbindungen, die in den Medien enthalten sein können, umfassen die Chlorid-, Phosphor- oder Sulfatsalze von Calcium, Magnesium, Natrium, Kobalt, Molybdän, Kalium, Mangan, Zink, Kupfer und Eisen.

Als Schwefelquelle können anorganische schwefelhaltige Verbindungen wie beispielsweise Sulfate, Sulfite, Dithionite, Tetrathionate, Thiosulfate, Sulfide aber auch organische Schwefelverbindungen, wie Mercaptane und Thiole, verwendet werden.

Als Phosphorquelle können Phosphorsäure, Kaliumdihydrogenphosphat oder Dikaliumhydrogenphosphat oder die entsprechenden natriumhaltigen Salze verwendet werden.

Chelatbildner können zum Medium gegeben werden, um die Metallionen in Lösung zu halten. Besonders geeignete Chelatbildner umfassen Dihydroxyphenole, wie Catechol oder Protocatechuat, oder organische Säuren, wie Citronensäure.

Die erfindungsgemäß eingesetzten Fermentationsmedien enthalten üblicherweise auch andere Wachstumsfaktoren, wie Vitamine oder Wachstumsförderer, zu denen beispielsweise Biotin, Riboflavin, Thiamin, Folsäure, Nikotinsäure, Panthothenat und Pyridoxin gehören. Wachstumsfaktoren und Salze stammen häufig von komplexen Medienkomponenten, wie Hefeextrakt, Melassen, Maisquellwasser und dergleichen. Dem Kulturmedium können überdies geeignete Vorstufen zugesetzt werden. Die genaue Zusammensetzung der Medienverbindungen hängt stark vom jeweiligen Experiment ab und wird für jeden spezifischen Fall individuell entschieden. Information über die Medienoptimierung ist erhältlich aus dem Lehrbuch "Applied Microbiol. Physiology, A Practical Approach" (Hrsg. P.M. Rhodes, P.F. Stanbury, IRL Press (1997) S. 53-73, ISBN 0 19 963577 3). Wachstumsmedien lassen sich auch von kommerziellen Anbietern beziehen, wie Standard 1 (Merck) oder BHI (Brain heart infusion, DIFCO) und dergleichen.

Sämtliche Medienkomponenten werden, entweder durch Hitze (20 min bei 1,5 bar und 121 °C) oder durch Sterilfiltration, sterilisiert. Die Komponenten können entweder zusammen oder nötigenfalls getrennt sterilisiert werden. Sämtliche Medienkomponenten können zu Beginn der Anzucht zugegen sein oder wahlfrei kontinuierlich oder chargenweise hinzugegeben werden.

Die Temperatur der Kultur liegt normalerweise zwischen 15 °C und 45 °C, vorzugsweise bei 25 °C bis 40 °C und kann während des Experimentes konstant gehalten oder verändert werden. Der pH-Wert des Mediums sollte im Bereich von 5 bis 8,5, vorzugsweise um 7,0 liegen. Der pH-Wert für die Anzucht lässt sich während der Anzucht durch Zugabe von basischen Verbindungen wie Natriumhydroxid, Kaliumhydroxid, Ammoniak bzw. Ammoniakwasser oder saure Verbindungen wie Phosphorsäure oder Schwefelsäure kontrollieren. Zur Kontrolle der Schaumentwicklung können Antischaummittel, wie z. B. Fettsäurepolyglykolester, eingesetzt werden. Zur Aufrechterhaltung der Stabilität von Plasmiden können dem Medium geeignete selektiv wirkende Stoffe, wie z. B. Antibiotika, hinzugefügt werden. Um aerobe Bedingungen aufrechtzuerhalten, werden Sauerstoff oder sauerstoffhaltige Gasmischungen, wie z. B. Umgebungsluft, in die Kultur eingetragen. Die Temperatur der Kultur liegt normalerweise bei 20 °C bis 45 °C und. Die Kultur wird so lange fortgesetzt, bis sich ein Maximum des gewünschten Produktes gebildet hat. Dieses Ziel wird normalerweise innerhalb von 10 Stunden bis 160 Stunden erreicht.

Die Fermentationsbrühe wird anschließend weiterverarbeitet. Je nach Anforderung kann die Biomasse ganz oder teilweise durch Separationsmethoden, wie z.B. Zentrifugation, Filtration, Dekantieren oder einer Kombination dieser Methoden aus der Fermentationsbrühe entfernt oder vollständig in ihr belassen werden.

Die Zellen können auch, falls die Polypeptide nicht in das Kulturmedium sezerniert, aufgeschlossen und das Produkt nach bekannten Proteinisolierungsverfahren aus dem Lysat gewonnen werden. Die Zellen können wahlweise durch hochfrequenten Ultraschall, durch hohen Druck, wie z.B. in einer French-Druckzelle, durch Osmolyse, durch Einwirkung von Detergenzien, lytischen Enzymen oder organischen Lösungsmitteln, durch Homogenisatoren oder durch Kombination mehrerer der aufgeführten Verfahren aufgeschlossen werden.

Eine Aufreinigung der Polypeptide kann mit bekannten, chromatographischen Verfahren erzielt werden, wie

Molekularsieb-Chromatographie (Gelfiltration), wie Q-Sepharose-Chromatographie, Ionenaustausch-Chromatographie und hydrophober Chromatographie, sowie mit anderen üblichen Verfahren wie Ultrafiltration, Kristallisation, Aussalzen, Dialyse und nativer Gelelektrophorese. Geeignete Verfahren werden beispielsweise in Cooper, F. G., Biochemische Arbeitsmethoden, Verlag Walter de Gruyter, Berlin, New York oder in Scopes, R., Protein Purification, Springer Verlag, New York, Heidelberg, Berlin beschrieben.

Vorteilhaft kann es sein, zur Isolierung des rekombinanten Proteins Vektorsysteme oder Oligonukleotide zu verwenden, die die cDNA um bestimmte Nukleotidsequenzen verlängern und damit für veränderte Polypeptide oder Fusionsproteine kodieren, die z.B. einer einfacheren Reinigung dienen. Derartige geeignete Modifikationen sind beispielsweise als Anker fungierende sogenannte "Tags", wie z.B. die als Hexa-Histidin-Anker bekannte Modifikation oder Epitope, die als Antigene von Antikörpern erkannt werden können (beschrieben zum Beispiel in Harlow, E. and Lane, D., 1988, Antibodies: A Laboratory Manual. Cold Spring Harbor (N.Y.) Press). Diese Anker können zur Anheftung der Proteine an einen festen Träger, wie z.B. einer Polymermatrix, dienen, die beispielsweise in einer Chromatographiesäule eingefüllt sein kann, oder an einer Mikrotiterplatte oder einem sonstigen Träger verwendet werden kann.

Gleichzeitig können diese Anker auch zur Erkennung der Proteine verwendet werden. Zur Erkennung der Proteine können außerdem übliche Marker, wie Fluoreszenzfarbstoffe, Enzymmarker, die nach Reaktion mit einem Substrat ein detektierbares Reaktionsprodukt bilden, oder radioaktive Marker, allein oder in Kombination mit den Ankern zur Derivatisierung der Proteine verwendet werden.

**3.6 Enzymimmobilisierung**

Die erfindungsgemäß eingesetzten Enzyme können in den hierin beschriebenen Verfahren frei oder immobilisiert eingesetzt werden. Unter einem immobilisierten Enzym versteht man ein Enzym, das an einen inerten Träger fixiert ist. Geeignete Trägermaterialien sowie die darauf immobilisierten Enzyme sind aus der EP-A-1149849, EP-A-1 069 183 und der DE-OS 100193773 sowie aus den darin zitierten Literaturstellen bekannt. Auf die Offenbarung dieser Schriften wird diesbezüglich in vollem Umfang Bezug genommen. Zu den geeigneten Trägermaterialien gehören beispielsweise Tone, Tonmineralien, wie Kaolinit, Diatomeenerde, Perlit, Siliciumdioxid, Aluminiumoxid, Natriumcarbonat, Calciumcarbonat, Cellulosepulver, Anionenaustauschermaterialien, synthetische Polymere, wie Polystyrol, Acrylharze, Phenolformaldehydharze, Polyurethane und Polyolefine, wie Polyethylen und Polypropylen. Die Trägermaterialien werden zur Herstellung der geträgerten Enzyme üblicherweise in einer feinteiligen, partikelförmigen Form eingesetzt, wobei poröse Formen bevorzugt sind. Die Partikelgröße des Trägermaterials beträgt üblicherweise nicht mehr als 5 mm, insbesondere nicht mehr als 2 mm (Sieblinie). Analog kann bei Einsatz der Dehydrogenase als Ganzzell-Katalysator eine freie oder immobilisierte Form gewählt werden. Trägermaterialien sind z.B. Ca-Alginat, und Carrageenan. Enzyme wie auch Zellen können auch direkt mit Glutaraldehyd vernetzt werden (Cross-linking zu CLEAs). Entsprechende und weitere Immobilisierungsverfahren sind beispielsweise in J. Lalonde und A. Margolin "Immobilization of Enzymes" in K. Drauz und H. Waldmann, Enzyme Catalysis in Organic Synthesis 2002, Vol. III, 991-1032, Wiley-VCH, Weinheim beschrieben.

**Experimenteller Teil**

**Materialien:**

Enzyme und Enzympuffer wurden, wenn keine anderen Angaben gemacht werden, von Fermentas, St. Leon-Rot oder NEB, Frankfurt bezogen.

LB-Medium:

| | |
|---|---|
| Bacto-Trypton | 10 g |
| Hefe-Extrakt | 5 g |
| Natriumchlorid | 5 g |
| doppeltdestilliertes Wasser | ad 1000 ml |

TB-Medium:

Lösung I:

| | |
|---|---|
| Bacto-Trypton | 12 g |
| Hefe-Extrakt | 24 |
| Glycerin, wasserfrei | 4 ml |
| doppeltdestilliertes Wasser | ad 900 ml |

Lösung II:

| | |
|---|---|
| Kaliumdihydrogenphosphat | 0,17 M |
| Kaliumhydrogenphosphat | 0,72 M |
| doppeltdestilliertes Wasser | ad 100 ml |

Beide Lösungen wurden nach dem Autoklavieren vereinigt.

**Expressionsvektoren**

Zur Expression von 12α-HSDH diente der Vektor pET22b(+), Novagen, Darmstadt, welcher eine MCS unter der Kontrolle eines T7-Promotors und -Transkriptionsstarts enthält und einen T7-Terminator besitzt. Die Expression wird mittels Isopropyl-β-D-thiogalactopyranosid (IPTG) induziert.

Dazu wurden 12α-HSDH kodierende Sequenzen PCR-amplifiziert. Die PCR-Produkte erhielt man unter Verwendung der genomischen DNA von *Clostridium sp.* group P strain 48-50 als Template und der später noch genauer beschriebenen Primerpaare. Die PCR-Produkte wurden auf ein Agarose-Gel aufgetragen, aufgetrennt und aus diesem ausgeschnitten. Anschließend wurden sie mit Hilfe von Ndel und BamHI restringiert und mit dem ebenfalls mit Ndel und BamHI geschnittenen pET22b(+)-Vektor ligiert.

**Mikroorganismen**

| **Stamm** | **Genotyp** |
|---|---|
| *Clostridium sp.* group P strain 48-50 | |
| *Escherichia coli* BL21 (DE3) | $F^-$ ompT gal dcm lon $hsdS_B(r_B^- m_B^-)$ λ(DE3 [lacI lacUV5-T7 gene 1 ind1 sam7 nin5]) |
| *Escherichia coli* Rosetta™ (DE3) | F-ompT $hsdS_B(RB^- mB^-)$ gal dcm λ(DE3 [lacI lacUV5-T7 gene 1 ind1 sam7 nin5]) pLysSRARE ($Cam^R$) |

**Methoden**

**1. Standardbedingungen für 12α-HSDH Aktivitätsbestimmung**

Die Aktivität wird wie folgt definiert: 1 U des Enzyms entspricht der Enzymmenge, welche die Umsetzung von 1 μmol/min einer 5 mM Cholsäurelösung in KaliumphosphatPuffer (50 mM, pH 8,0) bei Raumtemperatur (d.h. ca. 20 °C-23 °C) katalysiert.

Zur Aktivitätsbestimmung wurden 790 μl Kaliumphosphatpuffer (50 mM, pH 8,0), 100 μl Cholsäure (50 mM in Kaliumphosphatpuffer (50 mM, pH 8,0)) und 10 μl zu vermessende Enzymlösung in einer Küvette gemischt. Zum Start der Reaktion wurde 100 μl $NADP^+$ (2,5 mM) zugegeben und die Zunahme der Absorption bei 340 nm photometrisch bestimmt. Die Steigung über 30 s wurde bei RT ermittelt. Die Bestimmung der Aktivität erfolgte nach dem Lambert-Beerschen-Gesetz.

**2. Proteinkonzentrationsbestimmung mittels BCA-Assay**

Die Proteinkonzentration einer Lösung wurde bestimmt, indem die Absorption von 20 µl Proteinlösung, wie z.B. einer Zelllysat-Verdünnung bzw. eines resuspendierten Zelldebris-Pellets nach Ultraschallaufschluss, in 200 µl BCA-Lösung (Lösung A:Lösung B 50:1) des Analysen-Kits der Firma Bio-Rad, München bei 562 nm gemessen wurde. Dabei bildet der Bicinchoninsäure (BCA) mit einwertigen Kupferionen, die quantitativ aus der Reduktion zweiwertigen Kupferionen durch das Protein entstehen, eine violette Komplexverbindung, dessen Absorption bei 562 nm photometrisch gemessen werden kann. Die Bestimmung der Konzentration erfolgte über eine Rinderserumalbumin (BSA)-Eichgerade.

**3. Amplifizierung des 12α-HSDH Gens und Expression von 12α-HSDH**

Die Durchführung erfolgt analog zur Offenbarung der älteren PCT/EP2009/002190. (veröffentlicht unter Nr. WO2009/118176)

**3.1 Amplifizierung**

Dazu wurden folgende Primer verwendet:

Forward lang (lange Enzymversion, Ndel-Schnittstelle):

GGTATTCCATATGGATTTTATTGATTTTAAGGAGATG (SEQ ID NO: 14).

Forward kurz (kurze Enzymversion, Ndel-Schnittstelle)

GGTATTCCATATGATCTTTGACGGAAAGGTCGC (SEQ ID NO: 15).

Primer revers (BamH1-Schnittstelle)

CGGGATCCCTAGGGGCGCTGACCC (SEQ ID NO: 16).

Das Zielgen wurde unter Verwendung von *Pfu*-Polymerase durch PCR amplifiziert. Als Template diente die genomische DNA von *Clostridium sp.* group P strain 48-50 DSM 4029 (29,4 ng/µl), von der 1 µl eingesetzt wurde. Zur Amplifikation wurde 1 µl der *Pfu*-Polymerase verwendet. Als Puffer diente *Pfu*-Puffer (10x mit $MgSO_4$) (Fermentas, St. Leon-Rot). Es wurde jeweils 1,5 µl *forward*- und *revers*-Primer (10 µM) eingesetzt, sowie 2 µl Desoxynukleotidtriphosphate (20 µM). Der Ansatz wurde mit RNase-freiem Wasser auf 50 µl eingestellt. Die Reaktion wurde im Thermocycler der Firma Eppendorf durchgeführt. Der PCR-Ansatz wurde zunächst für 5 min bei 95°C gestartet, um die DNA zu denaturieren. Nun folgten bei der Klonierung der unbekannten DNA-Sequenzen 30 Zyklen beginnend mit einer Denaturierung bei 95°C für 30 s. Nachfolgend wurde der Ansatz für 30 s mittels Temperaturgradient auf jeweils 25-45°C abgekühlt, um ein Annealing der degenerierten Primer an die Target-DNA zu gewährleisten (konstante Annealing-Temperatur von 53°C). Daraufhin wurde eine Temperatur von 72°C für 90 s zur Primer-Extension eingestellt, da hier das Aktivitätsoptimum der verwendeten Polymerase liegt. Schließlich wurden die Ansätze für 10 min bei 72°C inkubiert und bis zur Entnahme aus dem Gerät bei 4°C gekühlt.

**3.2 Expression**

Das Zielgen wurde nach Amplifikation mittels Polymerasekettenreaktion über die Schnittstellen Ndel und BamHI in den Expressionsvektor pET22b+ einkloniert, in *E. coli* Rosetta DE3™ und *E. coli* BL21 DE3 Zellen eingebracht und exprimiert. Es handelt sich hierbei um nicht-pathogene Stämme, die die Produktion großer Mengen des Enzyms ermöglichen (bis zu 150000 U/l Kultur).

Zur Expression wurden 5 ml LB-Medium (mit 100 µg/ml Ampicillin) mit dem *E. coli* BL21 (DE)- oder Rosetta™-Klon, der mit einem Expressionsvektor transformiert wurde, für 16 h bei 37°C und 180 rpm inkubiert. 200 ml TB-Medium (mit 100 µg/ml Ampicillin) wurde damit inokuliert und bei 37°C und 180 rpm inkubiert. Bei einer $OD_{600}$ von 0,6-0,8 wurde die Expression von 12α-HSDH mit 1 mM IPTG induziert. Zu verschiedenen Zeitpunkten wurde 1 ml Zellsuspension mit $OD_{600}$ von 0,25 entnommen, für 1 min bei 13000 rpm pelletiert und bis zur weiteren Verwendung bei -20°C gelagert. Die Expression wurde nach 4 bzw. 22 h beendet, indem die Zellen bei 2700 g für 10 min bei 4°C pelletiert und anschließend eingefroren wurden.

Die Zellen wurden mit Hilfe von Ultraschall aufgeschlossen, indem das Pellet zunächst in 4-10 ml Kaliumphosphatpuffer (50 mM, pH 8,0) resuspendiert wurde. Im Ultraschalldesintegrator der Firma Branson wurden die Zellen bei einer Intensität von 30 % viermal 1 min mit jeweils 2 min Pause im Eisbad behandelt. Anschließend wurden die Zelltrümmer 1 h bei 4220 g und 4°C abzentrifugiert.
Proteingehalte und Enzymaktivitäten wurden wie oben beschrieben bestimmt.
Die erzielten Enzymaktivitäten nach Aufschluss der *E. coli*-Zellen sind in folgender Tabelle zusammengefasst.

| Stamm (E. coli) | Expressionsdauer [h] | Aktivität [U/l Kulturmedium] | | |
|---|---|---|---|---|
| | | HSDH_lang | HSDH_kurz | pET22b-Leervektor |
| Rosetta™ (DE3) | 4 | 3100 | 3400 | 200 |
| | 22 | 19500 | 24300 | 0 |
| BL21 (DE3) | 4 | 12700 | 12800 | - |
| | 22 | 36800 | 33000 | - |

### 3.3 12α-HSDH Mutante

Bei der 12α-HSDH ist eine Inhibierung durch das Produkt 12-Ketochenodesoxycholsäure zu beobachten, welche sich negativ auf die Reaktionsgeschwindigkeit im Prozess auswirken kann. Um diese Produktinhibierung der 12α-HSDH zu vermindern, wurde aus einer zufallsbasierten 12α-HSDH-Bibliothek, erstellt mittels error-prone PCR, unter anderem die Mutante 37D12 (vgl. SEQ ID NO: 17, 18) isoliert (vgl. Beschreibung in PCT/EP2009/002190). (veröffentlicht unter Nr. WO2009/118176)
Die Mutation der Mutante 37D12 befindet sich im Bereich des aktiven Zentrums zwischen der Substrat- und Cofaktorbindungstasche. Die Mutante zeigt eine deutlich verringerte Inhibierung bereits bei einem Umsatz von 1 %. Bei 5 % Umsatz zeigte das Wildtypenzym einen Verlust von 60 % im Gegensatz zu 20 % der Mutante 37D12. Die dreifache Aktivität verblieb bei der Mutante 37D12 gegenüber dem Wildtyp bei einem Umsatz von 25 %.

### 4. Dünnschichtchromatographie (TLC)

1 µl Reaktionsgemisch wurde durch Kieselgel-TLC analysiert, um die Umsetzung von Cholsäure zu 12-keto-CDCS zu analysieren. Die mobile Phase bestand aus einem Gemisch aus Aceton, Methylenchlorid und Essigsäure im Mischungsverhältnis 40/40/3 (Volumenteile). Substrat und Produkte wurden durch Besprühen mit einem Phosphomolybdänsäuremischung (4,76 g Phosphomolybdänsäurehydrat in 95,2 ml Essigsäure und 4,8 ml Schwefelsäure) und anschließendes Erwärmen sichtbar gemacht.

### 5. NADPH-Dehydrogenaseaktivität (DCIP als Elektronenakzeptor)

Die NADPH-Dehydrogenaseaktivität wurde unter Verwendung von Dichlorphenolindophenol (DCIP) als Elektronenakzeptor bestimmt. Das Reaktionsgemisch enthielt das Enzym, 50 mM Tris-HCl (pH 8), 4 % Cholsäure, 75 µM DCIP und 0,1 mM NADPH. Die Reaktion wurde durch Zugabe von NADPH gestartet und die Abnahme der Extinktion von DCIP bei 600 nm wurde aufgezeichnet. Die Aktivitätseinheit (µmol DCIP Reduktion pro Minute) wurde unter Verwendung eines Extinktionskoeffizienten für DCIP von 20 $mM^{-1}$ $cm^{-1}$ bei 600 nm bestimmt.

### 6. NADPH-Dehydrogenaseaktivität (Riboflavin als Elektronenakzeptor)

Die NADPH-Dehydrogenase unter Verwendung von Riboflavin als Elektronen akzeptor wurde in 50 mM Tris-HCl, 4 % Cholsäure, 10 µM Riboflavin und 0,1 mM NADPH bestimmt. Die Aktivitätseinheit (µmol NADPH oxidiert pro Minute) wurde unter Verwendung des Extinktionskoeffizienten für NADPH von 6,22 $mM^{-1}$ $cm^{-1}$ berechnet.

### 7. Klonierung und Expression der verwendeten NAD(P)H Dehydrogenasen

Käufliche Enzyme: Diaphorase aus *Clostridium kluyveri* wurde von Sigma-Aldrich (St. Louis) bezogen; Rinderleberkatalase wurde von Fluka (Steinheim) bezogen.
Die Protokolle für die DNA-Manipulationen (Restriktionsenzymverdau, Isolierung der Plasmid-DNA und Subklonierung in einen bakteriellen Wert) erfolgen entsprechend der Beschreibung in Sambrock et al., Molecular Cloning, 2. Aufl. Cold Spring Harbor Laboratory Press (1989).

### 7.1 Reduktasedomäne aus CYP102A1 aus *Bacillus megaterium* (Cytochrom P450 BM3-Reduktase) (EC 1.6.2.4; GI:117298; Swiss-Prot. P14779.2)

Der Expressionsvektor für die Reduktasedomäne von CYP102A1 (Cytochrom P450 BM3-Reduktase) (EC 1.6.2.4) wurde freundlicherweise von Herrn Dr. Girhard (ITB Universität Stuttgart, Stuttgart) bereitgestellt. Die Konstruktion des Expressionsplasmids wird beschrieben in Girhard et al. (Girhard, M. et al Cytochrom P450 monooxygenase from Clostridium acetobutylicum: A new alpha-fatty acid hydroxylase. Biochem. Biophysic. Res. Commun. 362 (2007) 114-119.)

### 7.2 Flavodoxin Reduktase aus *E. coli* (EC 1.18.1.2, GeneID: 948414, Produkt: NP_418359.1)

Der Expressionsvektor für Flavodoxin Reduktase (pET11a-*fpr*) wurde freundlicherweise von Herrn Professor Michael R. Waterman (Vanderbilt University, Tennessee) bereitgestellt. Die Konstruktion des Expressionsplasmids ist beschrieben in Jenkins, C.M. and Waterman, M.R., NADPH-Flavodoxin reductase and flavodoxin from Escherichia coli: Characteristics as a soluble Microsomal P450 Reductase, Biochemistry 37 (1998) 6106-6113.

### 7.3 NADPH-Dehydrogenase aus *Geobacillus stearothermophilus* Sulphitreduktase (GstR) (EC=1.8.1.2)

Das Expressionsplasmid für die NADPH-Dehydrogenase, abgeleitet von der Sulphitreduktase aus *Geobacillus stearothermophilus* (DSM 13240) wurde wie folgt konstruiert: Ein chimäres Genkonstrukt, pET28A1 GR (Eiben, S. Cyp102A P450 Monooxygenases: Comparative analysis and construction of cytochrome P450 chimera. Dissertation, Institut für Technische Biochemie der Universität Stuttgart (2007)) wurde durch EcoRI und HindIII verdaut und ein DNA-Fragment von 1,8 kb wurde isoliert und mit Agarosegelelektrophorese und QIA-quick® Gel extraction kit (Quiagen GmbH, Venlo) gereinigt. Das gereinigte DNA-Fragment wurd in den pET28a-Vektor in die Mehrfachklonierungsstelle eingefügt und in *Escherichia coli* DH4α eingefügt. Die transformierten Bakterien wurden über die Kanamycin-Resistenz selektioniert. Um die kodierende NADPH-Dehydrogenasesequenz am Leserahmen auszurichten, wurde ein Basenpaar in der *Eco*RI-Schnittstelle in der Plasmid-DNA unter Verwendung des QuickChange™ site-directed mutagenesis kit (Stratagene Europa, Amsterdam) deletiert. Die Nukleotidsequenzen für die Vorwärts- und Rückwärts-Primer, welche zur Mutagenese verwendet wurden, sind im Folgenden gezeigt. Das Mutationsprotokoll entsprach den Herstellerangaben. Die Mutation wurde durch DNA-Sequenzierung verifiziert.

SRdEcoRIf CCGCGGATCCGAATCTGTGACGGTATTGTACGG (SEQ ID NO:19)
SRdEcoRIr CCCGTACAATACCGTCACAGATTCGGATCCGCG (SEQ ID NO:20)

Die ursprüngliche Nukleotidsequenz der putativen Sulphitreduktaseflavin-Untereinheit von *Geobacillus stearothermophilus* stammte aus dem Genomprojekt beim *Advanced Centre of Genome Technology* der Universität von Oklahoma (ftp://ftp.genome.ouredu/pub/bstearo)

### 7.4 NAD(P)H-Flavin Reduktase (EC 1.5.1.29 / 1.16.1.3 GeneID: 6061282, Product P_001732642.1)

Das NAD(P)H-Flavin Reduktasegen wurde durch PCR unter Verwendung genomischer DNA amplifiziert, welche aus dem *Escherichia coli*-Stamm K12, Unterstamm DH5α, als Template isoliert wurde. Um die genomische DNA aus dem Bakterium zu isolieren, wurde der DNeasy Blood & Tissue Kit (Quiagen, Venlo) verwendet. 30 Zyklen zur Denaturierung (96 °C, 30 Sekunden), Annealing (47 °C, 30 Sekunden) und Elongation (72 °C, 3 Minuten) wurden durchgeführt, um das Targetgen zu amplifizieren. 50 μl PCR-Mischung enthielten 5 μl Pfu-Puffer (x 10), 0,25 mM Desoxyribonukleotide (jeweils ATP, GTP CTP und TTP), 0,02 μM Vorwärts- und Rückwärtsprimer, 0,0028 μg/ml genomische DNA und 5 Einheiten Pfu DNA-Polymerase (Eppendorf AG, Hamburg). Das PCR-Produkt wurde in den Plasmidvektor pET22b+ an der *Nde*I und *Hin*dIII-Schnittstelle subkloniert und der isolierte Klon wurde durch DNA-Sequenzierung der insertierten Region überprüft.

Primer für die PCR-Reaktion:

Fref GGAATTCATATGACAACCT TAAGCTGTAA (*Nde*I) (SEQ ID NO:21)
Frer AAATAAGCTTCAGATAAATGCAAACGCATC (*Hind*III) (SEQ ID NO:22)

### 8. Expression der Enzyme (Labormaßstab):

Die Enzyme wurden in *Escherichia coli* Bl21(DE3) exprimiert. Die mit den Expressionsvektoren transformierten Bakterien wurden in 100 ml TB-Medium kultiviert und die Proteinexpression wurde durch Zugabe von 1 mM IPTG

induziert. Nach Inkubation über Nacht bei 25°C und Schütteln bei 140 Upm wurden die Zellen abzentrifugiert. Die Zellpellets wurden in 5 ml 50 mM Tris-HCl, pH 8 suspendiert und durch Beschallen (6 x 1 Minute mit Unterbrechung) aufgeschlossen. Der nach Zentrifugation (30 Minuten, 8500 g) verbleibende Überstand wurde zur Aktivitätsmessung verwendet.

**BEISPIEL 1**

**$NADP^+$-Regeneration bei der enzymatischen Oxidation von Cholsäure unter Verwendung von Flavodoxin Reduktase und verschiedenen Redoxmediatoren**

Das $NADP^+$-Regenerationssystem wurde bei der enzymatischen Oxidation von Cholsäure mit Hilfe der HSDH getestet.

1.1 40 mg CS wurden mit 5 U des Enzyms HSDH, 0,1 mmol $NADP^+$ und 0,1 U Flavodoxin Reduktase (die U-Werte wurden bestimmt als $\mu$mol-Dichlorphenolindophenol-Reduktion/min) und 3 mmol verschiedener Redoxmediatoren in 1 ml Tris-HCl-Puffer, pH 8,0 bei 25 °C über Nacht inkubiert. Es wurden die Redoxmediatoren Dichlorphenolindophenol, Methylenblau und Indigocarmin eingesetzt. Die Umsetzung von Cholsäure wurde mittels Dünnschichtchromatographie getestet. Die Ergebnisse sind in Figur 2 dargestellt.

1.2 CS (4 % w/v) wurde mit 0,1 mmol NADH, 6 U/ml HSDH, 0,1 % Methylenblau und 0,5 U/ml Flavodoxin Reduktase in 100 ml 10 mmol Tris-HCl, pH 8,0 bei Zimmertemperatur inkubiert. Nach 7 Stunden wurde die gleiche Menge HSDH und Flavodoxin Reduktase dem Reaktionsgemisch zugesetzt und weitere 16 Stunden inkubiert. Die Konversion von Cholsäure wurde über Dünnschichtchromatographie unter Verwendung von Vergleichsmischungen aus Cholsäure und 12-Ketochenodesoxycholsäure-Standards näherungsweise bestimmt. Die Ergebnisse sind in Figur 3 dargestellt.

**BEISPIEL 2**

**Vergleich verschiedener NAD(P)H-Dehydrogenasen bei der Cofaktorregenerierung der HSDH-katalysierten Umsetzung von Cholsäure.**

Weiterhin wurde die $NADP^+$-Regeneration mit verschiedenen NAD(P)H-Dehydrogenasen bei der Oxidation von Cholsäure mit 12$\alpha$-HSDH untersucht.

Zunächst wurden die Aktivitäten der verschiedenen NAD(P)H-Dehydrogenasen in Gegenwart von 0,1 M CS bei pH 8,0 spektrophotometrisch bestimmt. Da Methylenblau in Gegenwart von Sauerstoff sofort oxidiert wird und einen zu hohen Absorptionskoeffizienten aufweist, wurde DCIP (Dichlorphenolindophenol) als Elektronenakzeptor an Stelle von Methylenblau verwendet. Die Enzyme wurden mit 75 $\mu$mol DCIP und 0,1 mmol NADP vermischt und die NADP-abhängige Reduktion von DCIP wurde über die Abnahme der Extinktion bei 600 nm aufgezeichnet. Wie in Tabelle 1 veranschaulicht, zeigen die Enzyme Flavodoxin Reduktase, Reduktase Domäne der CYP102A1 und NADPH Dehydrogenase aus Sulfit Reduktase eine höhere Aktivität im Vergleich zu anderen Enzymen. Die Enzyme mit höherer Aktivität wurden als nächstes bei der $NADP^+$-Regeneration für die Oxidation von CS durch 12$\alpha$-HSDH eingesetzt. Die Ergebnisse sind in Figur 4 dargestellt. Wie diese Figur zeigt, ist sowohl die Sulfit Reduktase aus Bacillus stearothermophylus und die Flavodoxin Reduktase vergleichbar aktiv.

TABELLE 1

| **Enzym** | **DCIP-Reduktion** | **Bemerkung** |
|---|---|---|
| Flavodoxin Reduktase aus *E. coli* | 4,1 | U/mg Protein |
| Reduktase Domäne der CYP102A1 | 2,25* | U/ml *E. coli-Lysat* |
| NADPH Dehydrogenase aus *Bacillus stearothermophilus* Sulfit Reduktase | 14,5 | U/mg Protein |
| Diaphorase aus *Clostridium kluyveri* | 0,26 | U/mg Protein |
| NAD(P)H-Oxidase *aus Lactobacillus sanfranciscensis* und *Thermos thermophilus HB27* | inaktiviert bei pH=8 | |
| Thioredoxinreduktase aus *Saccharomyces cerevisiae* | nicht nachweisbar | |

**Anmerkungen:**

Flavodoxin Reduktase, Reduktase Domäne der CYP102A1 und Sulfit Reduktase aus *Bacillus stearothermophilus,* NAD(P)H-Oxidase *aus Lactobacillus sanfranciscensis* und *Thermos thermophilus HB27* wurden in *E. coli* subkloniert und exprimiert.
Diaphorase aus *Clostridium kluyveri* und Thioredoxinreduktase aus *Saccharomyces cerevisiae* wurden von Sigma-Aldrich bezogen.
Die Konzentrationen der jeweiligen Enzyme wurden mit Hilfe eines Absorptionsspektrums oder eines BCA-Protein-Assays abgeschätzt.

* Die spezifische Aktivität wurde nicht bestimmt.

**BEISPIEL 3**

**Cofaktorregenerierung mittels Flavodoxin Reduktase in größerem Ansatz**

Das oben beschriebene Verfahren wurde auf einen 1-I-Maßstab übertragen. Dazu wurden 60 g CS in 1 Liter 10 mmol Kaliumphosphatpuffer (pH 8) mit 10 μmol $NADP^+$, 6000 U des Enzyms 12α-HSDH, 1 g Methylenblau, 25 mg (50.000 U) Katalase und 45 U Flavodoxin Reduktase vermischt und bei 25 °C inkubiert. Nach 3 Tagen wurde die 12α-Hydroxylierung der CS durch Dünnschichtchromatographie und HPLC bestimmt. Mit keinem der beiden Nachweisverfahren konnten Rückstände von CS nachgewiesen werden (> 99 % Umsetzung).

**BEISPIEL 4**

**$NADP^+$-Regeneration unter Verwendung von Riboflavin als Redoxmediator**

200 μl Zellextrakt aus *E. coli,* transformiert mit dem Expressionsvektor wurde in 1 ml Cholsäure-Oxidationsmischung, enthaltend 25 mM Tris-HCl, 4 % Cholsäure, 0,1 mM Riboflavin, 0,05 mg/ml Catalase, 50 U/ml 12 α-HSDH und 10 μM NADPH, über Nacht bei 25°C inkubiert. Die Oxidation von Cholsäure wurde durch Dünnschichtchromatographie analysiert. Die Ergebnisse sind in Figur 5 gezeigt.

**BEISPIEL 5**

**Toleranz gegenüber Cholsäure**

Um die Tauglichkeit verschiedener NAD(P)H-Dehydrogenasen für die CofaktorRegenerierung in Cholsäurehaltigen Reaktionsmedien zu überprüfen, wurden verschiedene enzymatische Tests durchgeführt, die im Folgenden näher beschrieben werden.

a) NADPH-Oxidation mit DCIP als Elektronenakzeptor

Die NADPH-Dehydrogenase Aktivität wurde unter Verwendung von Dichlorphenolindophenol (DCIP) als Substrat bestimmt. Die Reaktion wurde durch Zugabe von Enzym (2-50 μl) zu 1 ml Reaktionsgemisch, enthaltend 50 mM Tris-HCl (pH 8), 75 μM DCIP, 100 μM NADPH mit bzw. ohne 0,1 M Cholsäure gestartet. Die Reduktion von DCIP wurde durch Abnahme der Extinktion bei 600 nm spektrophotometrisch bestimmt. Die Aktivitätseinheit (μM DCIP reduziert/min) wurde mit Hilfe des Extinktions-Coeffizienten von DCIP von 20 $mM^{-1}$ $cm^{-1}$ bei 600 nm bestimmt.
Folgende Enzyme wurden getestet: Flavodoxin Reduktase aus *E. coli,* Reduktase Domäne der CYP102A1 aus *Bacillus megaterium;* Glutathion Reduktase aus *Saccharomyces cerevisiae* und Diaphorase aus *Clostridium kluyveri.* Die Ergebnisse sind in der folgenden Tabelle 2 zusammengestellt.
Die Toleranz von Flavodoxin Reduktase gegen steigende Konzentrationen an Cholsäure ist außerdem in beiliegender Figur 7 dargestellt.

TABELLE 2

| Enzyme (Organismus) | Ohne Cholsäure, U/mg Protein (%) | Mit 0, 1 M Cholsäure, U/mg Protein (%) | Anmerkung |
|---|---|---|---|
| Flavodoxin Reduktase (*E. coli*) | 4,2* (100%) | 3,9 (93%) | Expressed in *E. coli,* Purified |

(fortgesetzt)

| Enzyme (Organismus) | Ohne Cholsäure, U/mg Protein (%) | Mit 0, 1 M Cholsäure, U/mg Protein (%) | Anmerkung |
|---|---|---|---|
| Reduktase Domäne der CYP102A1 (*Bacillus megaterium*) | 5,5 (100%) | 1,9 (35 %) | Expressed in *E. coli,* Purified |
| Glutathion Reduktase (*Saccharomyces cerevesiae*) | 0,92 (100%) | 0,14 (15 %) | Sigma |
| Diaphorase (*Clostridium kluyveri*) | 8,4 (100%) | 0,26 (3 %) | Sigma |

Man beobachtet, dass die erfindungsgemäß verwendbaren Enzyme Flavodoxin-Reduktase und Cytochrom P 450 BM-3-Reduktase eine deutlich bessere Verträglichkeit gegenüber 0,1 M Cholsäure aufweisen als Glutathion Reduktase und Diaphorase.

b) NADPH-Oxidation mit Riboflavin als Elektronenakzeptor

Die NADPH-Oxidation unter Verwendung von Riboflavin als Elektronenakzeptor wurde in 1 ml Reaktionsmedium, enthaltend 2-50 μl Enzymlösung, 50 mM Tris-HCl (pH 8), 10 μM Riboflavin, 100 μM NADPH mit und ohne 0,1 M Cholsäure bestimmt. Die Oxidation von NADPH wurde über die Abnahme der Extinktion bei 340 nm spektrophotometrisch aufgezeichnet. Die Aktivitätseinheit (uM NADPH oxidiert/min) wurde mit Hilfes des millimolaren Extinktionscoeffizienten für NADPH von 6,22 $mM^{-1}$ $cm^{-1}$ bei 340 nm bestimmt.

Folgende Enzyme wurden getestet: NADPH Dehydrogenase aus *Bacillus stearothermophilus* Sulfit Reduktase, Flavin-Subunit von Sulfit-Reduktase aus *E. coli* und NAD(P)H-Flavin Reduktase aus *E. coli.* Die Ergebnisse sind in folgender Tabelle 3 zusammengefasst:

TABELLE 3

| Enzyme (Organismus) | Ohne Cholsäure, U/ml *E. coli* Zell-Lysate (%) | Mit 0, 1 M Cholsäure, U/ml *E. coli* Zell-Lysate (%) | Anmerkung |
|---|---|---|---|
| NADPH Dehydrogenase aus Sulfit-Reduktase (*Bacillus stearothermophilus*) | 14,2 (100%) | 22,1 (155 %) | Expressed in *E. coli* |
| NADPH Dehydrogenase aus Sulfit-Reduktase (*E. coli*) | 33,5 (100%) | 2,1 (6 %) | Expressed in *E. coli* |
| NAD(P)H-Flavin Reduktase (*E. coli*) | 683 (100%) | 37,7 (5 %) | Expressed in *E. coli* |

Man beobachtet eine überraschend deutliche Stabilität gegenüber 0,1 M Cholsäure für die im erfindungsgemäßen Verfahren verwendbare NADPH Dehydrogenase aus *Geobacillus stearothermophilus*-Sulfit-Reduktase.

Zuordnung der SEQ ID NOs:

| SEQ ID NO: | Beschreibung | Typ |
|---|---|---|
| 1 | 12α-HSDH; L | NS |
| 2 | 12α-HSDH; L | AS |
| 3 | 12α-HSDH; K | NS |
| 4 | 12α-HSDH; K | AS |
| 5 | 12α-HSDH Sequenzmotiv; L und K | AS |
| 6 | N-Terminus ; L | AS |
| 7 | N-Terminus ; L | AS |
| 8 | Sequenzmotiv; L und K | AS |
| 9 | N-terminales Sequenzmotiv; K | AS |

(fortgesetzt)

| SEQ ID NO: | Beschreibung | Typ |
|---|---|---|
| 10 | N-terminales Sequenzmotiv; K | AS |
| 11 | N-terminales Sequenzmotiv; L | AS |
| 12 | Sequenzmotiv; L und K | AS |
| 13 | C-terminales Sequenzmotiv; L und K | AS |
| 14 | PCR Primer; L | NS |
| 15 | PCR Primer; K | NS |
| 16 | PCR Primer; L und K | NS |
| 17 | Mutante 37D12; K | NS |
| 18 | Mutante 37D12 ; K | AS |
| 19 | PCR Primer | NS |
| 20 | PCR Primer | NS |
| 21 | PCR Primer | NS |
| 22 | PCR Primer | NS |
| 23 | Reductase Domäne der CYP102A1 aus *Bacillus megaterium* | NS |
| 24 | Reductase Domäne der CYP102A1 aus *Bacillus megaterium* | AS |
| 25 | Flavodoxin Reduktase aus *Escherichia coli* strain K12 | NS |
| 26 | Flavodoxin Reduktase aus *Escherichia coli* strain K12 | AS |
| 27 | NADPH Dehydrogenase aus *G. stearothermophilus* Sulfit-reduktase | NS |
| 28 | NADPH Dehydrogenase aus *G. stearothermophilus* Sulfit-reduktase | AS |
| 29 | NAD(P)H-Flavin Reduktase | NS |
| 30 | NAD(P)H-Flavin Reduktase | AS |
| AS = Aminosäuresequenz<br>NS = Nukleinsäuresequenz<br>L = Langversion<br>K = Kurzversion | | |

Auf die Offenbarung der hierin erwähnten Druckschriften wird ausdrücklich Bezug genommen.

## Patentansprüche

**1.** Verfahren zur enzymatischen Oxidation von 12α-Hydroxysteroiden der allgemeinen Formel

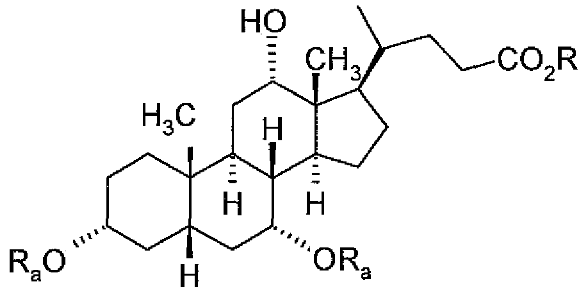

(2)

worin
R für Alkyl, H, ein Alkalimetallion oder $N(R^3)_4^+$steht, worin die Reste $R^3$ gleich oder verschieden sind und für H oder Alkyl stehen, und
die Reste $R_a$ gleich oder verschieden sind und für H oder Acyl stehen,
wobei man das Hydroxysteroid in Gegenwart einer 12α-Hydroxysteroiddehydrogenase und in Gegenwart eines Cofaktorregenerierungssystems, umfassend

a) eine NAD(P)H Dehydrogenase mit Cholsäuretoleranz, ausgewählt unter Flavodoxin Reduktasen (FDR; EC 1.18.1.2), NADPH Dehydrogenase aus *G. stearothermophilus* Sulfit Reduktasen (SR; EC 1.8.1.2), und Reduktase Domänen von CYP102A1 (EC 1.6.2.4) und davon abgeleiteten funktionalen Äquivalenten, die zur Cofaktorregenerierung befähigt sind und eine Sequenzidentität von wenigstens 85 % zur nativen Sequenz besitzen; und
b) einen Redox-Mediator, welcher Elektronen auf molekularen Sauerstoff überträgt, umsetzt, und wenigstens ein gebildetes Oxidationsprodukt aus dem Reaktionsansatz gegebenenfalls isoliert.

**2.** Verfahren nach Anspruch 1, wobei das Hydroxysteroid Cholsäure (CS) oder eine Cholsäurederivat ist.

**3.** Verfahren nach Anspruch 2 , wobei das Cholsäurederivat ein Salz, Amid oder Alkylester ist.

**4.** Verfahren nach Anspruch 2 oder 3, wobei CS oder ein Derivat davon zu 12-Ketochenodesoxycholsäure (12-Keto-CDCS) oder zum entsprechenden Derivat umgesetzt wird.

**5.** Verfahren nach einem der Ansprüche 1 bis 4, wobei die Reaktion in Gegenwart von $NAD(P)^+$ erfolgt.

**6.** Verfahren zur Herstellung von Ursodesoxycholsäure (UDCS) der Formel (1)

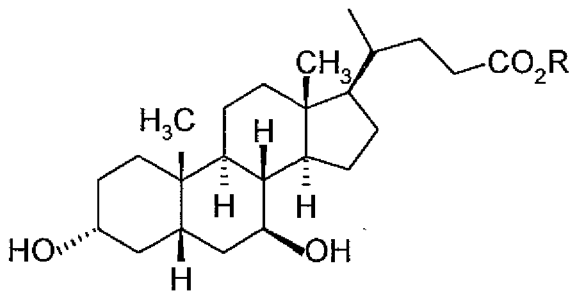

(1)

worin
R für Alkyl, H, ein Alkalimetallion oder $N(R^3)_4^+$steht, worin die Reste $R^3$ gleich oder verschieden sind und für H oder Alkyl stehen,
wobei man

a) eine Cholsäure (CS) der Formel (2)

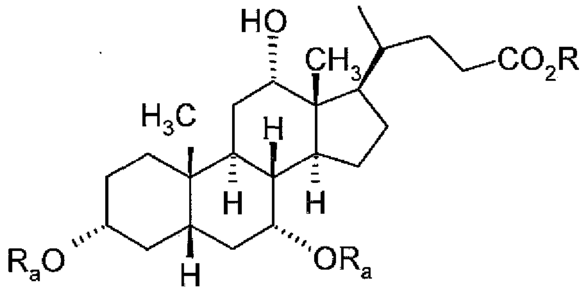

(2)

worin R die oben angegebenen Bedeutungen besitzen, und die Reste $R_a$ gleich oder verschieden sind und für H oder Acyl stehen, in Gegenwart einer 12$\alpha$-Hydroxysteroiddehydrogenase und in Gegenwart eines Cofaktorregenerierungssystems, umfassend

a) eine NAD(P)H Dehydrogenase mit Cholsäuretoleranz, ausgewählt unter Flavodoxin Reduktasen (FDR; EC 1.18.1.2), NADPH Dehydrogenase aus *G. stearothermophilus* Sulfit Reduktasen (SR; EC 1.8.1.2), und Reduktase Domänen von CYP102A1 (EC 1.6.2.4) und davon abgeleiteten funktionalen Äquivalenten, die zur Cofaktorregenerierung befähigt sind und eine Sequenzidentität von wenigstens 85 % zur nativen Sequenz besitzen; und
b) einen Redox-Mediator, welcher Elektronen auf molekularen Sauerstoff überträgt, zur korrespondierenden 12-Ketochenodesoxycholsäure (12-Keto CDCS) der Formel (3)

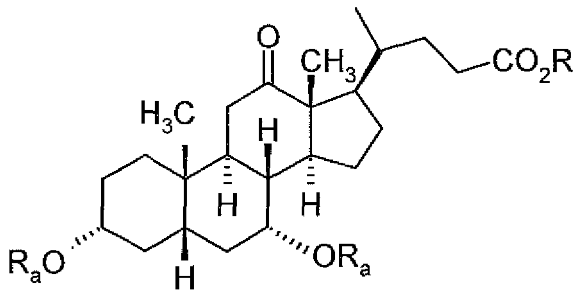

(3)

worin R und $R_a$ die oben angebebenen Bedeutungen besitzen, enzymatisch oxidiert ;

b) 12-Keto-CDCS der Formel (3) durch Desoxygenierung zu Chenodesoxycholsäure (CDCS) der Formel (4)

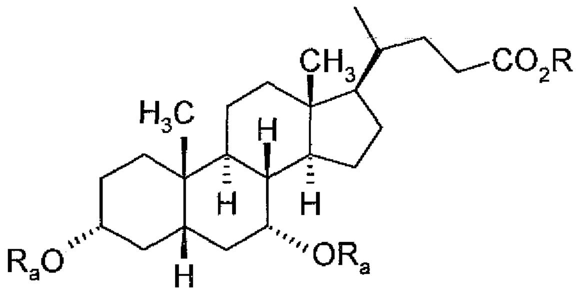

(4)

worin R und $R_a$ die oben angebebenen Bedeutungen besitzen, umsetzt;
c) CDCS der Formel (4) in Position 7 chemisch oxidiert zur 7-Keto-Lithocholsäure (KLCS) der Formel (5)

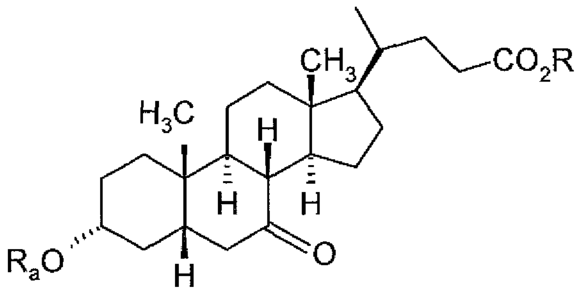

(5)

worin R und $R_a$ die oben angebebenen Bedeutungen besitzen;

d) KLCS der Formel (5) reduziert; und

e) das Reaktionsprodukt gegebenenfalls weiter aufreinigt, wobei, wenn $R_a$ für Acyl steht, diese Acylgruppe nach Durchführung der Reaktionsstufe b) oder d) gegebenenfalls abspaltet.

**7.** Verfahren nach Anspruch 6, wobei Stufe a) in Gegenwart des Cofaktors $NAD(P)^+$ erfolgt.

**8.** Verfahren nach einem der Ansprüche 6 und 7, wobei Stufe a) mit einer 12α-Hydroxysteroiddehydrogenase und/oder einer NAD(P)H Dehydrogenase mit Cholsäuretoleranz jeweils in immobilisierter Form erfolgt.

**9.** Verfahren nach einem der Ansprüche 1 bis 8, das in Gegenwart von Sauerstoff durchgeführt wird.

**10.** Verfahren nach Anspruch 9, wobei der Cofaktor einen $E_0$-Wert kleiner als der $E_0$-Wert der Dehydrogenase besitzt.

**11.** Verfahren nach Anspruch 9, wobei der Cofaktor ausgewählt ist unter $NADH/NAD^+$ und $NADPH/NADP^+$.

**12.** Verfahren nach einem der vorhergehenden Ansprüche, wobei der Mediator ausgewählt ist unter Methylenblau, Dichlorphenolindophenol (DCIP), Riboflavin, Ferricyanid und Indigocarmin.

## Claims

**1.** Method for enzymatic oxidation of 12α-hydroxysteroids of the general formula

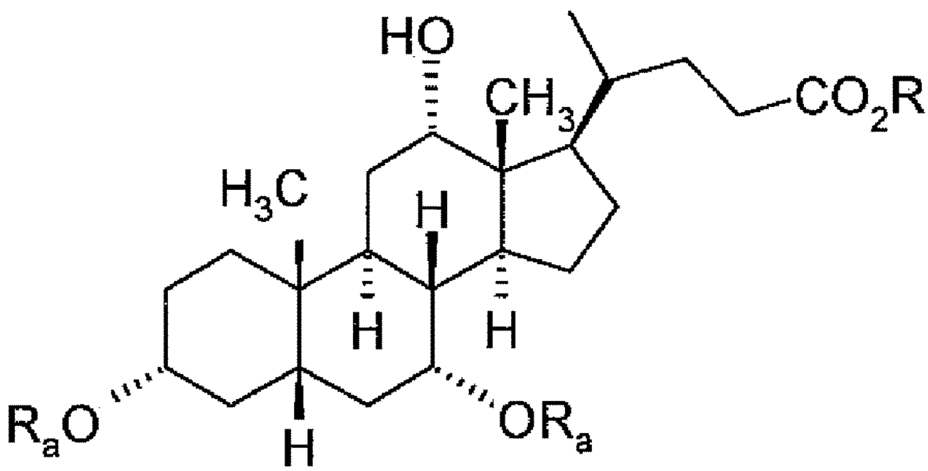

(2)

where R is alkyl, H is an alkali metal ion or $N(R^3)_4^+$, where the radicals $R^3$ are identical or different and are H or alkyl, and the radicals $R_a$ are identical or different and are H or acyl,

wherein the hydroxysteroid is reacted in the presence of a 12α-hydroxysteroid dehydrogenase and in the presence of a cofactor regeneration system, comprising

a) a NAD(P)H dehydrogenase with cholic acid tolerance, selected from flavodoxin reductases (FDR; EC 1.18.1.2), NADPH dehydrogenase of G. *stearothermophilus* sulfite reductases (SR; EC 1.8.1.2), and reductase

domains of CYP102A1 (EC 1.6.2.4) and functional equivalents derived therefrom which are capable of cofactor regeneration and have a sequence identity of at least 85 % of the native sequence; and
b) a redox mediator which transfers electrons to molecular oxygen and optionally isolates at least one formed oxidation product from the reaction mixture.

**2.** Method according to claim 1, wherein the hydroxysteroid is cholic acid (CS) or a cholic acid derivative.

**3.** Method according to claim 2, wherein the cholic acid derivative is a salt, amide or alkylester.

**4.** Method according to either claim 2 or claim 3, wherein CS or a derivative thereof is converted to 12-ketochenodeoxycholic acid (12-Keto-CDCS) or to the corresponding derivative.

**5.** Method according to any of claims 1 to 4, wherein the reaction takes place in the presence of $NAD(P)^+$.

**6.** Method for preparing urso-deoxycholic acid (UDCS) of formula (1)

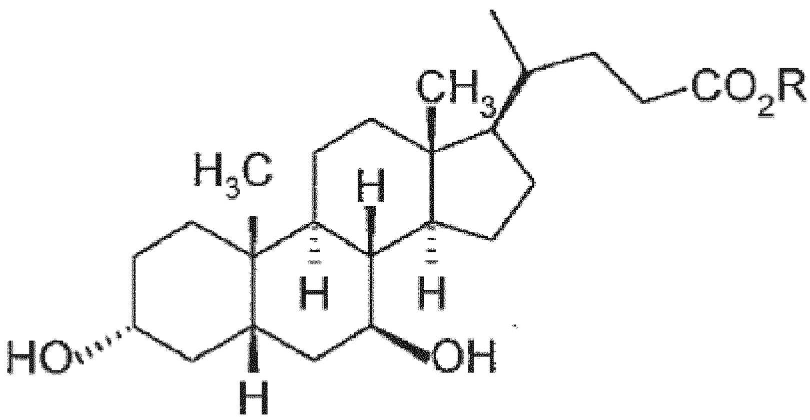

(1)

where R is alkyl, H is an alkali metal ion or $N(R^3)_4^+$, where the radicals $R^3$ are identical or different and are H or alkyl, wherein

a) a cholic acid (CS) of formula (2)

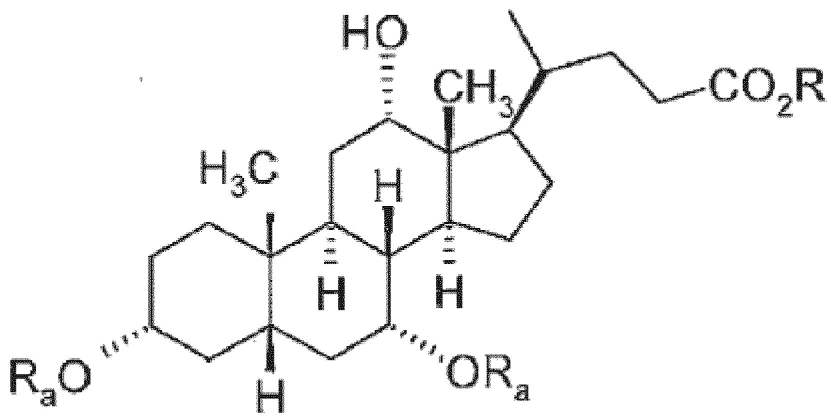

(2)

where R has the above-mentioned meanings, and the radicals $R_a$ are identical or different and are H or acyl, in the presence of a 12α-hydroxysteroid dehydrogenase and in the presence of a cofactor regeneration system, comprising

a) a NAD(P)H dehydrogenase with cholic acid tolerance, selected from flavodoxin reductases (FDR; EC 1.18.1.2), NADPH dehydrogenase of G. *stearothermophilus* sulphite reductases (SR; EC 1.8.1.2), and reductase domains of CYP102A1 (EC 1.6.2.4) and functional equivalents derived therefrom which are

capable of cofactor regeneration and have a sequence identity of at least 85 % of the native sequence; and
b) a redox mediator, which transfers electrons to molecular oxygen,

is enzymatically oxidised to corresponding 12-ketocheno-deoxycholic acid (12-keto CDCS) of formula (3)

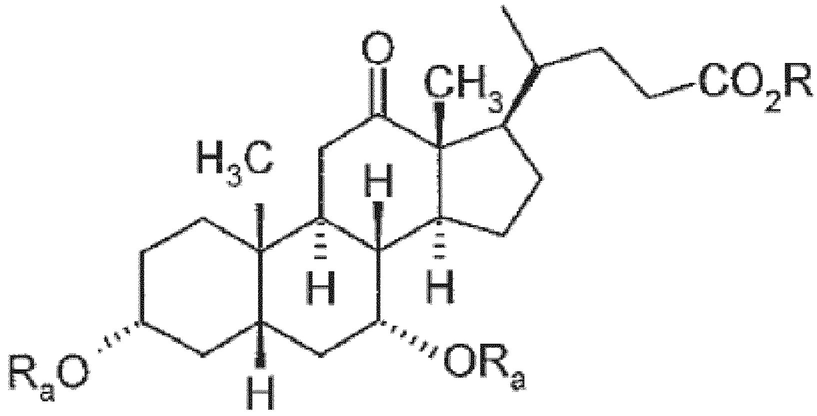

(3)

where R and $R_a$ have the above-mentioned meaning;
b) 12-keto CDCS of formula (3) is converted to cheno-deoxycholic acid (CDCS) of formula (4) by deoxygenation

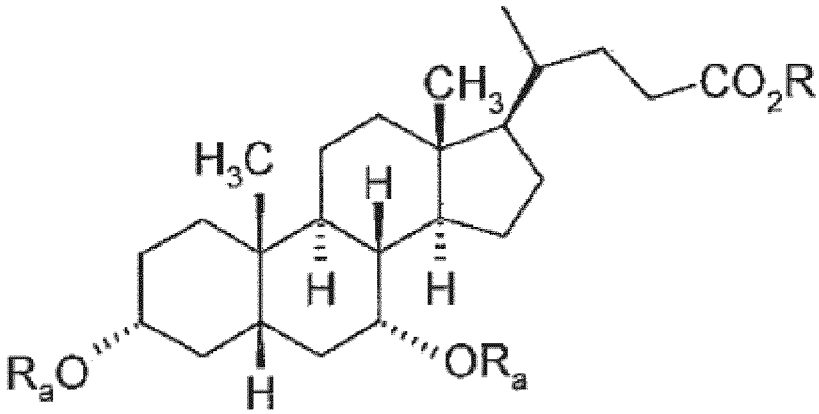

(4)

where R and $R_a$ have the above-mentioned meanings;
c) CDCS of formula (4) in position 7 is chemically oxidated to 7-keto-lithocholic acid (KLCS) of formula (5)

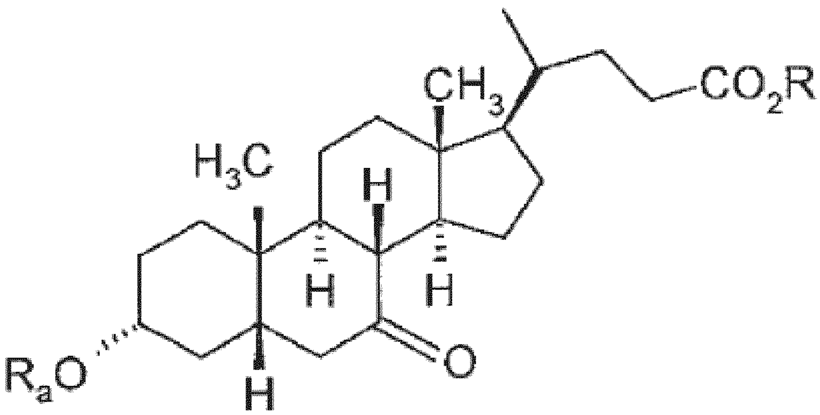

(5)

where R and $R_a$ have the above-mentioned meanings;
d) KLCS of formula (5) is reduced; and

e) the reaction product is optionally further purified, wherein, when $R_a$ is acyl, this acyl group is optionally separated after carrying out reaction step b) or d).

**7.** Method according to claim 6, wherein step a) is carried out in the presence of cofactor $NAD(P)^+$.

**8.** Method according to any of claims 6 and 7, wherein step a) is carried out with a 12α-hydroxysteroid dehydrogenase in immobilised form and/or a NAD(P)H dehydrogenase with cholic acid tolerance in immobilised form.

**9.** Method according to any of claims 1 to 8 which is carried out in the presence of oxygen.

**10.** Method according to claim 9, wherein the cofactor has an $E_0$-value smaller than the $E_0$-value of the dehydrogenase.

**11.** Method according to claim 9, wherein the cofactor is selected from NADH/$NAD^+$ and NADPH/$NADP^+$.

**12.** Method according to any of the preceding claims, wherein the mediator is selected from among methylene blue, dichlorophenolindophenol (DCIP), riboflavin, ferricyanide and indigo carmine.

**Revendications**

**1.** Procédé pour l'oxydation enzymatique des 12α-hydroxystéroïdes de formule général :

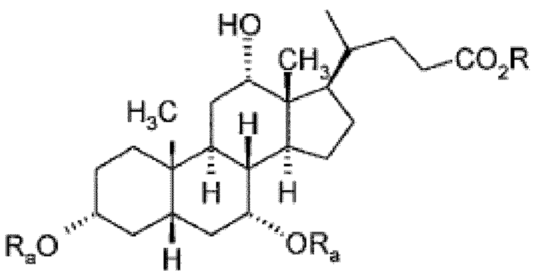

(2)

dans laquelle
R représente un groupe alkyle, H, un ion de métal alcalin ou $N(R^3)_4{}^+$, où les résidus $R^3$ sont identiques ou différents et représentent H ou un groupe alkyle, et
les résidus $R_a$ sont identiques ou différents et représentent H ou un groupe acyle,
dans lequel on fait réagir l'hydroxystéroïde en présence d'une 12α-hydroxystéroïde déshydrogénase et en présence d'un système de régénération de cofacteurs, comprenant

a) une NAD(P)H déshydrogénase présentant une tolérance pour l'acide cholique, choisie parmi les flavodoxine réductases (FDR ; EC 1.18.1.2), la NADPH déshydrogénase provenant des réductases de sulfite de *G. stearothermophilus* (SR ; EC 1.8.1.2), et les domaines de la réductase de CYP102A1 (EC 1.6.2.4) et les équivalents fonctionnels qui en sont dérivés, qui sont capables d'assurer une régénération des cofacteurs et possèdent une identité de séquence d'au moins 85 % par rapport à la séquence native ; et
b) un médiateur rédox, qui transfère des électrons à l'oxygène moléculaire, et dans lequel on isole le cas échéant au moins un produit d'oxydation formé à partir de la préparation réactionnelle.

**2.** Procédé selon la revendication 1, dans lequel l'hydroxystéroïde est l'acide cholique (CS) ou un dérivé d'acide cholique.

**3.** Procédé selon la revendication 2, dans lequel le dérivé d'acide cholique est un sel, un amide ou un ester d'alkyle.

**4.** Procédé selon la revendication 2 ou 3, dans lequel on fait réagir le CS ou un dérivé de celui-ci en acide 12-céto-chénodésoxycholique (12-céto-CDCS) ou en le dérivé correspondant.

**5.** Procédé selon l'une des revendications 1 à 4, dans lequel la réaction s'effectue en présence de $NAD(P)^+$.

**6.** Procédé pour la préparation d'acide ursodésoxycholique (UDCS) de formule (1)

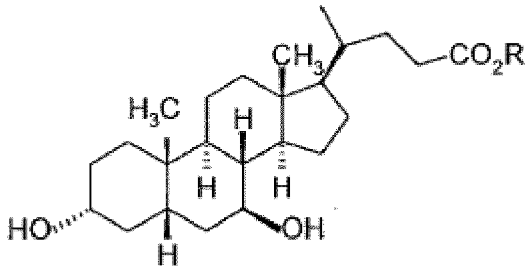

(1)

dans laquelle R représente un groupe alkyle, H, un ion de métal alcalin ou $N(R^3)_4^+$, où les résidus $R^3$ sont identiques ou différents et représentent H ou un groupe alkyle,
dans lequel

a) on oxyde enzymatiquement un acide cholique (CS) de formule (2)

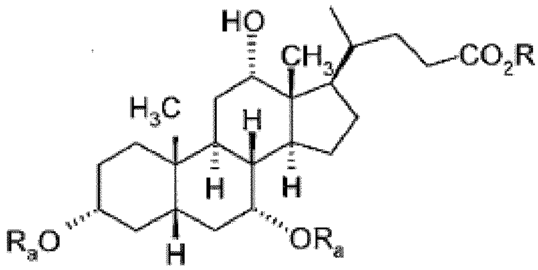

(2)

dans laquelle R possèdent les significations indiquées ci-dessus, et les résidus $R_a$ sont identiques ou différents et représentent H ou un groupe acyle, en présence d'une 12α-hydroxystéroïde déshydrogénase et en présence d'un système de régénération de cofacteurs, comprenant

a) une NAD(P)H déshydrogénase présentant une tolérance pour l'acide cholique, choisie parmi les flavodoxine réductases (FDR ; EC 1.18.1.2), la NADPH déshydrogénase provenant des réductases de sulfite de *G. stearothermophilus* (SR ; EC 1.8.1.2), et les domaines de la réductase de CYP102A1 (EC 1.6.2.4) et les équivalents fonctionnels qui en sont dérivés, qui sont capables d'assurer une régénération des cofacteurs et possèdent une identité de séquence d'au moins 85 % par rapport à la séquence native ; et
b) un médiateur rédox, qui transfère des électrons à l'oxygène moléculaire,

pour donner l'acide 12-céto-chénodésoxycholique (12-céto CDCS) correspondant de formule (3)

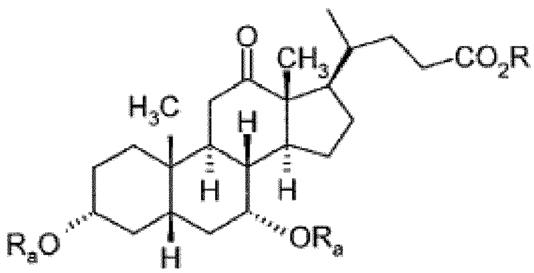

(3)

dans laquelle R et $R_a$ possèdent les significations indiquées ci-dessus ;
b) on fait réagir le 12-céto-CDCS de formule (3), par désoxygénation, pour donner l'acide chénodésoxycholique (CDCS) de formule (4)

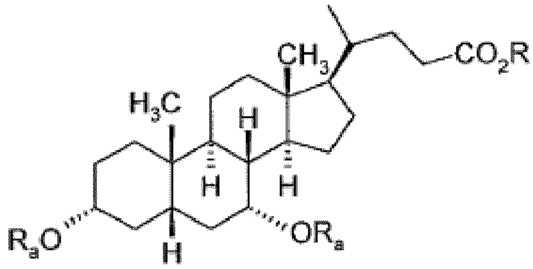

(4)

dans laquelle R et $R_a$ possèdent les significations indiquées ci-dessus ;
c) on oxyde chimiquement en position 7 le CDCS de formule (4) pour donner l'acide 7-céto-lithocholique (KLCS) de formule (5)

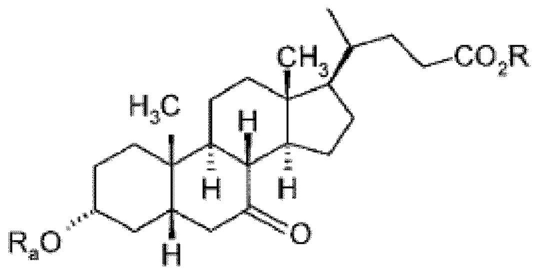

(5)

dans laquelle R et $R_a$ possèdent les significations indiquées ci-dessus ;
d) on réduit le KLCS de formule (5) ; et
e) on continue à purifier le cas échéant le produit réactionnel, en clivant le cas échéant, quand $R_a$ représente un groupe acyle, ce groupe acyle après réalisation de l'étape réactionnelle b) ou d).

**7.** Procédé selon la revendication 6, dans lequel l'étape a) s'effectue en présence du cofacteur $NAD(P)^+$.

**8.** Procédé selon l'une des revendications 6 et 7, dans lequel l'étape a) s'effectue avec une 12α-hydroxystéroïde déshydrogénase et/ou une NAD(P)H déshydrogénase présentant une tolérance pour l'acide cholique respectivement sous une forme immobilisée.

**9.** Procédé selon l'une des revendications 1 à 8, qui est réalisé en présence d'oxygène.

**10.** Procédé selon la revendication 9, dans lequel le cofacteur possède une valeur $E_0$ inférieure à la valeur $E_0$ de la déshydrogénase.

**11.** Procédé selon la revendication 9, dans lequel le cofacteur est choisi parmi la NADH/$NAD^+$ et la NADPH/$NADP^+$.

**12.** Procédé selon l'une des revendications précédentes, dans lequel le médiateur est choisi parmi le bleu de méthylène, le dichlorophénol-indophénol (DCIP), la riboflavine, le ferricyanure, et l'indigocarmine.

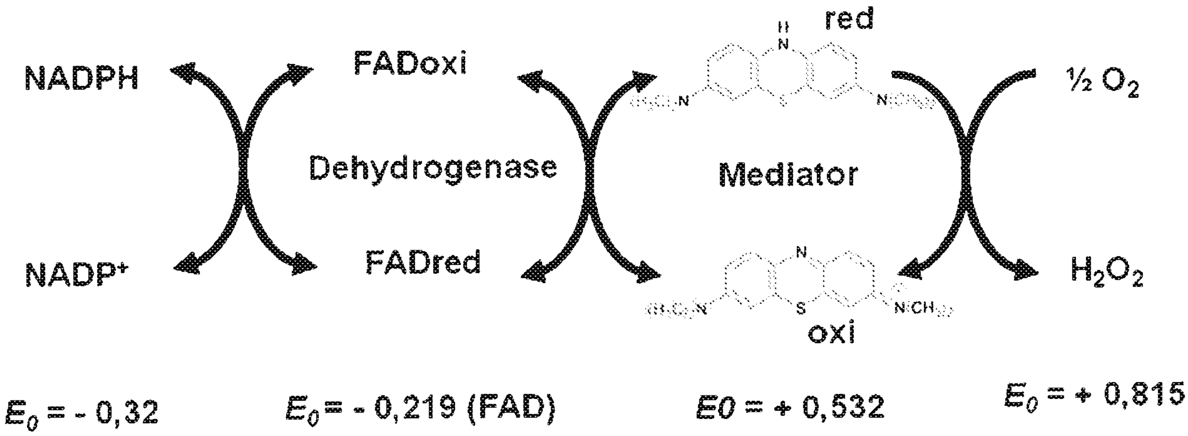
NADPH
FADoxi
red
½ $O_2$
Dehydrogenase
Mediator
$NADP^+$
FADred
$H_2O_2$
oxi
$E_0$ = - 0,32
$E_0$ = - 0,219 (FAD)
E0 = + 0,532
$E_0$ = + 0,815

**Fig.1**

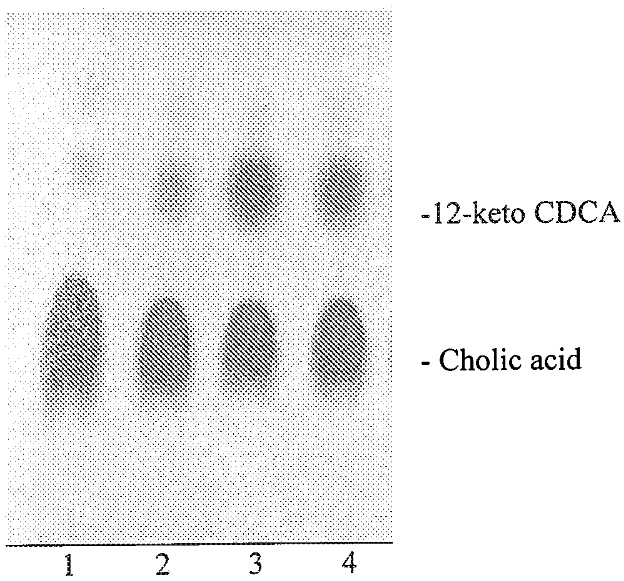
-12-keto CDCA
- Cholic acid
1
2
3
4

**Fig.2**

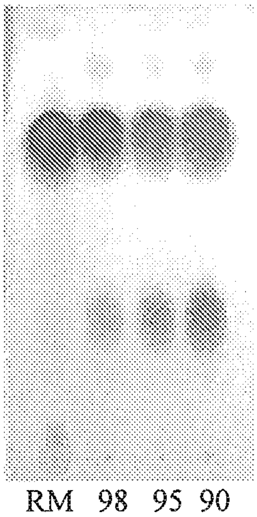


Fig.3

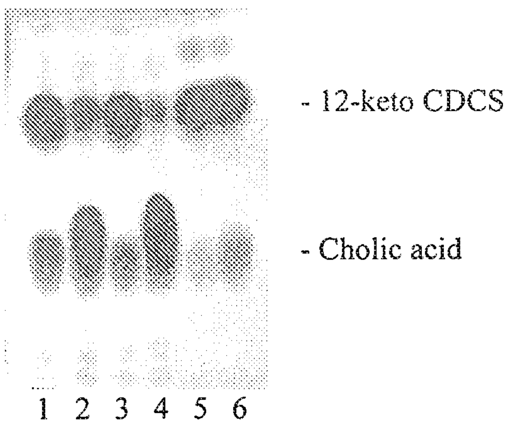


Fig.4

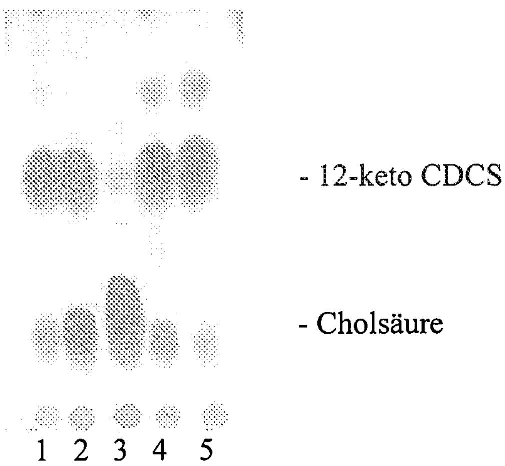


Fig.5

ATGGGCAGCAGCCATCATCATCATCATCACAGCAGCGGCCTGGTGCCGCGCGGCAGCCAT
ATGGCTAGCATGACTGGTGGACAGCAAATGGGTCGCGGATCCATGAAAAAGGCAGAAAAC
GCTCATAATACGCCGCTGCTTGTGCTATACGGTTCAAATATGGGAACAGCTGAAGGAACG
GCGCGTGATTTAGCAGATATTGCAATGAGCAAAGGATTTGCACCGCAGGTCGCAACGCTT
GATTCACACGCCGGAAATCTTCCGCGCGAAGGAGCTGTATTAATTGTAACGGCGTCTTAT
AACGGTCATCCGCCTGATAACGCAAAGCAATTTGTCGACTGGTTAGACCAAGCGTCTGCT
GATGAAGTAAAAGGCGTTCGCTACTCCGTATTTGGATGCGGCGATAAAAACTGGGCTACT
ACGTATCAAAAAGTGCCTGCTTTTATCGATGAAACGCTTGCCGCTAAAGGGGCAGAAAAC
ATCGCTGACCGCGGTGAAGCAGATGCAAGCGACGACTTTGAAGGCACATATGAAGAATGG
CGTGAACATATGTGGAGTGACGTAGCAGCCTACTTTAACCTCGACATTGAAAACAGTGAA
GATAATAAATCTACTCTTTCACTTCAATTTGTCGACAGCGCCGCGGATATGCCGCTTGCG
AAAATGCACGGTGCGTTTTCAACGAACGTCGTAGCAAGCAAAGAACTTCAACAGCCAGGC
AGTGCACGAAGCACGCGACATCTTGAAATTGAACTTCCAAAAGAAGCTTCTTATCAAGAA
GGAGATCATTTAGGTGTTATTCCTCGCAACTATGAAGGAATAGTAAACCGTGTAACAGCA
AGGTTCGGCCTAGATGCATCACAGCAAATCCGTCTGGAAGCAGAAGAAGAAAAATTAGCT
CATTTGCCACTCGCTAAAACAGTATCCGTAGAAGAGCTTCTGCAATACGTGGAGCTTCAA
GATCCTGTTACGCGCACGCAGCTTCGCGCAATGGCTGCTAAAACGGTCTGCCCGCCGCAT
AAAGTAGAGCTTGAAGCCTTGCTTGAAAAGCAAGCCTACAAAGAACAAGTGCTGGCAAAA
CGTTTAACAATGCTTGAACTGCTTGAAAAATACCCGGCGTGTGAAATGAAATTCAGCGAA
TTTATCGCCCTTCTGCCAAGCATACGCCCGCGCTATTACTCGATTTCTTCATCACCTCGT
GTCGATGAAAAACAAGCAAGCATCACGGTCAGCGTTGTCTCAGGAGAAGCGTGGAGCGGA
TATGGAGAATATAAAGGAATTGCGTCGAACTATCTTGCCGAGCTGCAAGAAGGAGATACG
ATTACGTGCTTTATTTCCACACCGCAGTCAGAATTTACGCTGCCAAAAGACCCTGAAACG
CCGCTTATCATGGTCGGACCGGGAACAGGCGTCGCGCCGTTTAGAGGCTTTGTGCAGGCG
CGCAAACAGCTAAAAGAACAAGGACAGTCACTTGGAGAAGCACATTTATACTTCGGCTGC
CGTTCACCTCATGAAGACTATCTGTATCAAGAAGAGCTTGAAAACGCCCAAAGCGAAGGC
ATCATTACGCTTCATACCGCTTTTTCTCGCATGCCAAATCAGCCGAAAACATACGTTCAG
CACGTAATGGAACAAGACGGCAAGAAATTGATTGAACTTCTTGATCAAGGAGCGCACTTC
TATATTTGCGGAGACGGAAGCCAAATGGCACCTGCCGTTGAAGCAACGCTTATGAAAAGC
TATGCTGACGTTCACCAAGTGAGTGAAGCAGACGCTCGCTTATGGCTGCAGCAGCTAGAA
GAAAAAGGCCGATACGCAAAAGACGTGTGGGCTGGGTAG

MGSSHHHHHHSSGLVPRGSHMASMTGGQQMGRGSMKKAENAHNTPLLVLY
GSNMGTAEGTARDLADIAMSKGFAPQVATLDSHAGNLPREGAVLIVTASY
NGHPPDNAKQFVDWLDQASADEVKGVRYSVFGCGDKNWATTYQKVPAFID
ETLAAKGAENIADRGEADASDDFEGTYEEWREHMWSDVAAYFNLDIENSE
DNKSTLSLQFVDSAADMPLAKMHGAFSTNVVASKELQQPGSARSTRHLEI
ELPKEASYQEGDHLGVIPRNYEGIVNRVTARFGLDASQQIRLEAEEEKLA
HLPLAKTVSVEELLQYVELQDPVTRTQLRAMAAKTVCPPHKVELEALLEK
QAYKEQVLAKRLTMLELLEKYPACEMKFSEFIALLPSIRPRYYSISSSPR
VDEKQASITVSVVSGEAWSGYGEYKGIASNYLAELQEGDTITCFISTPQS
EFTLPKDPETPLIMVGPGTGVAPFRGFVQARKQLKEQGQSLGEAHLYFGC
RSPHEDYLYQEELENAQSEGIITLHTAFSRMPNQPKTYVQHVMEQDGKKL
IELLDQGAHFYICGDGSQMAPAVEATLMKSYADVHQVSEADARLWLQQLE
EKGRYAKDVWAG

**Fig.6A**

>gi|49175990:c4112495-4111749 Escherichia coli str. K-12 substr. MG1655,
complete menome

ATGGCTGATTGGGTAACAGGCAAAGTCACTAAAGTGCAGAACTGGACCGACGCCCTGTTTAGTCTCACCGTTCAC
GCCCCCGTGCTTCCGTTTACCGCCGGGCAATTTACCAAGCTTGGCCTTGAAATCGACGGCGAACGCGTCCAGCGC
GCCTACTCCTATGTAAACTCGCCCGATAATCCCGATCTGGAGTTTTACCTGGTCACCGTCCCCGATGGCAAATTA
AGCCCACGACTGGCGGCACTGAAACCAGGCGATGAAGTGCAGGTGGTTAGCGAAGCGGCAGGATTCTTTGTGCTC
GATGAAGTGCCGCACTGCGAAACGCTATGGATGCTGGCAACCGGTACAGCGATTGGCCCTTATTTATCGATTCTG
CAACTAGGTAAAGATTTAGATCGCTTCAAAAATCTGGTCCTGGTGCACGCCGCACGTTATGCCGCCGACTTAAGC
TATTTGCCACTGATGCAGGAACTGGAAAAACGCTACGAAGGAAAACTGCGCATTCAGACGGTGGTCAGTCGGGAA
ACGGCAGCGGGGTCGCTCACCGGACGGATACCGGCATTAATTGAAAGTGGGGAACTGGAAAGCACGATTGGCCTG
CCGATGAATAAAGAAACCAGCCATGTGATGCTGTGCGGCAATCCACAGATGGTGCGCGATACACAACAGTTGCTG
AAAGAGACCCGGCAGATGACGAAACATTTACGTCGCCGACCGGGCCATATGACAGCGGAGCATTACTGGTAA

MADWVTGKVT KVQNWTDALF SLTVHAPVLP FTAGQFTKLG LEIDGERVQR
AYSYVNSPDNPDLEFYLVTV PDGKLSPRLA ALKPGDEVQV VSEAAGFFVL DEVPHCETLW
MLATGTAIGPYLSILQLGKD LDRFKNLVLV HAARYAADLS YLPLMQELEK RYEGKLRIQT
VVSRETAAGSLTGRIPALIE SGELESTIGL PMNKETSHVM LCGNPQMVRD TQQLLKETRQ MTKHLRRRPG
HMTAEHYW

**Fig.6B**

ATGGGCAGCAGCCATCATCATCATCATCACAGCAGCGGCCTGGTGCCGCGCGGCAGCCAT
ATGGCTAGCATGACTGGTGGACAGCAAATGGGTCGCGGATCCGAATCTGTGACGGTATTG
TACGGCTCGCAAACCGGCAATGCACAAAAGTTGGCTGAGAAAGCCGGCAAAGCGCTCAAA
GAGCGTGGTTTTGAAGCGAAAGTGTTGTCCATGCTTGATTTTAAGCCGAACGAATTAAAG
AAAGTGGAGACGCTGCTCATTGTCGTGAGCACGCATGGGGAAGGCGATCCGCCGGACAAT
GCGGTATCGTTTTACGAATTTCTCCACAGCAAACGGGCGCCAAAGCTCAACCATCTTCGT
TTCTCTGTCTTGGCGCTCGGCGATACGTCGTATGAACATTTTTGCCAGACGGGGAAAGAT
TTCGACAAACGTCTTGAGGAGCTGGGTGGAACACGGTTTTATCCGCGCGTCGATTGCGAT
GTCGACTATGAAGAAGCGGCGGCGAAATGGCTTGATGGCGTGCTTGGCGAGTTAAGCAAA
GAAGCGAATGCCCATGTTGGGGCCACGCCGCTTTTGTCCGCTGCGGCCACGGCGCCAAAA
ATGGAACCAGCTGTCGTCTATTCGCGGAAAAACCCGTTCCCGGCCGAGGTGCTTGAAAAC
ATCAACTTAAACGGCCGCGGATCGAACAAGGAAACGCGCCATTTGGAATTGTCGCTCGAG
GGATCGGGGTTGAAGTACGAACCGGGCGATGCGCTTGGCATTTTCCCGAAAAATGATCCC
GAGCTGGTTGAGCGGATCATTCAAGAAATGAAATGGAATCCGGAAGAAACGGTGACGATC
GACAAAGATGGGGAAGTGCGGTCGCTGCGTGAAGCGCTCACCTCACATTTTGAAATTACG
GTGCTGACGAAAGCGCTGTTGCAAAAGCTGGCGACGCTGTCAAAAAACAGCGAGCTCCAA
GCGCTTGTGGCGCCCGGCAATGAAGCCAAATTGAAAGAATACGCCAAAGGCCGTGATTTG
TTGGATGCGCTCCGCGATTTCGGCCCATGGGACGCCACTTTGCAGCAACTCATCTCCATC
TTGCGGAAAATGCCGCCGCGCCTGTACTCGATCGCGAGCAGCTTAGCGGCCTATCCAGAT
GAAGTGCACTTGACGATCGGTGCGGTCCGTTATGAGTCGCACGGCCGCCTGCGCAAAGGG
GTGTGCTCGACGTTCTGCGCCGAACGCGTCCAAATCGGCGATACGTTGCCGGTCTTTGTG
CAGCCGAACCCGAACTTTAAGTTGCCGAAGGATCCCGACACGCCGATCATTATGATCGGT
CCAGGCACCGGAGTGGCGCCGTTCCGCGCCTTTATGCAAGAGCGCGAGGCCATCGGGGCG
AAAGGAAAATCGTGGCTCTTTTTCGGGGACCAACACTTTATGACCGACTTCCTGTATCAA
ACCGAATGGCTCGCATGGTTGAAAAGCGGTGTGCTGACCAAAATGGATGTCGCCTTCTCT
CGCGATACGGAGAAGAAAGTGTATGTGCAGCACCGGATGCTTGAACGAAGCAAAGAGCTG
TTCGGTTGGCTTGAGGAAGGCGCTGTCGTCTACGTTTGCGGCGACAAGCAACACATGGCG
CGCGACGTTCATCAGACGTTGATCGAGATTATCGAAAAAGAGGGCGGCATGAGCCGCGAA
CAGGCGGAAGCGTATGTCACCGAGATGCAAAAGCAAAAACGGTATCAACGCGACGTCTAC
TAA

MGSSHHHHHHSSGLVPRGSHMASMTGGQQMGRGSESVTVLYGSQTGNAQK
LAEKAGKALKERGFEAKVLSMLDFKPNELKKVETLLIVVSTHGEGDPPDN
AVSFYEFLHSKRAPKLNHLRFSVLALGDTSYEHFCQTGKDFDKRLEELGG
TRFYPRVDCDVDYEEAAAKWLDGVLGELSKEANAHVGATPLLSAAATAPK
MEPAVVYSRKNPFPAEVLENINLNGRGSNKETRHLELSLEGSGLKYEPGD
ALGIFPKNDPELVERIIQEMKWNPEETVTIDKDGEVRSLREALTSHFEIT
VLTKALLQKLATLSKNSELQALVAPGNEAKLKEYAKGRDLLDALRDFGPW
DATLQQLISILRKMPPRLYSIASSLAAYPDEVHLTIGAVRYESHGRLRKG
VCSTFCAERVQIGDTLPVFVQPNPNFKLPKDPDTPIIMIGPGTGVAPFRA
FMQEREAIGAKGKSWLFFGDQHFMTDFLYQTEWLAWLKSGVLTKMDVAFS
RDTEKKVYVQHRMLERSKELFGWLEEGAVVYVCGDKQHMARDVHQTLIEI
IEKEGGMSREQAEAYVTEMQKQKRYQRDVY

Fig.6C

>gi|170079663:4123470-4124171 Escherichia coli str. K-12 substr. DH10B, complete genome
ATGACAACCTTAAGCTGTAAAGTGACCTCGGTAGAAGCTATCACGGATACCGTATATCGTGTCCGCATCG
TGCCAGACGCGGCCTTTTCTTTTCGTGCTGGTCAGTATTTGATGGTAGTGATGGATGAGCGCGACAAACG
TCCGTTCTCAATGGCTTCGACGCCGGATGAAAAAGGGTTTATCGAGCTGCATATTGGCGCTTCTGAAATC
AACCTTTACGCGAAAGCAGTCATGGACCGCATCCTCAAAGATCATCAAATCGTGGTCGACATTCCCCACG
GAGAAGCGTGGCTGCGCGATGATGAAGAGCGTCCGATGATTTTGATTGCGGGCGGCACCGGGTTCTCTTA
TGCCCGCTCGATTTTGCTGACAGCGTTGGCGCGTAACCCAAACCGTGATATCACCATTTACTGGGGCGGG
CGTGAAGAGCAGCATCTGTATGATCTCTGCGAGCTTGAGGCGCTTTCGTTGAAGCATCCTGGTCTGCAAG
TGGTGCCGGTGGTTGAACAACCGGAAGCGGGCTGGCGTGGGCGTACTGGCACCGTGTTAACGGCGGTATT
GCAGGATCACGGTACGCTGGCAGAGCATGATATCTATATTGCCGGACGTTTTGAGATGGCGAAAATTGCC
CGCGATCTGTTTTGCAGTGAGCGTAATGCGCGGGAAGATCGCCTGTTTGGCGATGCGTTTGCATTTATCT
GA

>sp|P0AEN1|FRE_ECOLI NAD(P)H-flavin reductase OS=Escherichia coli
>sp|P0AEN1|(strain K12) GN=fre PE=1 SV=2
MTTLSCKVTSVEAITDTVYRVRIVPDAAFSFRAGQYLMVVMDERDKRPFSMASTPDEKGF
IELHIGASEINLYAKAVMDRILKDHQIVVDIPHGEAWLRDDEERPMILIAGGTGFSYARS
ILLTALARNPNRDITIYWGGREEQHLYDLCELEALSLKHPGLQVVPVVEQPEAGWRGRTG
TVLTAVLQDHGTLAEHDIYIAGRFEMAKIARDLFCSERNAREDRLFGDAFAFI

**Fig.6D**

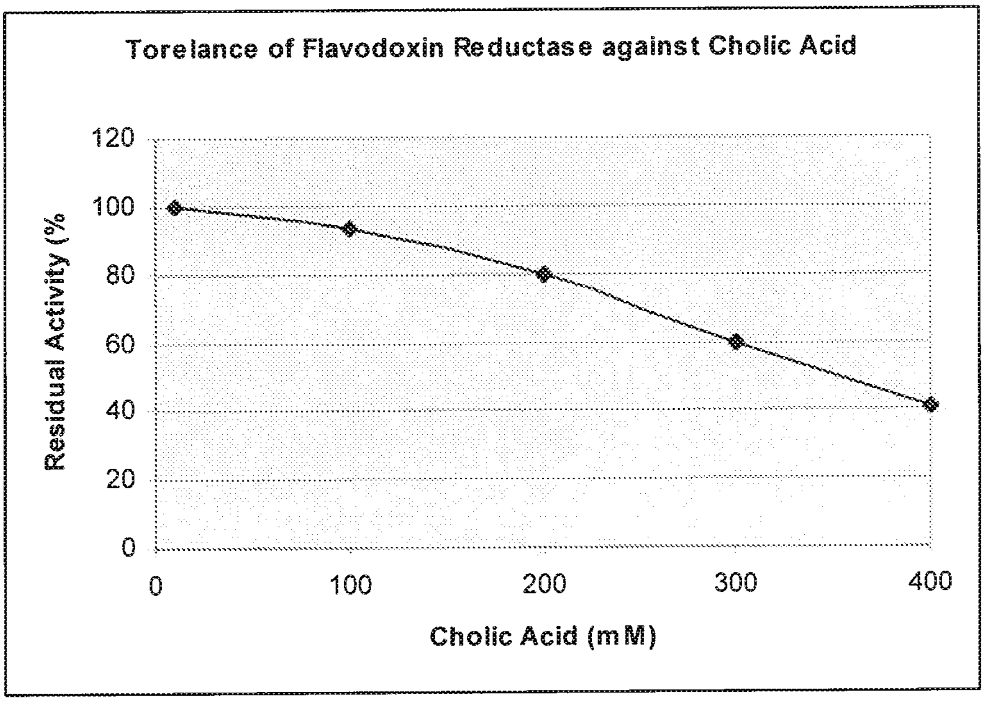
Torelance of Flavodoxin Reductase against Cholic Acid
Residual Activity (%
120
100
80
60
40
20
0
0
100
200
300
400
Cholic Acid (mM)

Fig.7

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 2009002190 W
- WO 2009118176 A
- WO 03071283 A
- EP 0424232 A
- EP 0230085 A
- ES 489661
- US 4547271 A
- EP 0386538 A
- JP 60006699 B
- IT 2000MI1177
- EP 1149849 A
- EP 1069183 A
- DE 100193773 A


### In der Beschreibung aufgeführte Nicht-Patentliteratur


- **R. WICHMANN.** *Adv. Biochem. Engin / Biotechnol,* 2005, vol. 92, 225-260
- **LEE, L. G. ; WHITESIDES, G.M.** *J. Am. Chem.Soc.,* 1985, vol. 107, 6999-7008
- **DRUECKHAMMER, D. G. et al.** *J. Org. Chem.,* 1985, vol. 50, 5387-5389
- **GREEN DE ; NEEDHAM DM ; DEWAN JG.** *Biochem J.,* 1937, vol. 31 (12), 2327-52
- **FUJII K ; HUENNEKENS FM.** *J Biol Chem.,* 1974, vol. 249 (21), 6745-53
- **COVES J ; NIVIERE V ; ESCHENBRENNER M ; FONTECAVE M.** *J Biol Chem.,* 1993, vol. 268 (25), 18604-9
- **NARHI LO ; FULCO AJ.** *J Biol Chem.,* 1986, vol. 261 (16), 7160-9
- **DUANE W ; HASTINGS JW.** *Mol Cell Biochem.,* 1975, vol. 6 (1), 53-64
- **PEARSON ; LIPMAN.** *Proc. Natl. Acad, Sci. (USA),* 1988, vol. 85 (8), 2444-2448
- **NARANG, S.A.** *Tetrahedron,* 1983, vol. 39, 3
- **ITAKURA et al.** *Annu. Rev. Biochem.,* 1984, vol. 53, 323
- **ITAKURA et al.** *Science,* 1984, vol. 198, 1056
- **IKE et al.** *Nucleic Acids Res.,* 1983, vol. 11, 477
- **ARKIN ; YOURVAN.** *PNAS,* 1992, vol. 89, 7811-7815
- **DELGRAVE et al.** *Protein Engineering,* 1993, vol. 6 (3), 327-331
- **HIGGINS DG ; SHARP PM.** Fast and sensitive multiple sequence alignments on a microcomputer. *Comput Appl. Biosci.,* April 1989, vol. 5 (2), 151-1
- **CHENNA ; RAMU ; SUGAWARA ; HIDEAKI ; KOIKE ; TADASHI ; LOPEZ ; RODRIGO ; GIBSON ; TOBY J.** Multiple sequence alignment with the Clustal series of programs. *Nucleic Acids Res,* 2003, vol. 31 (13), 3497-500
- Phosphoamiditmethode. Wiley Press, 896-897
- **SAMBROOK et al.** Molecular Cloning: A laboratory manual. Cold Spring Harbor Laboratory Press, 1989
- **SAMBROOK, J. ; FRITSCH, E.F. ; MANIATIS, T.** Molecular Cloning: A Laboratory Manual. Cold Spring Harbor Laboratory Press, 1989
- **SAMBROOK et al.** Molecular Cloning. Cold Spring Harbor Laboratory, 1989
- Current Protocols in Molecular Biology. John Wiley & Sons, 1985
- Nucleic Acids Hybridization: A Practical Approach. IRL Press at Oxford University Press, 1985
- **1991.** Essential Molecular Biology: A Practical Approach. IRL Press at Oxford University Press
- **SAMBROOK, J. ; FRITSCH, E.F. ; MANIATIS, T.** Molecular Cloning (A Laboratory Manual). Cold Spring Harbor Laboratory Press, 1989, 9.31-9.57
- Current Protocols in Molecular Biology. John Wiley & Sons, 1989, 6.3.1-6.3.6
- **SAMBROOK ; RUSSELL.** Molecular Cloning. Cold Spring Harbor Laboratory Press, 2001
- In vitro mutagenesis protocols. Humana Press, 1996
- **KEGLER-EBO DM ; DOCKTOR CM ; DIMAIO D.** *Nucleic Acids Res,* 1994, vol. 22, 1593
- **BARETTINO D ; FEIGENBUTZ M ; VALCÁREL R ; STUNNENBERG HG.** *Nucleic Acids Res,* 1994, vol. 22, 541
- **BARIK S.** *Mol Biotechnol,* 1995, vol. 3, 1
- **ECKERT KA ; KUNKEL TA.** *Nucleic Acids Res,* 1990, vol. 18, 3739
- An efficient random mutagenesis technique u-sing an E.coli mutator strain. **GREENER A ; CALLAHAN M ; JERPSETH B.** In: Trower MK (Hrsg.) In vitro mutagenesis protocols. Humana Press, 1996

- **STEMMER WPC.** *Nature,* 1994, vol. 370, 389
- **STEMMER WPC.** *Proc Natl Acad Sci USA,* 1994, vol. 91, 10747
- **REETZ MT ; JAEGER K-E.** *Topics Curr Chem,* 1999, vol. 200, 31
- Methods for optimizing industrial enzymes. **ZHAO H ; MOORE JC ; VOLKOV AA ; ARNOLD FH.** Manual of industrial microbiology and biotechnology. American Society for Microbiology, 1999
- **GOEDDEL.** Gene Expression Technology: Methods in Enzymology. Academic Press, 1990, vol. 185
- Buch Cloning Vectors. Elsevier, 1985
- **T. MANIATIS ; E.F. FRITSCH ; J. SAMBROOK.** Molecular Cloning: A Laboratory Manual. Cold Spring Harbor Laboratory, 1989
- **T.J. SILHAVY, M.L. BERMAN ; L.W. ENQUIST.** Experiments with Gene Fusions. Cold Spring Harbor Laboratory, 1984
- **AUSUBEL, F.M. et al.** Current Protocols in Molecular Biology. Greene Publishing Assoc. and Wiley Interscience, 1987
- Cloning Vectors. Elsevier, 1985
- Current Protocols in Molecular Biology. Wiley Interscience, 1997
- **SAMBROOK et al.** Molecular Cloning: A Laboratory Manual. Cold Spring Harbor Laboratory Press, 1989
- **HARRIS ; HYLEMON.** *Biochim Biophys Acta,* 1978, vol. 528 (1), 148-57
- **MACDONALD et al.** *Biochim Biophys Acta,* 1976, vol. 450 (2), 142-53
- *Experientia,* vol. 37 (5), 451-2
- **C. GIORDANO et al.** *Angew. Chem.,* 1985, vol. 97, 510
- Bioverfahrenstechnik. Gustav Fischer Verlag, 1991
- Bioreaktoren und periphere Einrichtungen. Vieweg Verlag, 1994
- Manual of Methods for General Bacteriology. American Society for Bacteriology, 1981
- Applied Microbiol. Physiology, A Practical Approach. IRL Press, 1997, 53-73
- **HARLOW, E. ; LANE, D.** Antibodies: A Laboratory Manual. Cold Spring Harbor (N.Y.) Press, 1988
- Immobilization of Enzymes. **J. LALONDE ; A. MARGOLIN.** Enzyme Catalysis in Organic Synthesis. Wiley-VCH, 2002, vol. III, 991-1032
- **SAMBROCK et al.** Molecular Cloning. Cold Spring Harbor Laboratory Press, 1989
- **GIRHARD, M. et al.** Cytochrom P450 monooxygenase from Clostridium acetobutylicum: A new alpha-fatty acid hydroxylase. *Biochem. Biophysic. Res. Commun.,* 2007, vol. 362, 114-119
- **JENKINS, C.M. ; WATERMAN, M.R.** NADPH-Flavodoxin reductase and flavodoxin from Escherichia coli: Characteristics as a soluble Microsomal P450 Reductase. *Biochemistry,* 1998, vol. 37, 6106-6113
- **EIBEN, S.** Cyp102A P450 Monooxygenases: Comparative analysis and construction of cytochrome P450 chimera. *Dissertation, Institut für Technische Biochemie der Universität Stuttgart,* 2007

SEQUENCE LISTING

<110> PHARMAZELL GmbH

<120> NAD(P)+-Cofaktorregenerierungssystem und dessen Anwendungen

<130> M/50102-EP

<160> 30

<170> PatentIn version 3.3

<210> 1
<211> 813
<212> DNA

<213> Clostridium sp.

<220>
<221> CDS
<222> (1)..(813)

<400> 1

atg gat ttt att gat ttt aag gag atg ggc aga atg ggg atc ttt gac 48
Met Asp Phe Ile Asp Phe Lys Glu Met Gly Arg Met Gly Ile Phe Asp
1 5 10 15

gga aag gtc gca atc att act ggc ggg ggc aag gcc aaa tcg atc ggc 96
Gly Lys Val Ala Ile Ile Thr Gly Gly Gly Lys Ala Lys Ser Ile Gly
20 25 30

tac ggc att gcc gtg gcc tat gct aag gag ggg gcc aac ctg gtc ctg 144
Tyr Gly Ile Ala Val Ala Tyr Ala Lys Glu Gly Ala Asn Leu Val Leu
35 40 45

acc ggc aga aac gag cag aaa ctg ctg gac gcc aag gag gag ctg gag 192
Thr Gly Arg Asn Glu Gln Lys Leu Leu Asp Ala Lys Glu Glu Leu Glu
50 55 60

cgc ctc tac ggc atc aag gtg ttg ccg ctg gcg gtg gac gtc acc ccc 240
Arg Leu Tyr Gly Ile Lys Val Leu Pro Leu Ala Val Asp Val Thr Pro
65 70 75 80

agc gat gag tcg gag gac cgg gtc aag gaa gcc gtg cag aag gtc atc 288
Ser Asp Glu Ser Glu Asp Arg Val Lys Glu Ala Val Gln Lys Val Ile
85 90 95

gcc gaa ttc ggc cgc atc gac gtg ctg atc aac aac gcc cag gcg tcg 336
Ala Glu Phe Gly Arg Ile Asp Val Leu Ile Asn Asn Ala Gln Ala Ser
100 105 110

gcc tcg ggc atc ccc ctg tcc atg cag acc aaa gac cac ttt gac ctg 384
Ala Ser Gly Ile Pro Leu Ser Met Gln Thr Lys Asp His Phe Asp Leu
115 120 125

ggc atc tac tcc ggg ctc tac gcc acc ttc tac tac atg agg gag tgc 432
Gly Ile Tyr Ser Gly Leu Tyr Ala Thr Phe Tyr Tyr Met Arg Glu Cys
130 135 140

tat ccc tac ctg aag gag acc cag ggc tcg gtc atc aac ttc gcc tcc 480
Tyr Pro Tyr Leu Lys Glu Thr Gln Gly Ser Val Ile Asn Phe Ala Ser
145 150 155 160

ggc gcc ggc ctc ttc ggc aac gtg ggt cag tgc tcc tac gcc gcc gcc 528
Gly Ala Gly Leu Phe Gly Asn Val Gly Gln Cys Ser Tyr Ala Ala Ala
165 170 175

aaa gag ggc atc cgc ggc ctc tcc cgc gtc gcg gcc acc gag tgg ggc 576
Lys Glu Gly Ile Arg Gly Leu Ser Arg Val Ala Ala Thr Glu Trp Gly

```
            180                     185                     190

aag gac aac atc aac gtc aac gtg gtc tgc ccc ctg gcc atg acc gcc       624
Lys Asp Asn Ile Asn Val Asn Val Val Cys Pro Leu Ala Met Thr Ala
        195                 200                 205

cag ctg gag aac ttc aag ctc tcc tac cct gag gcc tac gag aaa aac       672
Gln Leu Glu Asn Phe Lys Leu Ser Tyr Pro Glu Ala Tyr Glu Lys Asn
    210                 215                 220

ctc aga ggg gtg ccc atg ggc cgc ttc ggt gac ccc gag ctg gac atc       720
Leu Arg Gly Val Pro Met Gly Arg Phe Gly Asp Pro Glu Leu Asp Ile
225                 230                 235                 240

ggc cgg gtc tgc gtg cag ctc ggc tcg ccc gac ttc aag tac atg tcc       768
Gly Arg Val Cys Val Gln Leu Gly Ser Pro Asp Phe Lys Tyr Met Ser
                245                 250                 255

ggc gag acc ctc acc ctg gaa ggc ggc atg ggt cag cgc ccc tag           813
Gly Glu Thr Leu Thr Leu Glu Gly Gly Met Gly Gln Arg Pro
            260                 265                 270
```

<210> 2
<211> 270
<212> PRT
<213> Clostridium sp.

<400> 2

```
Met Asp Phe Ile Asp Phe Lys Glu Met Gly Arg Met Gly Ile Phe Asp
1               5                   10                  15

Gly Lys Val Ala Ile Ile Thr Gly Gly Gly Lys Ala Lys Ser Ile Gly
            20                  25                  30

Tyr Gly Ile Ala Val Ala Tyr Ala Lys Glu Gly Ala Asn Leu Val Leu
        35                  40                  45

Thr Gly Arg Asn Glu Gln Lys Leu Leu Asp Ala Lys Glu Glu Leu Glu
    50                  55                  60

Arg Leu Tyr Gly Ile Lys Val Leu Pro Leu Ala Val Asp Val Thr Pro
65                  70                  75                  80

Ser Asp Glu Ser Glu Asp Arg Val Lys Glu Ala Val Gln Lys Val Ile
                85                  90                  95

Ala Glu Phe Gly Arg Ile Asp Val Leu Ile Asn Asn Ala Gln Ala Ser
            100                 105                 110

Ala Ser Gly Ile Pro Leu Ser Met Gln Thr Lys Asp His Phe Asp Leu
        115                 120                 125

Gly Ile Tyr Ser Gly Leu Tyr Ala Thr Phe Tyr Tyr Met Arg Glu Cys
    130                 135                 140

Tyr Pro Tyr Leu Lys Glu Thr Gln Gly Ser Val Ile Asn Phe Ala Ser
145                 150                 155                 160

Gly Ala Gly Leu Phe Gly Asn Val Gly Gln Cys Ser Tyr Ala Ala Ala
                165                 170                 175

Lys Glu Gly Ile Arg Gly Leu Ser Arg Val Ala Ala Thr Glu Trp Gly
            180                 185                 190

Lys Asp Asn Ile Asn Val Asn Val Val Cys Pro Leu Ala Met Thr Ala
        195                 200                 205

Gln Leu Glu Asn Phe Lys Leu Ser Tyr Pro Glu Ala Tyr Glu Lys Asn
    210                 215                 220

Leu Arg Gly Val Pro Met Gly Arg Phe Gly Asp Pro Glu Leu Asp Ile
225                 230                 235                 240

Gly Arg Val Cys Val Gln Leu Gly Ser Pro Asp Phe Lys Tyr Met Ser
                245                 250                 255

Gly Glu Thr Leu Thr Leu Glu Gly Gly Met Gly Gln Arg Pro
            260                 265                 270
```

<210> 3
<211> 774
<212> DNA
<213> Artificial

<220>
<223> Künstliche Kurzsequenz

<220>
<221> CDS
<222> (1)..(774)

<400> 3

```
atc ttt gac gga aag gtc gca atc att act ggc ggg ggc aag gcc aaa        48
Ile Phe Asp Gly Lys Val Ala Ile Ile Thr Gly Gly Gly Lys Ala Lys
1               5                   10                  15

tcg atc ggc tac ggc att gcc gtg gcc tat gct aag gag ggg gcc aac        96
Ser Ile Gly Tyr Gly Ile Ala Val Ala Tyr Ala Lys Glu Gly Ala Asn
            20                  25                  30

ctg gtc ctg acc ggc aga aac gag cag aaa ctg ctg gac gcc aag gag       144
Leu Val Leu Thr Gly Arg Asn Glu Gln Lys Leu Leu Asp Ala Lys Glu
        35                  40                  45

gag ctg gag cgc ctc tac ggc atc aag gtg ttg ccg ctg gcg gtg gac       192
Glu Leu Glu Arg Leu Tyr Gly Ile Lys Val Leu Pro Leu Ala Val Asp
    50                  55                  60

gtc acc ccc agc gat gag tcg gag gac cgg gtc aag gaa gcc gtg cag       240
Val Thr Pro Ser Asp Glu Ser Glu Asp Arg Val Lys Glu Ala Val Gln
65                  70                  75                  80

aag gtc atc gcc gaa ttc ggc cgc atc gac gtg ctg atc aac aac gcc       288
Lys Val Ile Ala Glu Phe Gly Arg Ile Asp Val Leu Ile Asn Asn Ala
                85                  90                  95

cag gcg tcg gcc tcg ggc atc ccc ctg tcc atg cag acc aaa gac cac       336
Gln Ala Ser Ala Ser Gly Ile Pro Leu Ser Met Gln Thr Lys Asp His
```

```
                100                     105                     110

ttt gac ctg ggc atc tac tcc ggg ctc tac gcc acc ttc tac tac atg          384
Phe Asp Leu Gly Ile Tyr Ser Gly Leu Tyr Ala Thr Phe Tyr Tyr Met
        115                     120                     125

agg gag tgc tat ccc tac ctg aag gag acc cag ggc tcg gtc atc aac          432
Arg Glu Cys Tyr Pro Tyr Leu Lys Glu Thr Gln Gly Ser Val Ile Asn
    130                     135                     140

ttc gcc tcc ggc gcc ggc ctc ttc ggc aac gtg ggt cag tgc tcc tac          480
Phe Ala Ser Gly Ala Gly Leu Phe Gly Asn Val Gly Gln Cys Ser Tyr
145                     150                     155                     160

gcc gcc gcc aaa gag ggc atc cgc ggc ctc tcc cgc gtc gcg gcc acc          528
Ala Ala Ala Lys Glu Gly Ile Arg Gly Leu Ser Arg Val Ala Ala Thr
                165                     170                     175

gag tgg ggc aag gac aac atc aac gtc aac gtg gtc tgc ccc ctg gcc          576
Glu Trp Gly Lys Asp Asn Ile Asn Val Asn Val Val Cys Pro Leu Ala
            180                     185                     190

atg acc gcc cag ctg gag aac ttc aag ctc tcc tac cct gag gcc tac          624
Met Thr Ala Gln Leu Glu Asn Phe Lys Leu Ser Tyr Pro Glu Ala Tyr
        195                     200                     205

gag aaa aac ctc aga ggg gtg ccc atg ggc cgc ttc ggt gac ccc gag          672
Glu Lys Asn Leu Arg Gly Val Pro Met Gly Arg Phe Gly Asp Pro Glu
    210                     215                     220

ctg gac atc ggc cgg gtc tgc gtg cag ctc ggc tcg ccc gac ttc aag          720
Leu Asp Ile Gly Arg Val Cys Val Gln Leu Gly Ser Pro Asp Phe Lys
225                     230                     235                     240

tac atg tcc ggc gag acc ctc acc ctg gaa ggc ggc atg ggt cag cgc          768
Tyr Met Ser Gly Glu Thr Leu Thr Leu Glu Gly Gly Met Gly Gln Arg
                245                     250                     255

ccc tag                                                                   774
Pro
```

<210> 4
<211> 257
<212> PRT
<213> Artificial

<220>
<223> Synthetic Construct

<400> 4

```
Ile Phe Asp Gly Lys Val Ala Ile Ile Thr Gly Gly Gly Lys Ala Lys
1               5                   10                  15

Ser Ile Gly Tyr Gly Ile Ala Val Ala Tyr Ala Lys Glu Gly Ala Asn
            20                  25                  30

Leu Val Leu Thr Gly Arg Asn Glu Gln Lys Leu Leu Asp Ala Lys Glu
        35                  40                  45

Glu Leu Glu Arg Leu Tyr Gly Ile Lys Val Leu Pro Leu Ala Val Asp
    50                  55                  60

Val Thr Pro Ser Asp Glu Ser Glu Asp Arg Val Lys Glu Ala Val Gln
65                  70                  75                  80

Lys Val Ile Ala Glu Phe Gly Arg Ile Asp Val Leu Ile Asn Asn Ala
                85                  90                  95

Gln Ala Ser Ala Ser Gly Ile Pro Leu Ser Met Gln Thr Lys Asp His
            100                 105                 110

Phe Asp Leu Gly Ile Tyr Ser Gly Leu Tyr Ala Thr Phe Tyr Tyr Met
        115                 120                 125

Arg Glu Cys Tyr Pro Tyr Leu Lys Glu Thr Gln Gly Ser Val Ile Asn
    130                 135                 140

Phe Ala Ser Gly Ala Gly Leu Phe Gly Asn Val Gly Gln Cys Ser Tyr
145                 150                 155                 160

Ala Ala Ala Lys Glu Gly Ile Arg Gly Leu Ser Arg Val Ala Ala Thr
                165                 170                 175

Glu Trp Gly Lys Asp Asn Ile Asn Val Asn Val Val Cys Pro Leu Ala
            180                 185                 190

Met Thr Ala Gln Leu Glu Asn Phe Lys Leu Ser Tyr Pro Glu Ala Tyr
        195                 200                 205

Glu Lys Asn Leu Arg Gly Val Pro Met Gly Arg Phe Gly Asp Pro Glu
    210                 215                 220

Leu Asp Ile Gly Arg Val Cys Val Gln Leu Gly Ser Pro Asp Phe Lys
225                 230                 235                 240

Tyr Met Ser Gly Glu Thr Leu Thr Leu Glu Gly Gly Met Gly Gln Arg
                245                 250                 255

Pro
```

<210> 5
<211> 4
<212> PRT
<213> Artificial

<220>
<223> Teilsequenz

<400> 5

```
Leu Ile Asn Asn
1
```

<210> 6
<211> 41
<212> PRT
<213> Artificial

<220>
<223> N-terminale Teilsequenz Langversion

<400> 6

```
Met Asp Phe Ile Asp Phe Lys Glu Met Gly Arg Met Gly Ile Phe Asp
1               5                   10                  15

Gly Lys Val Ala Ile Ile Thr Gly Gly Gly Lys Ala Lys Ser Ile Gly
            20                  25                  30

Tyr Gly Ile Ala Val Ala Tyr Ala Lys
        35                  40
```

<210> 7
<211> 14
<212> PRT
<213> Artificial

<220>
<223> Nterminale Teilsequenz Langversion

<400> 7

```
Met Asp Phe Ile Asp Phe Lys Glu Met Gly Arg Met Gly Ile
1               5                   10
```

<210> 8
<211> 15
<212> PRT
<213> Artificial

<220>
<223> Nterrminale Teilsequenz Kurzversion

<400> 8

```
Ile Thr Gly Gly Gly Lys Ala Lys Ser Ile Gly Tyr Gly Ile Ala
1               5                   10                  15
```

<210> 9
<211> 5
<212> PRT
<213> Artificial

<220>
<223> Nterminale Teilsequenz Kurzversion

<400> 9

```
Ile Phe Asp Gly Lys
1               5
```

<210> 10
<211> 6
<212> PRT
<213> Artificial

<220>
<223> Nterminale Teilsequenz Kurzversion

<400> 10

```
Gly Ile Phe Asp Gly Lys
1               5
```

<210> 11
<211> 6
<212> PRT
<213> Artificial

<220>
<223> Teilsequenz 2

<400> 11

```
Arg Met Gly Ile Phe Asp
1               5
```

<210> 12
<211> 7
<212> PRT
<213> Artificial

<220>
<223> Teilsequenzmotiv

<400> 12

```
Val Leu Thr Gly Arg Asn Glu
1               5
```

<210> 13
<211> 8
<212> PRT
<213> Artificial

<220>

<223> Cterminale Teilsequenz

<400> 13

```
Phe Gly Asp Pro Glu Leu Asp Ile
1               5
```

<210> 14
<211> 37
<212> DNA
<213> Artificial

<220>
<223> PCR Primer forward lang

<400> 14
ggtattccat atggatttta ttgattttaa ggagatg 37

<210> 15
<211> 33
<212> DNA
<213> Artificial

<220>
<223> PCR Primer Forward kurz

<400> 15
ggtattccat atgatctttg acggaaaggt cgc 33

<210> 16
<211> 24
<212> DNA
<213> Artificial

<220>
<223> PCR Primer Revers

<400> 16
cgggatccct aggggcgctg accc 24

<210> 17
<211> 777
<212> DNA
<213> Artificial

<220>
<223> Mutant Q97H

<220>
<221> CDS
<222> (1)..(777)

<400> 17

```
atg atc ttt gac gga aag gtc gca atc att act ggc ggg ggc aag gcc      48
Met Ile Phe Asp Gly Lys Val Ala Ile Ile Thr Gly Gly Gly Lys Ala
1               5                   10                  15

aaa tcg atc ggc tac ggc att gcc gtg gcc tat gct aag gag ggg gcc      96
Lys Ser Ile Gly Tyr Gly Ile Ala Val Ala Tyr Ala Lys Glu Gly Ala
            20                  25                  30

aac ctg gtc ctg acc ggc aga aac gag cag aaa ctg ctg gac gcc aag     144
Asn Leu Val Leu Thr Gly Arg Asn Glu Gln Lys Leu Leu Asp Ala Lys
        35                  40                  45

gag gag ctg gag cgc ctc tac ggc atc aag gtg ttg ccg ctg gcg gtg     192
Glu Glu Leu Glu Arg Leu Tyr Gly Ile Lys Val Leu Pro Leu Ala Val
    50                  55                  60

gac gtc acc ccc agc gat gag tcg gag gac cgg gtc aag gaa gcc gtg     240
Asp Val Thr Pro Ser Asp Glu Ser Glu Asp Arg Val Lys Glu Ala Val
65                  70                  75                  80

cag aag gtc atc gcc gaa ttc ggc cgc atc gac gtg ctg atc aac aac     288
Gln Lys Val Ile Ala Glu Phe Gly Arg Ile Asp Val Leu Ile Asn Asn
                85                  90                  95

gcc cat gcg tcg gcc tcg ggc atc ccc ctg tcc atg cag acc aaa gac     336
Ala His Ala Ser Ala Ser Gly Ile Pro Leu Ser Met Gln Thr Lys Asp
            100                 105                 110

cac ttt gac ctg ggc atc tac tcc ggg ctc tac gcc acc ttc tac tac     384
His Phe Asp Leu Gly Ile Tyr Ser Gly Leu Tyr Ala Thr Phe Tyr Tyr
        115                 120                 125

atg agg gag tgc tat ccc tac ctg aag gag act cag ggc tcg gtc atc     432
Met Arg Glu Cys Tyr Pro Tyr Leu Lys Glu Thr Gln Gly Ser Val Ile
    130                 135                 140

aac ttc gcc tcc ggc gcc ggc ctc ttc ggc aac gtg ggt cag tgc tcc     480
Asn Phe Ala Ser Gly Ala Gly Leu Phe Gly Asn Val Gly Gln Cys Ser
145                 150                 155                 160

tac gcc gcc gcc aaa gag ggc atc cgc ggc ctc tcc cgc gtc gcg gcc     528
Tyr Ala Ala Ala Lys Glu Gly Ile Arg Gly Leu Ser Arg Val Ala Ala
                165                 170                 175

acc gag tgg ggc aag gac aac atc aac gtc aac gtg gtc tgc ccc ctg     576
Thr Glu Trp Gly Lys Asp Asn Ile Asn Val Asn Val Val Cys Pro Leu
            180                 185                 190

gcc atg acc gcc cag ctg gag aac ttc aag ctc tcc tac cct gag gcc     624
Ala Met Thr Ala Gln Leu Glu Asn Phe Lys Leu Ser Tyr Pro Glu Ala
        195                 200                 205

tac gag aaa aac ctc aga ggg gtg ccc atg ggc cgc ttc ggt gac ccc     672
Tyr Glu Lys Asn Leu Arg Gly Val Pro Met Gly Arg Phe Gly Asp Pro
    210                 215                 220

gag ctg gac atc ggc cgg gtc tgc gtg cag ctc ggc tcg ccc gac ttc     720
Glu Leu Asp Ile Gly Arg Val Cys Val Gln Leu Gly Ser Pro Asp Phe
225                 230                 235                 240

aag tac atg tcc ggc gag acc ctc acc ctg gaa ggc ggc atg ggt cag     768
Lys Tyr Met Ser Gly Glu Thr Leu Thr Leu Glu Gly Gly Met Gly Gln
                245                 250                 255

cgc ccc tag                                                         777
Arg Pro
```

<210> 18
<211> 258
<212> PRT
<213> Artificial

<220>
<223> Synthetic Construct

<400> 18

```
Met Ile Phe Asp Gly Lys Val Ala Ile Ile Thr Gly Gly Gly Lys Ala
1               5                   10                  15

Lys Ser Ile Gly Tyr Gly Ile Ala Val Ala Tyr Ala Lys Glu Gly Ala
            20                  25                  30

Asn Leu Val Leu Thr Gly Arg Asn Glu Gln Lys Leu Leu Asp Ala Lys
        35                  40                  45

Glu Glu Leu Glu Arg Leu Tyr Gly Ile Lys Val Leu Pro Leu Ala Val
    50                  55                  60

Asp Val Thr Pro Ser Asp Glu Ser Glu Asp Arg Val Lys Glu Ala Val
65                  70                  75                  80

Gln Lys Val Ile Ala Glu Phe Gly Arg Ile Asp Val Leu Ile Asn Asn
                85                  90                  95

Ala His Ala Ser Ala Ser Gly Ile Pro Leu Ser Met Gln Thr Lys Asp
            100                 105                 110

His Phe Asp Leu Gly Ile Tyr Ser Gly Leu Tyr Ala Thr Phe Tyr Tyr
        115                 120                 125
```

```
Met Arg Glu Cys Tyr Pro Tyr Leu Lys Glu Thr Gln Gly Ser Val Ile
    130                 135                 140

Asn Phe Ala Ser Gly Ala Gly Leu Phe Gly Asn Val Gly Gln Cys Ser
145                 150                 155                 160

Tyr Ala Ala Ala Lys Glu Gly Ile Arg Gly Leu Ser Arg Val Ala Ala
                165                 170                 175

Thr Glu Trp Gly Lys Asp Asn Ile Asn Val Asn Val Val Cys Pro Leu
            180                 185                 190

Ala Met Thr Ala Gln Leu Glu Asn Phe Lys Leu Ser Tyr Pro Glu Ala
        195                 200                 205

Tyr Glu Lys Asn Leu Arg Gly Val Pro Met Gly Arg Phe Gly Asp Pro
    210                 215                 220

Glu Leu Asp Ile Gly Arg Val Cys Val Gln Leu Gly Ser Pro Asp Phe
225                 230                 235                 240

Lys Tyr Met Ser Gly Glu Thr Leu Thr Leu Glu Gly Gly Met Gly Gln
                245                 250                 255

Arg Pro
```

<210> 19
<211> 33
<212> DNA
<213> Artificial

<220>
<223> PCR Primer

<400> 19
ccgcggatcc gaatctgtga cggtattgta cgg 33

<210> 20
<211> 33
<212> DNA
<213> Artificial

<220>
<223> PCR Primer

<400> 20
cccgtacaat accgtcacag attcggatcc gcg 33

<210> 21
<211> 29
<212> DNA
<213> Artificial

<220>
<223> PCR Primer

<400> 21
ggaattcata tgacaacctt aagctgtaa 29

<210> 22
<211> 30
<212> DNA
<213> Artificial

<220>
<223> PCR Primer

<400> 22
aaataagctt cagataaatg caaacgcatc 30

<210> 23
<211> 1839
<212> DNA
<213> Bacillus megaterium

<220>
<221> CDS
<222> (1)..(1839)

<400> 23

```
atg ggc agc agc cat cat cat cat cat cac agc agc ggc ctg gtg ccg      48
Met Gly Ser Ser His His His His His His Ser Ser Gly Leu Val Pro
1               5                   10                  15

cgc ggc agc cat atg gct agc atg act ggt gga cag caa atg ggt cgc      96
Arg Gly Ser His Met Ala Ser Met Thr Gly Gly Gln Gln Met Gly Arg
            20                  25                  30

gga tcc atg aaa aag gca gaa aac gct cat aat acg ccg ctg ctt gtg     144
Gly Ser Met Lys Lys Ala Glu Asn Ala His Asn Thr Pro Leu Leu Val
        35                  40                  45

cta tac ggt tca aat atg gga aca gct gaa gga acg gcg cgt gat tta     192
Leu Tyr Gly Ser Asn Met Gly Thr Ala Glu Gly Thr Ala Arg Asp Leu
    50                  55                  60

gca gat att gca atg agc aaa gga ttt gca ccg cag gtc gca acg ctt     240
Ala Asp Ile Ala Met Ser Lys Gly Phe Ala Pro Gln Val Ala Thr Leu
65                  70                  75                  80

gat tca cac gcc gga aat ctt ccg cgc gaa gga gct gta tta att gta     288
Asp Ser His Ala Gly Asn Leu Pro Arg Glu Gly Ala Val Leu Ile Val
                85                  90                  95

acg gcg tct tat aac ggt cat ccg cct gat aac gca aag caa ttt gtc     336
Thr Ala Ser Tyr Asn Gly His Pro Pro Asp Asn Ala Lys Gln Phe Val
            100                 105                 110

gac tgg tta gac caa gcg tct gct gat gaa gta aaa ggc gtt cgc tac     384
Asp Trp Leu Asp Gln Ala Ser Ala Asp Glu Val Lys Gly Val Arg Tyr
        115                 120                 125

tcc gta ttt gga tgc ggc gat aaa aac tgg gct act acg tat caa aaa     432
Ser Val Phe Gly Cys Gly Asp Lys Asn Trp Ala Thr Thr Tyr Gln Lys
    130                 135                 140

gtg cct gct ttt atc gat gaa acg ctt gcc gct aaa ggg gca gaa aac     480
Val Pro Ala Phe Ile Asp Glu Thr Leu Ala Ala Lys Gly Ala Glu Asn
145                 150                 155                 160

atc gct gac cgc ggt gaa gca gat gca agc gac gac ttt gaa ggc aca     528
```

```
Ile Ala Asp Arg Gly Glu Ala Asp Ala Ser Asp Asp Phe Glu Gly Thr
                165                 170                 175

tat gaa gaa tgg cgt gaa cat atg tgg agt gac gta gca gcc tac ttt      576
Tyr Glu Glu Trp Arg Glu His Met Trp Ser Asp Val Ala Ala Tyr Phe
            180                 185                 190

aac ctc gac att gaa aac agt gaa gat aat aaa tct act ctt tca ctt      624
Asn Leu Asp Ile Glu Asn Ser Glu Asp Asn Lys Ser Thr Leu Ser Leu
        195                 200                 205

caa ttt gtc gac agc gcc gcg gat atg ccg ctt gcg aaa atg cac ggt      672
Gln Phe Val Asp Ser Ala Ala Asp Met Pro Leu Ala Lys Met His Gly
    210                 215                 220

gcg ttt tca acg aac gtc gta gca agc aaa gaa ctt caa cag cca ggc      720
Ala Phe Ser Thr Asn Val Val Ala Ser Lys Glu Leu Gln Gln Pro Gly
225                 230                 235                 240

agt gca cga agc acg cga cat ctt gaa att gaa ctt cca aaa gaa gct      768
Ser Ala Arg Ser Thr Arg His Leu Glu Ile Glu Leu Pro Lys Glu Ala
                245                 250                 255

tct tat caa gaa gga gat cat tta ggt gtt att cct cgc aac tat gaa      816
Ser Tyr Gln Glu Gly Asp His Leu Gly Val Ile Pro Arg Asn Tyr Glu
            260                 265                 270

gga ata gta aac cgt gta aca gca agg ttc ggc cta gat gca tca cag      864
Gly Ile Val Asn Arg Val Thr Ala Arg Phe Gly Leu Asp Ala Ser Gln
        275                 280                 285

caa atc cgt ctg gaa gca gaa gaa gaa aaa tta gct cat ttg cca ctc      912
Gln Ile Arg Leu Glu Ala Glu Glu Glu Lys Leu Ala His Leu Pro Leu
    290                 295                 300

gct aaa aca gta tcc gta gaa gag ctt ctg caa tac gtg gag ctt caa      960
Ala Lys Thr Val Ser Val Glu Glu Leu Leu Gln Tyr Val Glu Leu Gln
305                 310                 315                 320

gat cct gtt acg cgc acg cag ctt cgc gca atg gct gct aaa acg gtc     1008
Asp Pro Val Thr Arg Thr Gln Leu Arg Ala Met Ala Ala Lys Thr Val
                325                 330                 335

tgc ccg ccg cat aaa gta gag ctt gaa gcc ttg ctt gaa aag caa gcc     1056
Cys Pro Pro His Lys Val Glu Leu Glu Ala Leu Leu Glu Lys Gln Ala
            340                 345                 350

tac aaa gaa caa gtg ctg gca aaa cgt tta aca atg ctt gaa ctg ctt     1104
Tyr Lys Glu Gln Val Leu Ala Lys Arg Leu Thr Met Leu Glu Leu Leu
        355                 360                 365

gaa aaa tac ccg gcg tgt gaa atg aaa ttc agc gaa ttt atc gcc ctt     1152
Glu Lys Tyr Pro Ala Cys Glu Met Lys Phe Ser Glu Phe Ile Ala Leu
    370                 375                 380

ctg cca agc ata cgc ccg cgc tat tac tcg att tct tca tca cct cgt     1200
Leu Pro Ser Ile Arg Pro Arg Tyr Tyr Ser Ile Ser Ser Ser Pro Arg
385                 390                 395                 400

gtc gat gaa aaa caa gca agc atc acg gtc agc gtt gtc tca gga gaa     1248
Val Asp Glu Lys Gln Ala Ser Ile Thr Val Ser Val Val Ser Gly Glu
                405                 410                 415

gcg tgg agc gga tat gga gaa tat aaa gga att gcg tcg aac tat ctt     1296
Ala Trp Ser Gly Tyr Gly Glu Tyr Lys Gly Ile Ala Ser Asn Tyr Leu
            420                 425                 430

gcc gag ctg caa gaa gga gat acg att acg tgc ttt att tcc aca ccg     1344
Ala Glu Leu Gln Glu Gly Asp Thr Ile Thr Cys Phe Ile Ser Thr Pro
```

```
        435                 440                 445

    cag tca gaa ttt acg ctg cca aaa gac cct gaa acg ccg ctt atc atg      1392
    Gln Ser Glu Phe Thr Leu Pro Lys Asp Pro Glu Thr Pro Leu Ile Met
        450                 455                 460

    gtc gga ccg gga aca ggc gtc gcg ccg ttt aga ggc ttt gtg cag gcg      1440
    Val Gly Pro Gly Thr Gly Val Ala Pro Phe Arg Gly Phe Val Gln Ala
    465                 470                 475                 480

    cgc aaa cag cta aaa gaa caa gga cag tca ctt gga gaa gca cat tta      1488
    Arg Lys Gln Leu Lys Glu Gln Gly Gln Ser Leu Gly Glu Ala His Leu
                    485                 490                 495

    tac ttc ggc tgc cgt tca cct cat gaa gac tat ctg tat caa gaa gag      1536
    Tyr Phe Gly Cys Arg Ser Pro His Glu Asp Tyr Leu Tyr Gln Glu Glu
                500                 505                 510

    ctt gaa aac gcc caa agc gaa ggc atc att acg ctt cat acc gct ttt      1584
    Leu Glu Asn Ala Gln Ser Glu Gly Ile Ile Thr Leu His Thr Ala Phe
            515                 520                 525

    tct cgc atg cca aat cag ccg aaa aca tac gtt cag cac gta atg gaa      1632
    Ser Arg Met Pro Asn Gln Pro Lys Thr Tyr Val Gln His Val Met Glu
        530                 535                 540

    caa gac ggc aag aaa ttg att gaa ctt ctt gat caa gga gcg cac ttc      1680
    Gln Asp Gly Lys Lys Leu Ile Glu Leu Leu Asp Gln Gly Ala His Phe
    545                 550                 555                 560

    tat att tgc gga gac gga agc caa atg gca cct gcc gtt gaa gca acg      1728
    Tyr Ile Cys Gly Asp Gly Ser Gln Met Ala Pro Ala Val Glu Ala Thr
                    565                 570                 575

    ctt atg aaa agc tat gct gac gtt cac caa gtg agt gaa gca gac gct      1776
    Leu Met Lys Ser Tyr Ala Asp Val His Gln Val Ser Glu Ala Asp Ala
                580                 585                 590

    cgc tta tgg ctg cag cag cta gaa gaa aaa ggc cga tac gca aaa gac      1824
    Arg Leu Trp Leu Gln Gln Leu Glu Glu Lys Gly Arg Tyr Ala Lys Asp
            595                 600                 605

    gtg tgg gct ggg tag                                                  1839
    Val Trp Ala Gly
        610
```

<210> 24
<211> 612
<212> PRT
<213> Bacillus megaterium

<400> 24

```
Met Gly Ser Ser His His His His His His Ser Ser Gly Leu Val Pro
1               5                   10                  15

Arg Gly Ser His Met Ala Ser Met Thr Gly Gly Gln Gln Met Gly Arg
            20                  25                  30

Gly Ser Met Lys Lys Ala Glu Asn Ala His Asn Thr Pro Leu Leu Val
        35                  40                  45

Leu Tyr Gly Ser Asn Met Gly Thr Ala Glu Gly Thr Ala Arg Asp Leu
    50                  55                  60
```

```
Ala Asp Ile Ala Met Ser Lys Gly Phe Ala Pro Gln Val Ala Thr Leu
65                  70                  75                  80

Asp Ser His Ala Gly Asn Leu Pro Arg Glu Gly Ala Val Leu Ile Val
                85                  90                  95

Thr Ala Ser Tyr Asn Gly His Pro Pro Asp Asn Ala Lys Gln Phe Val
            100                 105                 110

Asp Trp Leu Asp Gln Ala Ser Ala Asp Glu Val Lys Gly Val Arg Tyr
        115                 120                 125

Ser Val Phe Gly Cys Gly Asp Lys Asn Trp Ala Thr Thr Tyr Gln Lys
    130                 135                 140

Val Pro Ala Phe Ile Asp Glu Thr Leu Ala Ala Lys Gly Ala Glu Asn
145                 150                 155                 160

Ile Ala Asp Arg Gly Glu Ala Asp Ala Ser Asp Asp Phe Glu Gly Thr
                165                 170                 175

Tyr Glu Glu Trp Arg Glu His Met Trp Ser Asp Val Ala Ala Tyr Phe
            180                 185                 190

Asn Leu Asp Ile Glu Asn Ser Glu Asp Asn Lys Ser Thr Leu Ser Leu
        195                 200                 205

Gln Phe Val Asp Ser Ala Ala Asp Met Pro Leu Ala Lys Met His Gly
    210                 215                 220

Ala Phe Ser Thr Asn Val Val Ala Ser Lys Glu Leu Gln Gln Pro Gly
225                 230                 235                 240

Ser Ala Arg Ser Thr Arg His Leu Glu Ile Glu Leu Pro Lys Glu Ala
                245                 250                 255

Ser Tyr Gln Glu Gly Asp His Leu Gly Val Ile Pro Arg Asn Tyr Glu
            260                 265                 270

Gly Ile Val Asn Arg Val Thr Ala Arg Phe Gly Leu Asp Ala Ser Gln
        275                 280                 285

Gln Ile Arg Leu Glu Ala Glu Glu Glu Lys Leu Ala His Leu Pro Leu
    290                 295                 300

Ala Lys Thr Val Ser Val Glu Glu Leu Leu Gln Tyr Val Glu Leu Gln
305                 310                 315                 320

Asp Pro Val Thr Arg Thr Gln Leu Arg Ala Met Ala Ala Lys Thr Val
                325                 330                 335
```

```
Cys Pro Pro His Lys Val Glu Leu Glu Ala Leu Leu Glu Lys Gln Ala
            340                 345                 350

Tyr Lys Glu Gln Val Leu Ala Lys Arg Leu Thr Met Leu Glu Leu Leu
        355                 360                 365

Glu Lys Tyr Pro Ala Cys Glu Met Lys Phe Ser Glu Phe Ile Ala Leu
    370                 375                 380

Leu Pro Ser Ile Arg Pro Arg Tyr Tyr Ser Ile Ser Ser Ser Pro Arg
385                 390                 395                 400

Val Asp Glu Lys Gln Ala Ser Ile Thr Val Ser Val Val Ser Gly Glu
                405                 410                 415

Ala Trp Ser Gly Tyr Gly Glu Tyr Lys Gly Ile Ala Ser Asn Tyr Leu
            420                 425                 430

Ala Glu Leu Gln Glu Gly Asp Thr Ile Thr Cys Phe Ile Ser Thr Pro
        435                 440                 445

Gln Ser Glu Phe Thr Leu Pro Lys Asp Pro Glu Thr Pro Leu Ile Met
    450                 455                 460

Val Gly Pro Gly Thr Gly Val Ala Pro Phe Arg Gly Phe Val Gln Ala
465                 470                 475                 480

Arg Lys Gln Leu Lys Glu Gln Gly Gln Ser Leu Gly Glu Ala His Leu
                485                 490                 495

Tyr Phe Gly Cys Arg Ser Pro His Glu Asp Tyr Leu Tyr Gln Glu Glu
            500                 505                 510

Leu Glu Asn Ala Gln Ser Glu Gly Ile Ile Thr Leu His Thr Ala Phe
        515                 520                 525

Ser Arg Met Pro Asn Gln Pro Lys Thr Tyr Val Gln His Val Met Glu
    530                 535                 540

Gln Asp Gly Lys Lys Leu Ile Glu Leu Leu Asp Gln Gly Ala His Phe
545                 550                 555                 560

Tyr Ile Cys Gly Asp Gly Ser Gln Met Ala Pro Ala Val Glu Ala Thr
                565                 570                 575

Leu Met Lys Ser Tyr Ala Asp Val His Gln Val Ser Glu Ala Asp Ala
            580                 585                 590

Arg Leu Trp Leu Gln Gln Leu Glu Glu Lys Gly Arg Tyr Ala Lys Asp
        595                 600                 605

Val Trp Ala Gly
```

610

<210> 25
<211> 747
<212> DNA
<213> Escherichia coli

<220>
<221> CDS
<222> (1)..(747)

<400> 25

```
atg gct gat tgg gta aca ggc aaa gtc act aaa gtg cag aac tgg acc      48
Met Ala Asp Trp Val Thr Gly Lys Val Thr Lys Val Gln Asn Trp Thr
1               5                   10                  15

gac gcc ctg ttt agt ctc acc gtt cac gcc ccc gtg ctt ccg ttt acc      96
Asp Ala Leu Phe Ser Leu Thr Val His Ala Pro Val Leu Pro Phe Thr
            20                  25                  30

gcc ggg caa ttt acc aag ctt ggc ctt gaa atc gac ggc gaa cgc gtc     144
Ala Gly Gln Phe Thr Lys Leu Gly Leu Glu Ile Asp Gly Glu Arg Val
        35                  40                  45

cag cgc gcc tac tcc tat gta aac tcg ccc gat aat ccc gat ctg gag     192
Gln Arg Ala Tyr Ser Tyr Val Asn Ser Pro Asp Asn Pro Asp Leu Glu
    50                  55                  60

ttt tac ctg gtc acc gtc ccc gat ggc aaa tta agc cca cga ctg gcg     240
Phe Tyr Leu Val Thr Val Pro Asp Gly Lys Leu Ser Pro Arg Leu Ala
65                  70                  75                  80

gca ctg aaa cca ggc gat gaa gtg cag gtg gtt agc gaa gcg gca gga     288
Ala Leu Lys Pro Gly Asp Glu Val Gln Val Val Ser Glu Ala Ala Gly
                85                  90                  95

ttc ttt gtg ctc gat gaa gtg ccg cac tgc gaa acg cta tgg atg ctg     336
Phe Phe Val Leu Asp Glu Val Pro His Cys Glu Thr Leu Trp Met Leu
            100                 105                 110

gca acc ggt aca gcg att ggc cct tat tta tcg att ctg caa cta ggt     384
Ala Thr Gly Thr Ala Ile Gly Pro Tyr Leu Ser Ile Leu Gln Leu Gly
        115                 120                 125

aaa gat tta gat cgc ttc aaa aat ctg gtc ctg gtg cac gcc gca cgt     432
Lys Asp Leu Asp Arg Phe Lys Asn Leu Val Leu Val His Ala Ala Arg
    130                 135                 140

tat gcc gcc gac tta agc tat ttg cca ctg atg cag gaa ctg gaa aaa     480
Tyr Ala Ala Asp Leu Ser Tyr Leu Pro Leu Met Gln Glu Leu Glu Lys
145                 150                 155                 160

cgc tac gaa gga aaa ctg cgc att cag acg gtg gtc agt cgg gaa acg     528
Arg Tyr Glu Gly Lys Leu Arg Ile Gln Thr Val Val Ser Arg Glu Thr
                165                 170                 175

gca gcg ggg tcg ctc acc gga cgg ata ccg gca tta att gaa agt ggg     576
Ala Ala Gly Ser Leu Thr Gly Arg Ile Pro Ala Leu Ile Glu Ser Gly
            180                 185                 190

gaa ctg gaa agc acg att ggc ctg ccg atg aat aaa gaa acc agc cat     624
Glu Leu Glu Ser Thr Ile Gly Leu Pro Met Asn Lys Glu Thr Ser His
        195                 200                 205

gtg atg ctg tgc ggc aat cca cag atg gtg cgc gat aca caa cag ttg     672
Val Met Leu Cys Gly Asn Pro Gln Met Val Arg Asp Thr Gln Gln Leu
    210                 215                 220

ctg aaa gag acc cgg cag atg acg aaa cat tta cgt cgc cga ccg ggc     720
Leu Lys Glu Thr Arg Gln Met Thr Lys His Leu Arg Arg Arg Pro Gly
225                 230                 235                 240

cat atg aca gcg gag cat tac tgg taa                                 747
His Met Thr Ala Glu His Tyr Trp
                245
```

<210> 26

<211> 248

<212> PRT
<213> Escherichia coli

<400> 26

```
Met Ala Asp Trp Val Thr Gly Lys Val Thr Lys Val Gln Asn Trp Thr
1               5                   10                  15

Asp Ala Leu Phe Ser Leu Thr Val His Ala Pro Val Leu Pro Phe Thr
            20                  25                  30

Ala Gly Gln Phe Thr Lys Leu Gly Leu Glu Ile Asp Gly Glu Arg Val
        35                  40                  45

Gln Arg Ala Tyr Ser Tyr Val Asn Ser Pro Asp Asn Pro Asp Leu Glu
    50                  55                  60

Phe Tyr Leu Val Thr Val Pro Asp Gly Lys Leu Ser Pro Arg Leu Ala
65                  70                  75                  80

Ala Leu Lys Pro Gly Asp Glu Val Gln Val Val Ser Glu Ala Ala Gly
                85                  90                  95

Phe Phe Val Leu Asp Glu Val Pro His Cys Glu Thr Leu Trp Met Leu
            100                 105                 110

Ala Thr Gly Thr Ala Ile Gly Pro Tyr Leu Ser Ile Leu Gln Leu Gly
        115                 120                 125

Lys Asp Leu Asp Arg Phe Lys Asn Leu Val Leu Val His Ala Ala Arg
    130                 135                 140

Tyr Ala Ala Asp Leu Ser Tyr Leu Pro Leu Met Gln Glu Leu Glu Lys
145                 150                 155                 160

Arg Tyr Glu Gly Lys Leu Arg Ile Gln Thr Val Val Ser Arg Glu Thr
                165                 170                 175

Ala Ala Gly Ser Leu Thr Gly Arg Ile Pro Ala Leu Ile Glu Ser Gly
            180                 185                 190

Glu Leu Glu Ser Thr Ile Gly Leu Pro Met Asn Lys Glu Thr Ser His
        195                 200                 205
```

```
Val Met Leu Cys Gly Asn Pro Gln Met Val Arg Asp Thr Gln Gln Leu
    210                 215                 220

Leu Lys Glu Thr Arg Gln Met Thr Lys His Leu Arg Arg Arg Pro Gly
225                 230                 235                 240

His Met Thr Ala Glu His Tyr Trp
                245
```

<210> 27
<211> 1743
<212> DNA
<213> Geobacillus stearothermophilus

<220>
<221> CDS
<222> (1)..(1743)

<400> 27

```
atg ggc agc agc cat cat cat cat cat cac agc agc ggc ctg gtg ccg        48
Met Gly Ser Ser His His His His His His Ser Ser Gly Leu Val Pro
1               5                   10                  15

cgc ggc agc cat atg gct agc atg act ggt gga cag caa atg ggt cgc        96
Arg Gly Ser His Met Ala Ser Met Thr Gly Gly Gln Gln Met Gly Arg
            20                  25                  30

gga tcc gaa tct gtg acg gta ttg tac ggc tcg caa acc ggc aat gca       144
Gly Ser Glu Ser Val Thr Val Leu Tyr Gly Ser Gln Thr Gly Asn Ala
        35                  40                  45

caa aag ttg gct gag aaa gcc ggc aaa gcg ctc aaa gag cgt ggt ttt       192
Gln Lys Leu Ala Glu Lys Ala Gly Lys Ala Leu Lys Glu Arg Gly Phe
    50                  55                  60

gaa gcg aaa gtg ttg tcc atg ctt gat ttt aag ccg aac gaa tta aag       240
Glu Ala Lys Val Leu Ser Met Leu Asp Phe Lys Pro Asn Glu Leu Lys
65                  70                  75                  80

aaa gtg gag acg ctg ctc att gtc gtg agc acg cat ggg gaa ggc gat       288
Lys Val Glu Thr Leu Leu Ile Val Val Ser Thr His Gly Glu Gly Asp
                85                  90                  95

ccg ccg gac aat gcg gta tcg ttt tac gaa ttt ctc cac agc aaa cgg       336
Pro Pro Asp Asn Ala Val Ser Phe Tyr Glu Phe Leu His Ser Lys Arg
            100                 105                 110

gcg cca aag ctc aac cat ctt cgt ttc tct gtc ttg gcg ctc ggc gat       384
Ala Pro Lys Leu Asn His Leu Arg Phe Ser Val Leu Ala Leu Gly Asp
        115                 120                 125

acg tcg tat gaa cat ttt tgc cag acg ggg aaa gat ttc gac aaa cgt       432
Thr Ser Tyr Glu His Phe Cys Gln Thr Gly Lys Asp Phe Asp Lys Arg
    130                 135                 140

ctt gag gag ctg ggt gga aca cgg ttt tat ccg cgc gtc gat tgc gat       480
Leu Glu Glu Leu Gly Gly Thr Arg Phe Tyr Pro Arg Val Asp Cys Asp
145                 150                 155                 160

gtc gac tat gaa gaa gcg gcg gcg aaa tgg ctt gat ggc gtg ctt ggc       528
Val Asp Tyr Glu Glu Ala Ala Ala Lys Trp Leu Asp Gly Val Leu Gly
                165                 170                 175

gag tta agc aaa gaa gcg aat gcc cat gtt ggg gcc acg ccg ctt ttg       576
Glu Leu Ser Lys Glu Ala Asn Ala His Val Gly Ala Thr Pro Leu Leu
```

```
tcc gct gcg gcc acg gcg cca aaa atg gaa cca gct gtc gtc tat tcg      624
Ser Ala Ala Ala Thr Ala Pro Lys Met Glu Pro Ala Val Val Tyr Ser
        195                 200                 205

cgg aaa aac ccg ttc ccg gcc gag gtg ctt gaa aac atc aac tta aac      672
Arg Lys Asn Pro Phe Pro Ala Glu Val Leu Glu Asn Ile Asn Leu Asn
    210                 215                 220

ggc cgc gga tcg aac aag gaa acg cgc cat ttg gaa ttg tcg ctc gag      720
Gly Arg Gly Ser Asn Lys Glu Thr Arg His Leu Glu Leu Ser Leu Glu
225                 230                 235                 240

gga tcg ggg ttg aag tac gaa ccg ggc gat gcg ctt ggc att ttc ccg      768
Gly Ser Gly Leu Lys Tyr Glu Pro Gly Asp Ala Leu Gly Ile Phe Pro
                245                 250                 255

aaa aat gat ccc gag ctg gtt gag cgg atc att caa gaa atg aaa tgg      816
Lys Asn Asp Pro Glu Leu Val Glu Arg Ile Ile Gln Glu Met Lys Trp
            260                 265                 270

aat ccg gaa gaa acg gtg acg atc gac aaa gat ggg gaa gtg cgg tcg      864
Asn Pro Glu Glu Thr Val Thr Ile Asp Lys Asp Gly Glu Val Arg Ser
        275                 280                 285

ctg cgt gaa gcg ctc acc tca cat ttt gaa att acg gtg ctg acg aaa      912
Leu Arg Glu Ala Leu Thr Ser His Phe Glu Ile Thr Val Leu Thr Lys
    290                 295                 300

gcg ctg ttg caa aag ctg gcg acg ctg tca aaa aac agc gag ctc caa      960
Ala Leu Leu Gln Lys Leu Ala Thr Leu Ser Lys Asn Ser Glu Leu Gln
305                 310                 315                 320

gcg ctt gtg gcg ccc ggc aat gaa gcc aaa ttg aaa gaa tac gcc aaa     1008
Ala Leu Val Ala Pro Gly Asn Glu Ala Lys Leu Lys Glu Tyr Ala Lys
                325                 330                 335

ggc cgt gat ttg ttg gat gcg ctc cgc gat ttc ggc cca tgg gac gcc     1056
Gly Arg Asp Leu Leu Asp Ala Leu Arg Asp Phe Gly Pro Trp Asp Ala
            340                 345                 350

act ttg cag caa ctc atc tcc atc ttg cgg aaa atg ccg ccg cgc ctg     1104
Thr Leu Gln Gln Leu Ile Ser Ile Leu Arg Lys Met Pro Pro Arg Leu
        355                 360                 365

tac tcg atc gcg agc agc tta gcg gcc tat cca gat gaa gtg cac ttg     1152
Tyr Ser Ile Ala Ser Ser Leu Ala Ala Tyr Pro Asp Glu Val His Leu
    370                 375                 380

acg atc ggt gcg gtc cgt tat gag tcg cac ggc cgc ctg cgc aaa ggg     1200
Thr Ile Gly Ala Val Arg Tyr Glu Ser His Gly Arg Leu Arg Lys Gly
385                 390                 395                 400

gtg tgc tcg acg ttc tgc gcc gaa cgc gtc caa atc ggc gat acg ttg     1248
Val Cys Ser Thr Phe Cys Ala Glu Arg Val Gln Ile Gly Asp Thr Leu
                405                 410                 415

ccg gtc ttt gtg cag ccg aac ccg aac ttt aag ttg ccg aag gat ccc     1296
Pro Val Phe Val Gln Pro Asn Pro Asn Phe Lys Leu Pro Lys Asp Pro
            420                 425                 430

gac acg ccg atc att atg atc ggt cca ggc acc gga gtg gcg ccg ttc     1344
Asp Thr Pro Ile Ile Met Ile Gly Pro Gly Thr Gly Val Ala Pro Phe
        435                 440                 445

cgc gcc ttt atg caa gag cgc gag gcc atc ggg gcg aaa gga aaa tcg     1392
Arg Ala Phe Met Gln Glu Arg Glu Ala Ile Gly Ala Lys Gly Lys Ser
    450                 455                 460
```

```
tgg ctc ttt ttc ggg gac caa cac ttt atg acc gac ttc ctg tat caa      1440
Trp Leu Phe Phe Gly Asp Gln His Phe Met Thr Asp Phe Leu Tyr Gln
465                 470                 475                 480

acc gaa tgg ctc gca tgg ttg aaa agc ggt gtg ctg acc aaa atg gat      1488
Thr Glu Trp Leu Ala Trp Leu Lys Ser Gly Val Leu Thr Lys Met Asp
                485                 490                 495

gtc gcc ttc tct cgc gat acg gag aag aaa gtg tat gtg cag cac cgg      1536
Val Ala Phe Ser Arg Asp Thr Glu Lys Lys Val Tyr Val Gln His Arg
            500                 505                 510

atg ctt gaa cga agc aaa gag ctg ttc ggt tgg ctt gag gaa ggc gct      1584
Met Leu Glu Arg Ser Lys Glu Leu Phe Gly Trp Leu Glu Glu Gly Ala
        515                 520                 525

gtc gtc tac gtt tgc ggc gac aag caa cac atg gcg cgc gac gtt cat      1632
Val Val Tyr Val Cys Gly Asp Lys Gln His Met Ala Arg Asp Val His
    530                 535                 540

cag acg ttg atc gag att atc gaa aaa gag ggc ggc atg agc cgc gaa      1680
Gln Thr Leu Ile Glu Ile Ile Glu Lys Glu Gly Gly Met Ser Arg Glu
545                 550                 555                 560

cag gcg gaa gcg tat gtc acc gag atg caa aag caa aaa cgg tat caa      1728
Gln Ala Glu Ala Tyr Val Thr Glu Met Gln Lys Gln Lys Arg Tyr Gln
                565                 570                 575

cgc gac gtc tac taa                                                  1743
Arg Asp Val Tyr
            580
```

<210> 28
<211> 580
<212> PRT
<213> Geobacillus stearothermophilus

<400> 28

```
Met Gly Ser Ser His His His His His His Ser Ser Gly Leu Val Pro
1               5                   10                  15

Arg Gly Ser His Met Ala Ser Met Thr Gly Gly Gln Gln Met Gly Arg
            20                  25                  30

Gly Ser Glu Ser Val Thr Val Leu Tyr Gly Ser Gln Thr Gly Asn Ala
        35                  40                  45

Gln Lys Leu Ala Glu Lys Ala Gly Lys Ala Leu Lys Glu Arg Gly Phe
    50                  55                  60

Glu Ala Lys Val Leu Ser Met Leu Asp Phe Lys Pro Asn Glu Leu Lys
65                  70                  75                  80

Lys Val Glu Thr Leu Leu Ile Val Val Ser Thr His Gly Glu Gly Asp
                85                  90                  95

Pro Pro Asp Asn Ala Val Ser Phe Tyr Glu Phe Leu His Ser Lys Arg
            100                 105                 110
```

```
Ala Pro Lys Leu Asn His Leu Arg Phe Ser Val Leu Ala Leu Gly Asp
        115                 120                 125

Thr Ser Tyr Glu His Phe Cys Gln Thr Gly Lys Asp Phe Asp Lys Arg
    130                 135                 140

Leu Glu Glu Leu Gly Gly Thr Arg Phe Tyr Pro Arg Val Asp Cys Asp
145                 150                 155                 160

Val Asp Tyr Glu Glu Ala Ala Ala Lys Trp Leu Asp Gly Val Leu Gly
                165                 170                 175

Glu Leu Ser Lys Glu Ala Asn Ala His Val Gly Ala Thr Pro Leu Leu
            180                 185                 190

Ser Ala Ala Ala Thr Ala Pro Lys Met Glu Pro Ala Val Val Tyr Ser
        195                 200                 205

Arg Lys Asn Pro Phe Pro Ala Glu Val Leu Glu Asn Ile Asn Leu Asn
    210                 215                 220

Gly Arg Gly Ser Asn Lys Glu Thr Arg His Leu Glu Leu Ser Leu Glu
225                 230                 235                 240

Gly Ser Gly Leu Lys Tyr Glu Pro Gly Asp Ala Leu Gly Ile Phe Pro
                245                 250                 255

Lys Asn Asp Pro Glu Leu Val Glu Arg Ile Ile Gln Glu Met Lys Trp
            260                 265                 270

Asn Pro Glu Glu Thr Val Thr Ile Asp Lys Asp Gly Glu Val Arg Ser
        275                 280                 285

Leu Arg Glu Ala Leu Thr Ser His Phe Glu Ile Thr Val Leu Thr Lys
    290                 295                 300

Ala Leu Leu Gln Lys Leu Ala Thr Leu Ser Lys Asn Ser Glu Leu Gln
305                 310                 315                 320

Ala Leu Val Ala Pro Gly Asn Glu Ala Lys Leu Lys Glu Tyr Ala Lys
                325                 330                 335

Gly Arg Asp Leu Leu Asp Ala Leu Arg Asp Phe Gly Pro Trp Asp Ala
            340                 345                 350

Thr Leu Gln Gln Leu Ile Ser Ile Leu Arg Lys Met Pro Pro Arg Leu
        355                 360                 365

Tyr Ser Ile Ala Ser Ser Leu Ala Ala Tyr Pro Asp Glu Val His Leu
    370                 375                 380

Thr Ile Gly Ala Val Arg Tyr Glu Ser His Gly Arg Leu Arg Lys Gly
```

385 390 395 400

Val Cys Ser Thr Phe Cys Ala Glu Arg Val Gln Ile Gly Asp Thr Leu
405 410 415

Pro Val Phe Val Gln Pro Asn Pro Asn Phe Lys Leu Pro Lys Asp Pro
420 425 430

Asp Thr Pro Ile Ile Met Ile Gly Pro Gly Thr Gly Val Ala Pro Phe
435 440 445

Arg Ala Phe Met Gln Glu Arg Glu Ala Ile Gly Ala Lys Gly Lys Ser
450 455 460

Trp Leu Phe Phe Gly Asp Gln His Phe Met Thr Asp Phe Leu Tyr Gln
465 470 475 480

Thr Glu Trp Leu Ala Trp Leu Lys Ser Gly Val Leu Thr Lys Met Asp
485 490 495

Val Ala Phe Ser Arg Asp Thr Glu Lys Lys Val Tyr Val Gln His Arg
500 505 510

Met Leu Glu Arg Ser Lys Glu Leu Phe Gly Trp Leu Glu Glu Gly Ala
515 520 525

Val Val Tyr Val Cys Gly Asp Lys Gln His Met Ala Arg Asp Val His
530 535 540

Gln Thr Leu Ile Glu Ile Ile Glu Lys Glu Gly Gly Met Ser Arg Glu
545 550 555 560

Gln Ala Glu Ala Tyr Val Thr Glu Met Gln Lys Gln Lys Arg Tyr Gln
565 570 575

Arg Asp Val Tyr
580

<210> 29
<211> 702
<212> DNA
<213> Escherichia coli

<220>
<221> CDS
<222> (1)..(702)

<400> 29

atg aca acc tta agc tgt aaa gtg acc tcg gta gaa gct atc acg gat 48
Met Thr Thr Leu Ser Cys Lys Val Thr Ser Val Glu Ala Ile Thr Asp
1 5 10 15

acc gta tat cgt gtc cgc atc gtg cca gac gcg gcc ttt tct ttt cgt 96
Thr Val Tyr Arg Val Arg Ile Val Pro Asp Ala Ala Phe Ser Phe Arg
20 25 30

gct ggt cag tat ttg atg gta gtg atg gat gag cgc gac aaa cgt ccg 144
Ala Gly Gln Tyr Leu Met Val Val Met Asp Glu Arg Asp Lys Arg Pro
35 40 45

ttc tca atg gct tcg acg ccg gat gaa aaa ggg ttt atc gag ctg cat 192
Phe Ser Met Ala Ser Thr Pro Asp Glu Lys Gly Phe Ile Glu Leu His
50 55 60

att ggc gct tct gaa atc aac ctt tac gcg aaa gca gtc atg gac cgc 240
Ile Gly Ala Ser Glu Ile Asn Leu Tyr Ala Lys Ala Val Met Asp Arg
65 70 75 80

atc ctc aaa gat cat caa atc gtg gtc gac att ccc cac gga gaa gcg 288
Ile Leu Lys Asp His Gln Ile Val Val Asp Ile Pro His Gly Glu Ala
85 90 95

tgg ctg cgc gat gat gaa gag cgt ccg atg att ttg att gcg ggc ggc 336
Trp Leu Arg Asp Asp Glu Glu Arg Pro Met Ile Leu Ile Ala Gly Gly
100 105 110

acc ggg ttc tct tat gcc cgc tcg att ttg ctg aca gcg ttg gcg cgt 384
Thr Gly Phe Ser Tyr Ala Arg Ser Ile Leu Leu Thr Ala Leu Ala Arg
115 120 125

aac cca aac cgt gat atc acc att tac tgg ggc ggg cgt gaa gag cag 432
Asn Pro Asn Arg Asp Ile Thr Ile Tyr Trp Gly Gly Arg Glu Glu Gln
130 135 140

cat ctg tat gat ctc tgc gag ctt gag gcg ctt tcg ttg aag cat cct 480
His Leu Tyr Asp Leu Cys Glu Leu Glu Ala Leu Ser Leu Lys His Pro
145 150 155 160

ggt ctg caa gtg gtg ccg gtg gtt gaa caa ccg gaa gcg ggc tgg cgt 528
Gly Leu Gln Val Val Pro Val Val Glu Gln Pro Glu Ala Gly Trp Arg
165 170 175

ggg cgt act ggc acc gtg tta acg gcg gta ttg cag gat cac ggt acg 576
Gly Arg Thr Gly Thr Val Leu Thr Ala Val Leu Gln Asp His Gly Thr
180 185 190

ctg gca gag cat gat atc tat att gcc gga cgt ttt gag atg gcg aaa 624
Leu Ala Glu His Asp Ile Tyr Ile Ala Gly Arg Phe Glu Met Ala Lys
195 200 205

att gcc cgc gat ctg ttt tgc agt gag cgt aat gcg cgg gaa gat cgc 672
Ile Ala Arg Asp Leu Phe Cys Ser Glu Arg Asn Ala Arg Glu Asp Arg
210 215 220

ctg ttt ggc gat gcg ttt gca ttt atc tga 702
Leu Phe Gly Asp Ala Phe Ala Phe Ile
225 230

<210> 30
<211> 233
<212> PRT
<213> Escherichia coli

<400> 30

```
Met Thr Thr Leu Ser Cys Lys Val Thr Ser Val Glu Ala Ile Thr Asp
1               5                   10                  15

Thr Val Tyr Arg Val Arg Ile Val Pro Asp Ala Ala Phe Ser Phe Arg
            20                  25                  30

Ala Gly Gln Tyr Leu Met Val Val Met Asp Glu Arg Asp Lys Arg Pro
        35                  40                  45

Phe Ser Met Ala Ser Thr Pro Asp Glu Lys Gly Phe Ile Glu Leu His
    50                  55                  60

Ile Gly Ala Ser Glu Ile Asn Leu Tyr Ala Lys Ala Val Met Asp Arg
65                  70                  75                  80

Ile Leu Lys Asp His Gln Ile Val Val Asp Ile Pro His Gly Glu Ala
                85                  90                  95

Trp Leu Arg Asp Asp Glu Glu Arg Pro Met Ile Leu Ile Ala Gly Gly
            100                 105                 110

Thr Gly Phe Ser Tyr Ala Arg Ser Ile Leu Leu Thr Ala Leu Ala Arg
        115                 120                 125

Asn Pro Asn Arg Asp Ile Thr Ile Tyr Trp Gly Gly Arg Glu Glu Gln
    130                 135                 140

His Leu Tyr Asp Leu Cys Glu Leu Glu Ala Leu Ser Leu Lys His Pro
145                 150                 155                 160

Gly Leu Gln Val Val Pro Val Val Glu Gln Pro Glu Ala Gly Trp Arg
                165                 170                 175

Gly Arg Thr Gly Thr Val Leu Thr Ala Val Leu Gln Asp His Gly Thr
            180                 185                 190

Leu Ala Glu His Asp Ile Tyr Ile Ala Gly Arg Phe Glu Met Ala Lys
        195                 200                 205

Ile Ala Arg Asp Leu Phe Cys Ser Glu Arg Asn Ala Arg Glu Asp Arg
    210                 215                 220

Leu Phe Gly Asp Ala Phe Ala Phe Ile
225                 230
```